# EUROPEAN PATENT SPECIFICATION

(11) **EP 4 349 831 B1**
(45) Date of publication and mention of the grant of the patent: **19.08.2026**
(21) Application number: 22810644.9
(22) Date of filing: 27.05.2022
(51) Int. Cl.: C07D 403/06, C07D 403/14, A61K 31/454, A61K 31/4545, A61K 31/496, A61K 31/501, A61P 15/00, C07H 17/04

(54) **QUINOLINAMINE COMPOUND, PREPARATION METHOD THEREFOR AND APPLICATION THEREOF IN PHARMACEUTICALS**
CHINOLINAMINVERBINDUNG, HERSTELLUNGSVERFAHREN DAFÜR UND ANWENDUNG DAVON IN ARZNEIMITTELN
COMPOSÉ QUINOLINAMINE, SON PROCÉDÉ DE PRÉPARATION ET SON UTILISATION DANS DES PRODUITS PHARMACEUTIQUES

(30) Priority: 27.05.2021 CN 202110606803; 24.08.2021 CN 202110976020
(43) Date of publication of application: 10.04.2024
(73) Proprietor: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YANG, Fanglong, Shanghai 200245 (CN); JIA, Minqiang, Shanghai 200245 (CN); TANG, Huanyu, Shanghai 200245 (CN); QUE, Yonglei, Shanghai 200245 (CN); HE, Feng, Shanghai 200245 (CN); TAO, Weikang, Shanghai 200245 (CN)
(74) Representative: Potter Clarkson
(86) International application number: PCT/CN2022/095441
(87) International publication number: WO 2022/247920

(56) References cited:
- WO-A1-2016/135052
- WO-A1-2020/127843
- WO-A1-2020/127843
- WO-A1-2021/151062
- CN-A- 107 207 463
- CN-A- 107 531 681
- CN-A- 107 531 681
- JP-A- 2011 026 251
- JP-A- 2011 026 251
- CAMPOS JOANA F. ET AL: "Synthesis of Benzo-Fused 11H-Pyrido[2,1-b]quinazolin-11-ones by a Buchwald-Hartwig Coupling/Pyridine Dearomatization Sequence in Eucalyptol", SYNTHESIS, 27 July 2020 (2020-07-27), STUTTGART, DE., XP093188597, ISSN: 0039-7881, DOI: 10.1055/s-0040-1707158
- PITTALÀ VALERIA ET AL: "Synthesis and Endothelin Receptor Binding Affinity of a Novel Class of 2-Substituted-4-aryl-3-quinolinecarboxylic Acid Derivatives", MEDICINAL CHEMISTRY (SHP-SARIQAH, UNITED ARAB EMIRATES), 1 January 2008 (2008-01-01), pages 129 - 137, XP093188722, Retrieved from the Internet <URL:https://www.eurekaselect.com/article/27322> DOI: 10.2174/157340608783789095

## Description

### TECHNICAL FIELD

The present disclosure belongs to the field of pharmaceutics and relates to a quinolinamine compound, a preparation method therefor, and pharmaceutical use thereof. In particular, the present disclosure relates to a quinolinamine compound of general formula (I), a preparation method therefor, a pharmaceutical composition containing the compound, and use thereof as a therapeutic agent, especially use thereof as a miRNA regulator and use thereof in preparing a medicament for treating a disease or condition that is ameliorated by regulating the level of a miRNA.

### BACKGROUND

MicroRNAs (miRNAs) are a class of non-encoding, single-stranded RNA molecules of about 22 nucleotides in length that are encoded by endogenous genes and are involved in the regulation of post-transcriptional gene expression in animals and plants. Each miRNA can have multiple target genes, and several miRNAs can also regulate the same gene. This complex network can be used for accurately regulating and controlling the target genes. miR-124 is widely expressed in tissues throughout the body and is particularly highly expressed in brain tissues. Research shows that the overexpression of miR-124 can promote the transition of activated macrophages/microglia to quiescence, thereby inhibiting the autoimmune disease encephalomyelitis (Ponomarev ED et al., Nat Med, 2011; 17:67-70). In addition, miR-124 can promote the conversion of macrophages to M2 type, thereby exerting anti-inflammatory effects (Veremeyko T, et al., Plos One, 2013; 8:e81774). miR-124 also affects T cell differentiation, and miR-124-treated T cells have decreased levels of both IFN-γ and TNFα. The overexpression of miR-124 exerts anti-inflammatory effects by down-regulating STAT3 protein so as to reduce the expression of inflammatory cytokine IL-17 and inhibit the differentiation of Th17 cells (Wei J et al., Cancer Res, 2013; 73:3913-3926). Statistical studies have reported that the level of miR-124 in pediatric ulcerative colitis is much lower than that in healthy individuals, suggesting that the up-regulation of miR-124 may inhibit intestinal inflammatory responses (Koukos G, et al., Gastroenterology, 2013; 145:842-852). In addition, Nakamachi's team found that miR-124 was significantly down-regulated in synovial cells from rheumatoid arthritis patients as compared to osteoarthritis patients (Nakamachi, Y, et al., Arthritis Rheum, 2009; 60:1294-1304). The above studies suggest that the development of a novel small-molecule drug for up-regulating miR-124 can be used to effectively treat related inflammatory diseases. Inflammation is a protective response of the immune system to local infection or tissue damage, and a severe inflammatory response may damage the body. The inflammatory response is generally manifested by pain, fever, redness, swelling, and loss of function. Inflammatory diseases encompass a variety of conditions, including autoimmune-related inflammatory diseases, inflammatory diseases in the central nervous system (CNS), inflammatory diseases in the joints, inflammatory diseases in the digestive tract, inflammatory diseases in the skin, and the like. Inflammatory bowel disease (IBD) and rheumatoid arthritis (RA) are the two most common inflammatory diseases that have received a lot of attention.

Inflammatory bowel disease is an idiopathic inflammatory disease of the intestinal tract, with clinical manifestations of diarrhea, abdominal pain, and possibly even bloody stools. At present, the etiology and pathogenesis of IBD are not yet completely clear, and it is known that the inflammatory response caused by an abnormal reaction in intestinal mucosal immune system plays an important role in the pathogenesis of IBD, and that multiple factors such as environment, heredity, infection, and immune factors may contribute to the disease. IBD usually refers to ulcerative colitis (UC) and Crohn's disease (CD), wherein ulcerative colitis is a continuous inflammation of the mucosal and submucosal layers of the colon, which usually involves the rectum firstly and gradually spreads to the whole colon, while Crohn's disease may involve the whole digestive tract and is a discontinuous full-thickness inflammation, with most frequently involved parts being the terminal ileum, colon, and perianal area. IBD is usually manifested by excessive immune cell invasion of the intestinal mucosa, imbalance of T cell subsets including Th17, Th1, and Treg, and excessive activation of macrophages and dendritic cells. Drugs currently on the market or in clinical trials include JAK inhibitors and TNFα antibodies for attenuating inflammatory responses, IL-12 and IL-23 antibodies for inhibiting the differentiation of Th1 and Th17, and integrin α4β7 antibodies for blocking inflammatory cell infiltration.

Rheumatoid arthritis is a systemic inflammatory disease affecting the joint lining tissue (called synovium), characterized by polyarticular, symmetrical, and aggressive joint inflammation of the small joints of the hands and feet, and often accompanied by involvement of extra-articular organs, which may lead to joint deformity and loss of function. Inflammatory cytokines (like tumor necrosis factor TNFα and interleukins IL-1 and IL-6) play an important role in the pathogenesis of rheumatoid arthritis (RA). Small-molecule disease-modifying antirheumatic drugs (DMARDs) or biological drugs like TNFα inhibitors are commonly used for treatment. However, patients who respond to these drugs often become non-responders after a few years of use. Therefore, there is a need to develop a treatment method with a novel mechanism of action that is both therapeutically effective and safe for long-term use.

Related published patent applications include WO2010143169A2, WO2015001518A1, WO2016009065A2, WO2017158201A1, WO2020127843A1, WO2016135052A1, etc.

### SUMMARY

The present disclosure provides a compound of general formula (I) or a pharmaceutically acceptable salt thereof: 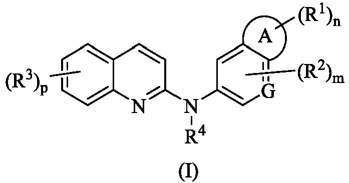 wherein
ring A is 3- to 8-membered cycloalkyl or 3- to 8-membered heterocyclyl;
G is N atom or CR^{2a};
each R¹ is identical or different and is independently selected from the group consisting of hydrogen atom, deuterium atom, halogen, C₁₋₆ alkyl, C₁₋₆ alkoxy, oxo, and -C(O)R⁸;
each R² is identical or different and is independently selected from the group consisting of hydrogen atom, halogen, and C₁₋₆ alkyl;
each R³ is identical or different and is independently selected from the group consisting of halogen, C₁₋₆ alkyl C₁₋₆ alkoxy, 3- to 8-membered cycloalkyl, 3- to 8-membered heterocyclyl, and cyano;
or two adjacent R³, together with the carbon atom on the benzene ring to which they are attached, form a 5- or 6-membered cycloalkyl;
R⁴ is hydrogen atom or 3- to 8-membered heterocyclyl, wherein the 3- to 8-membered heterocyclyl is substituted with one or more identical or different substituents selected from the group consisting of hydroxy and carboxyl;
R⁸ is C₁₋₆ alkyl;
R^{2a} is selected from the group consisting of hydrogen atom, halogen, and C₁₋₆ alkyl;
n is 0, 1, 2, 3, or 4;
m is 0, 1, or 2; and
p is 1, 2, 3, or 4.

In some embodiments of the present disclosure, the compound of general formula (I) or the pharmaceutically acceptable salt thereof is provided, wherein
ring A is 3- to 8-membered cycloalkyl or 3- to 8-membered heterocyclyl;
G is N atom or CR^{2a};
each R¹ is identical or different and is independently selected from the group consisting of hydrogen atom, halogen, C₁₋₆ alkyl, C₁₋₆ alkoxy, oxo, and -C(O)R⁸;
each R² is identical or different and is independently selected from the group consisting of hydrogen atom, halogen, and C₁₋₆ alkyl;
each R³ is identical or different and is independently selected from the group consisting of halogen, C₁₋₆ alkyl C₁₋₆ alkoxy, 3- to 8-membered cycloalkyl, and 3- to 8-membered heterocyclyl, ;
or two adjacent R³, together with the carbon atom on the benzene ring to which they are attached, form a 5- or 6-membered cycloalkyl;
R⁴ is hydrogen atom or 3- to 8-membered heterocyclyl, wherein the 3- to 8-membered heterocyclyl is substituted with one or more identical or different substituents selected from the group consisting of hydroxy and carboxyl;
R⁸ is C₁₋₆ alkyl;
R^{2a} is selected from the group consisting of hydrogen atom, halogen and C₁₋₆ alkyl;
n is 0, 1, 2, 3, or 4;
m is 0, 1, or 2; and
p is 1, 2, 3, or 4.

In some embodiments of the present disclosure, the compound of general formula (I) or the pharmaceutically acceptable salt thereof is a compound of general formula (IC) or a pharmaceutically acceptable salt thereof: 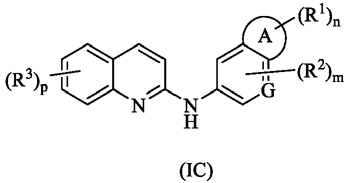 wherein
ring A, G, R¹ to R³, n, m, and p are as defined in general formula (I).

In some embodiments of the present disclosure, the compound of general formula (I) or general formula (IC) or the pharmaceutically acceptable salt thereof is provided, wherein 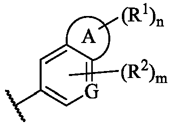 is not 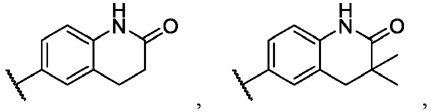 and

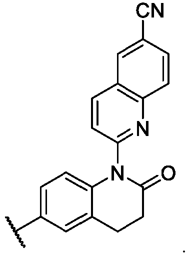

In some embodiments of the present disclosure, the compound of general formula (I) or the pharmaceutically acceptable salt thereof is a compound of general formula (I-1) or general formula (I-2) or a pharmaceutically acceptable salt thereof: 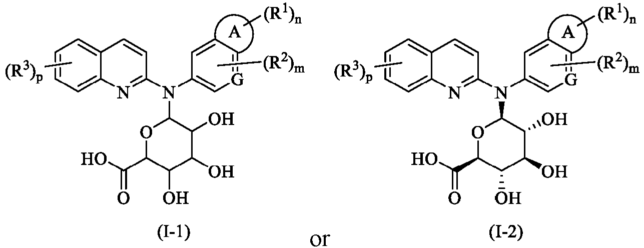 wherein
ring A, G, R¹ to R³, n, m, and p are as defined in general formula (I).

In some embodiments of the present disclosure, the compound of general formula (I) or the pharmaceutically acceptable salt thereof is a compound of general formula (I-3) or general formula (I-4) or a pharmaceutically acceptable salt thereof: 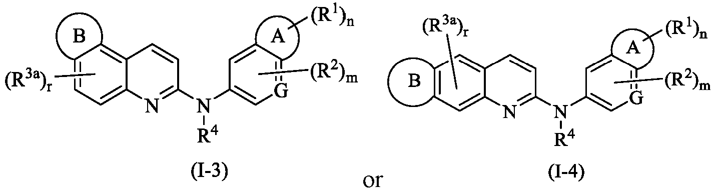 wherein
ring B is a 5- or 6-membered cycloalkyl;
each R^{3a} is identical or different and is independently selected from the group consisting of halogen, C₁₋₆ alkyl, C₁₋₆ alkoxy, and cyano;
r is 0, 1, or 2;
ring A, G, R¹, R², R⁴, n, and m are as defined in general formula (I).

In some embodiments of the present disclosure, the compound of general formula (I) or the pharmaceutically acceptable salt thereof is a compound of general formula (I-3C) or general formula (I-4C) or a pharmaceutically acceptable salt thereof: 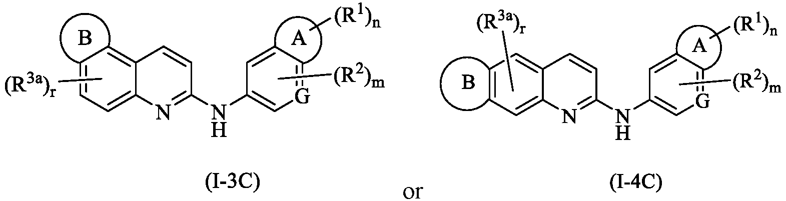 wherein
ring A, ring B, G, R¹, R², R^{3a}, n, r, and m are as defined in general formula (I-3) or general formula (I-4).

In some embodiments of the present disclosure, the compound of general formula (I-3), general formula (I-3C), general formula (I-4), or general formula (I-4C) or the pharmaceutically acceptable salt thereof is provided, wherein ring B is 5- or 6-membered cycloalkyl; preferably, ring B is cyclopentyl.

In some embodiments of the present disclosure, the compound of general formula (I), general formula (I-1), general formula (I-2), general formula (I-3), general formula (I-3C), general formula (I-4), general formula (I-4C), or general formula (IC) or the pharmaceutically acceptable salt thereof is provided, wherein ring A is 3- to 8-membered cycloalkyl or 3- to 8-membered heterocyclyl; preferably, ring A is 5- or 6-membered cycloalkyl or 5- or 6-membered heterocyclyl.

In some embodiments of the present disclosure, the compound of general formula (I), general formula (I-1), general formula (I-2), general formula (I-3), general formula (I-3C), general formula (I-4), general formula (I-4C), or general formula (IC) or the pharmaceutically acceptable salt thereof is provided, wherein each R¹ is identical or different and is independently selected from the group consisting of hydrogen atom, deuterium atom, halogen, -C(O)R⁸, C₁₋₆ alkyl, C₁₋₆ alkoxy, and oxo, preferably selected from the group consisting of hydrogen atom, deuterium atom, halogen, -C(O)R⁸, and C₁₋₆ alkyl; R⁸ is as defined in general formula (I).

In some embodiments of the present disclosure, the compound of general formula (I), general formula (I-1), general formula (I-2), general formula (I-3), general formula (I-3C), general formula (I-4), general formula (I-4C), or general formula (IC) or the pharmaceutically acceptable salt thereof is provided, wherein each R¹ is identical or different and is independently selected from the group consisting of hydrogen atom, deuterium atom, fluorine, acetyl, methyl, and oxo.

In some embodiments of the present disclosure, the compound of general formula (I), general formula (I-1), general formula (I-2), general formula (I-3), general formula (I-3C), general formula (I-4), general formula (I-4C), or general formula (IC) or the pharmaceutically acceptable salt thereof is provided, wherein each R¹ is identical or different and is independently selected from the group consisting of hydrogen atom, halogen, -C(O)R⁸, C₁₋₆ alkyl, C₁₋₆ alkoxy, and oxo; R⁸ is as defined in general formula (I).

In some embodiments of the present disclosure, the compound of general formula (I), general formula (I-1), general formula (I-2), general formula (I-3), general formula (I-3C), general formula (I-4), general formula (I-4C), or general formula (IC) or the pharmaceutically acceptable salt thereof is provided, wherein R⁸ is C₁₋₆ alkyl; preferably, R⁸ is methyl.

In some embodiments of the present disclosure, the compound of general formula (I) or the pharmaceutically acceptable salt thereof is a compound of general formula (II) or a pharmaceutically acceptable salt thereof: 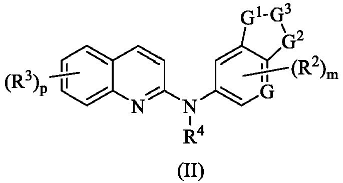 wherein 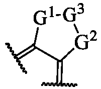 is selected from the group consisting of 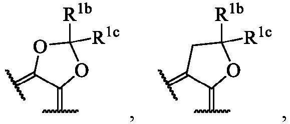 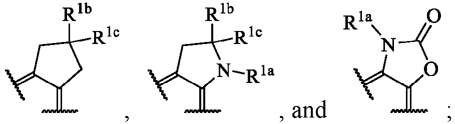
R^{1a} is selected from the group consisting of hydrogen atom, C₁₋₆ alkyl, and -C(O)R⁸;
R^{1b} and R^{1c} are identical or different and are each independently selected from the group consisting of hydrogen atom, deuterium atom, halogen, and C₁₋₆ alkyl;
G, R² to R⁴, R⁸, m and p are as defined in general formula (I).

In some embodiments of the present disclosure, the compound of general formula (II) or the pharmaceutically acceptable salt thereof is provided, wherein 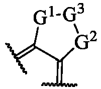 is selected from the group consisting of 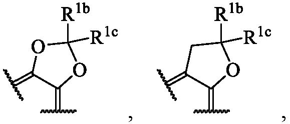 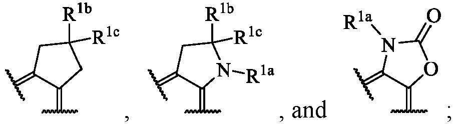
R^{1a} is selected from the group consisting of hydrogen atom, C₁₋₆ alkyl, and -C(O)R⁸;
R^{1b} and R^{1c} are identical or different and are each independently selected from the group consisting of hydrogen atom, halogen, and C₁₋₆ alkyl;
G, R² to R⁴, R⁸, m, and p are as defined in general formula (I).

In some embodiments of the present disclosure, the compound of general formula (I) or general formula (II) or the pharmaceutically acceptable salt thereof is a compound of general formula (IIC) or a pharmaceutically acceptable salt thereof: 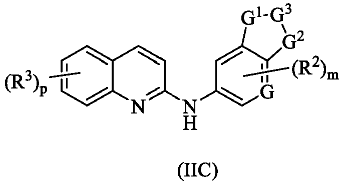 wherein
G, G¹, G², G³, R², R³, m, and p are as defined in general formula (II).

In some embodiments of the present disclosure, the compound of general formula (I) or general formula (II) or the pharmaceutically acceptable salt thereof is a compound of general formula (IID-1) or (IID-2) or a pharmaceutically acceptable salt thereof: 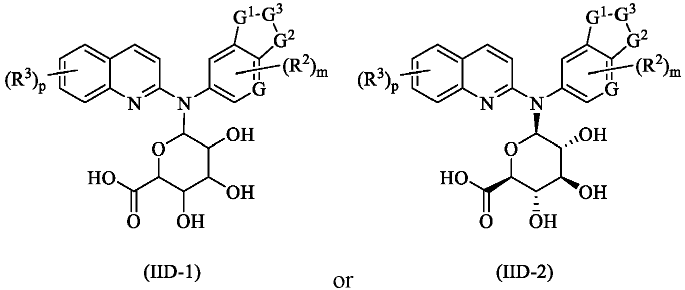 wherein
G, G¹, G², G³, R², R³, m, and p are as defined in general formula (II).

In some embodiments of the present disclosure, the compound of general formula (II), general formula (IIC), general formula (IID-1), or general formula (IID-2) or the pharmaceutically acceptable salt thereof is provided, wherein 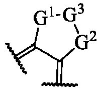 is selected from the group consisting of

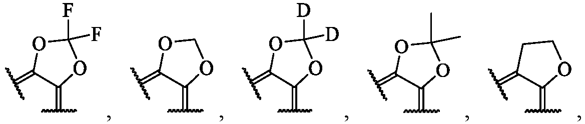

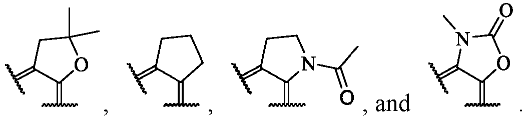

In some embodiments of the present disclosure, the compound of general formula (I) or the pharmaceutically acceptable salt thereof is a compound of general formula (III) or a pharmaceutically acceptable salt thereof: 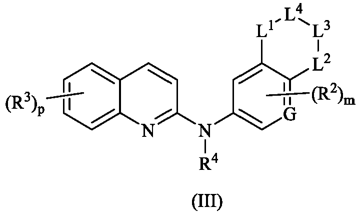 wherein 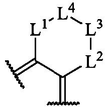 is 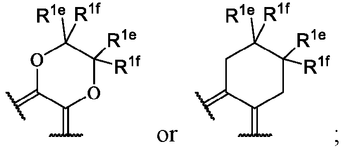
R^{1e} and R^{1f} are identical or different and are each independently selected from the group consisting of hydrogen atom, deuterium atom, halogen, and C₁₋₆ alkyl;
G, R² to R⁴, m, and p are as defined in general formula (I).

In some embodiments of the present disclosure, the compound of general formula (III) or the pharmaceutically acceptable salt thereof is provided, wherein 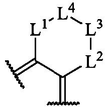 is 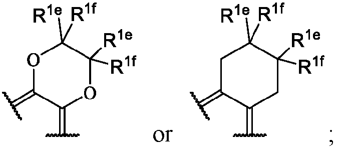
R^{1e} and R^{1f} are identical or different and are each independently selected from the group consisting of hydrogen atom, halogen, and C₁₋₆ alkyl;
G, R² to R⁴, m, and p are as defined in general formula (I).

In some embodiments of the present disclosure, the compound of general formula (I) or general formula (III) or the pharmaceutically acceptable salt thereof is a compound of general formula (IIIC) or a pharmaceutically acceptable salt thereof: 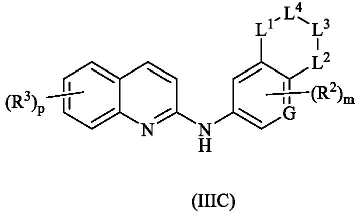 wherein
G, L¹, L², L³, L⁴, R², R³, m, and p are as defined in general formula (III).

In some embodiments of the present disclosure, the compound of general formula (I) or general formula (III) or the pharmaceutically acceptable salt thereof is a compound of general formula (IIID-1) or general formula (IIID-2) or a pharmaceutically acceptable salt thereof: 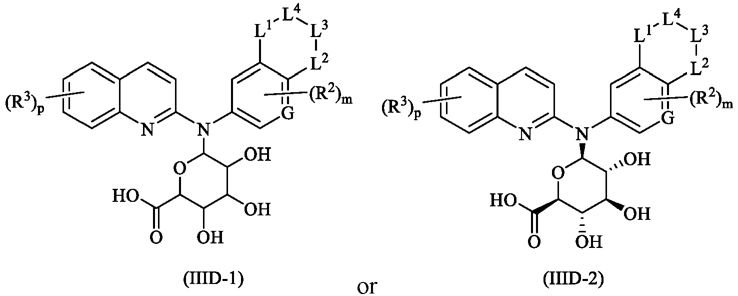 wherein
G, L¹, L², L³, L⁴, R², R³, m, and p are as defined in general formula (III).

In some embodiments of the present disclosure, the compound of general formula (III), general formula (IIIC), general formula (IIID-1), or general formula (IIID-2) or the pharmaceutically acceptable salt thereof is provided, wherein 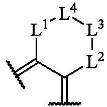 is selected from the group consisting of

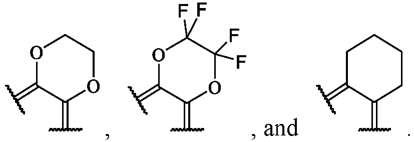

In some embodiments of the present disclosure, the compounds of general formula (I), general formula (I-1), general formula (I-2), general formula (I-3), general formula (I-4), general formula (IC), general formula (I-3C), general formula (I-4C), general formula (IIC), general formula (IIIC), general formula (II), general formula (IID-1), general formula (IID-2), general formula (IIID-1), general formula (IIID-2), and general formula (III) or the pharmaceutically acceptable salts thereof are provided, wherein each R² is identical or different and is independently selected from the group consisting of hydrogen atom, halogen, and C₁₋₆ alkyl; preferably, R² is hydrogen atom.

In some embodiments of the present disclosure, the compound of general formula (I), general formula (I-1), general formula (I-2), general formula (IC), general formula (IIC), general formula (IIIC), general formula (II), general formula (IID-1), general formula (IID-2), general formula (IIID-1), general formula (IIID-2), or general formula (III) or the pharmaceutically acceptable salt thereof is provided, wherein each R³ is identical or different and is independently halogen or C₁₋₆ alkyl; preferably, R³ is halogen; more preferably, R³ is Cl.

In some embodiments of the present disclosure, the compound of general formula (I-3), general formula (I-4), general formula (I-3C), or general formula (I-4C) or the pharmaceutically acceptable salt thereof is provided, wherein each R^{3a} is identical or different and is independently halogen or C₁₋₆ alkyl; preferably, R^{3a} is halogen; more preferably, R^{3a} is Cl.

In some embodiments of the present disclosure, the compound of general formula (I), general formula (I-1), general formula (I-2), general formula (IC), general formula (IIC), general formula (IIIC), general formula (II), general formula (IID-1), general formula (IID-2), general formula (IIID-1), general formula (IIID-2), or general formula (III) or the pharmaceutically acceptable salt thereof is provided, wherein each R³ is identical or different and is independently selected from the group consisting of halogen, C₁₋₆ alkyl, C₁₋₆ alkoxy, and cyano.

In some embodiments of the present disclosure, the compound of general formula (I), general formula (I-1), general formula (I-2), general formula (IC), general formula (IIC), general formula (IIIC), general formula (II), general formula (IID-1), general formula (IID-2), general formula (IIID-1), general formula (IIID-2), or general formula (III) or the pharmaceutically acceptable salt thereof is provided, wherein each R³ is identical or different and is independently selected from the group consisting of halogen, C₁₋₆ alkyl, C₁₋₆ alkoxy, 3- to 8-membered cycloalkyl, 3- to 8-membered heterocyclyl, and cyano; preferably, each R³ is identical or different and is independently selected from the group consisting of Cl, Br, methyl, methoxy, cyano, cyclopropyl, tetrahydropyranyl, and dihydropyranyl; more preferably, each R³ is identical or different and is independently selected from the group consisting of Cl, methyl, methoxy, cyano, cyclopropyl, and tetrahydropyranyl.

In some embodiments of the present disclosure, the compound of general formula (I-3), general formula (I-4), general formula (I-3C), or general formula (I-4C) or the pharmaceutically acceptable salt thereof is provided, wherein each R^{3a} is identical or different and is independently selected from the group consisting of halogen, C₁₋₆ alkyl, C₁₋₆ alkoxy, and cyano.

In some embodiments of the present disclosure, the compound of general formula (I), general formula (I-1), general formula (I-2), general formula (IC), general formula (IIC), general formula (IIIC), general formula (II), general formula (IID-1), general formula (IID-2), general formula (IIID-1), general formula (IIID-2), or general formula (III) or the pharmaceutically acceptable salt thereof is provided, wherein p is 2, 3, or 4, and two adjacent R³, together with the carbon atom on the benzene ring to which they are attached, form a 5- or 6-membered cycloalkyl; preferably, p is 3.

In some embodiments of the present disclosure, the compound of general formula (I), general formula (I-1), general formula (I-2), general formula (I-3), general formula (I-4), general formula (IC), general formula (I-3C), general formula (I-4C), general formula (IIC), general formula (IIIC), general formula (II), general formula (IID-1), general formula (IID-2), general formula (IIID-1), general formula (IIID-2), or general formula (III) or the pharmaceutically acceptable salt thereof is provided, wherein m is 0. In some embodiments of the present disclosure, the compound of general formula (I), general formula (I-1), general formula (I-2), general formula (IC), general formula (IIC), general formula (IIIC), general formula (II), general formula (IID-1), general formula (IID-2), general formula (IIID-1), general formula (IIID-2), or general formula (III) or the pharmaceutically acceptable salt thereof is provided, wherein p is 1 or 2.

In some embodiments of the present disclosure, the compound of general formula (I), general formula (I-1), general formula (I-2), general formula (IC), general formula (IIC), general formula (IIIC), general formula (II), general formula (IID-1), general formula (IID-2), general formula (IIID-1), general formula (IIID-2), or general formula (III) or the pharmaceutically acceptable salt thereof is provided, wherein p is 3.

In some embodiments of the present disclosure, the compound of general formula (I-3), general formula (I-3C), general formula (I-4C), or general formula (I-4) or the pharmaceutically acceptable salt thereof is provided, wherein r is 1 or 0.

In some embodiments of the present disclosure, the compound of general formula (I), general formula (IC), general formula (I-1), general formula (I-2), general formula (II), general formula (IIC), general formula (IID-1), general formula (IID-2), general formula (III), general formula (IIIC), general formula (IIID-1), or general formula (IIID-2) or the pharmaceutically acceptable salt thereof is provided, wherein 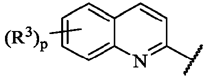 is 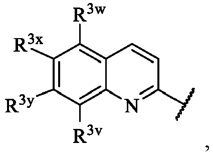 wherein R^{3v}, R^{3w}, R^{3x}, and R^{3y} are identical or different and are each independently selected from the group consisting of hydrogen atom, halogen, C₁₋₆ alkyl, C₁₋₆ alkoxy, cyano, 3- to 8-membered cycloalkyl, 3-to 8-membered heterocyclyl, or adjacent two of R^{3v}, R^{3w}, R^{3x}, and R^{3y}, together with the carbon atom to which they are attached, form a 5- or 6-membered cycloalkyl, provided that R^{3v}, R^{3w}, R^{3x}, and R^{3y} are not all hydrogen atoms; preferably, 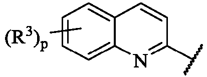 is 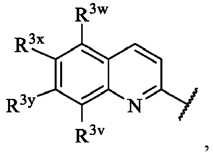 wherein R^{3v} is halogen, R^{3w}, R^{3x}, and R^{3y} are identical or different and are each independently selected from the group consisting of hydrogen atom, halogen, C₁₋₆ alkyl, C₁₋₆ alkoxy, cyano, 3- to 8-membered cycloalkyl, and 3- to 8-membered heterocyclyl, or R^{3w} and R^{3x}, together with the carbon atom to which they are attached, form 5- or 6-membered cycloalkyl; more preferably, 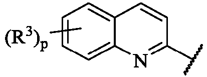 is 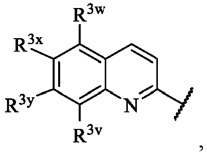 wherein R^{3v} is halogen, and R^{3w}, R^{3x}, and R^{3y} are identical or different and are each independently selected from the group consisting of hydrogen atom, halogen, C₁₋₆ alkyl, C₁₋₆ alkoxy, cyano, 3- to 6-membered cycloalkyl, and 3- to 6-membered heterocyclyl.

In some embodiments of the present disclosure, the compound of general formula (I), general formula (IC), general formula (I-1), general formula (I-2), general formula (II), general formula (IIC), general formula (IID-1), general formula (IID-2), general formula (III), general formula (IIIC), general formula (IIID-1), or general formula (IIID-2) or the pharmaceutically acceptable salt thereof is provided, wherein when 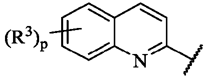 is 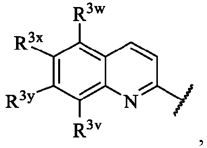 R^{3v} is Cl.

In some embodiments of the present disclosure, the compound of general formula (I), general formula (IC), general formula (I-1), general formula (I-2), general formula (II), general formula (IIC), general formula (IID-1), general formula (IID-2), general formula (III), general formula (IIIC), general formula (IIID-1), or general formula (IIID-2) or the pharmaceutically acceptable salt thereof is provided, wherein when 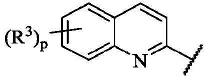 is 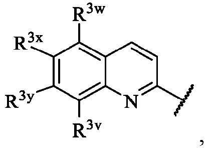 R^{3w}, R^{3x}, and R^{3y} are identical or different and are each independently selected from the group consisting of hydrogen atom, Cl, Br, methyl, methoxy, cyano, cyclopropyl, tetrahydropyranyl, and dihydropyranyl; more preferably, R^{3w}, R^{3x}, and R^{3y} are identical or different and are each independently selected from the group consisting of hydrogen atom, Cl, methyl, methoxy, cyano, cyclopropyl, and tetrahydropyranyl.

In some embodiments of the present disclosure, the compound of general formula (I) or general formula (II) or the pharmaceutically acceptable salt thereof is a compound of general formula (II-1) or a pharmaceutically acceptable salt thereof: 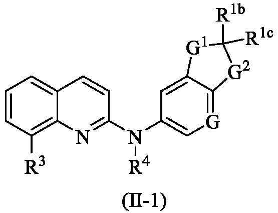 wherein
G, R³, R⁴, G¹, G², R^{1b}, and R^{1c} are as defined in general formula (II).

In some embodiments of the present disclosure, the compound of general formula (I), general formula (II), or general formula (II-1) or the pharmaceutically acceptable salt thereof is a compound of general formula (II-1C) or a pharmaceutically acceptable salt thereof:

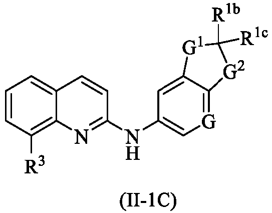

Wherein
G¹ and G² are both O atoms;
G, R³, R^{1b}, and R^{1c} are as defined in general formula (II-1).

In some embodiments of the present disclosure, the compound of general formula (II-1) or (II-1C) or the pharmaceutically acceptable salt thereof is provided, wherein G¹ and G² are both O atoms.

In some embodiments of the present disclosure, the compound of general formula (I) or general formula (III) or the pharmaceutically acceptable salt thereof is a compound of general formula (III-1) or a pharmaceutically acceptable salt thereof:

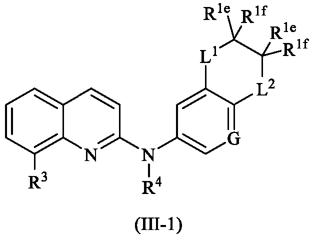

Wherein
L¹ and L² are both O atoms;
G, R³, R⁴, R^{1e}, and R^{1f} are as defined in general formula (III).

In some embodiments of the present disclosure, the compound of general formula (I), general formula (III), or general formula (III-1) or the pharmaceutically acceptable salt thereof is a compound of general formula (III-1C) or a pharmaceutically acceptable salt thereof:

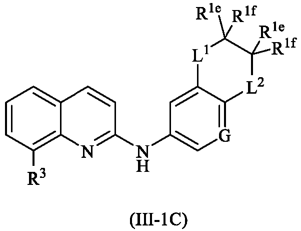

Wherein
L¹ and L² are both O atoms;
G, R³, R^{1e}, and R^{1f} are as defined in general formula (III-1).

In some embodiments of the present disclosure, the compound of general formula (II-1), general formula (II-1C), general formula (III-1), or general formula (III-1C) or the pharmaceutically acceptable salt thereof is provided, wherein R³ is selected from the group consisting of halogen, C₁₋₆ alkyl, C₁₋₆ alkoxy, and cyano.

In some embodiments of the present disclosure, the compound of general formula (II-1), general formula (II-1C), general formula (III-1), or general formula (III-1C) or the pharmaceutically acceptable salt thereof is provided, wherein R³ is halogen or C₁₋₆ alkyl; preferably, R³ is halogen, more preferably Cl.

In some embodiments of the present disclosure, the compound of general formula (I), general formula (I-3), general formula (I-4), general formula (II), general formula (II-1), general formula (III), or general formula (III-1) or the pharmaceutically acceptable salt thereof is provided, wherein R⁴ is hydrogen atom or 3- to 8-membered heterocyclyl, wherein the 3- to 8-membered heterocyclyl is substituted with one or more identical or different substituents selected from the group consisting of hydroxy and carboxyl; preferably, R⁴ is

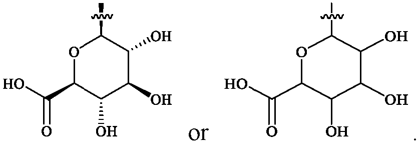

In some embodiments of the present disclosure, the compound of general formula (I), general formula (I-3), general formula (I-4), general formula (II), general formula (II-1), general formula (III), or general formula (III-1) or the pharmaceutically acceptable salt thereof is provided, wherein R⁴ is hydrogen atom.

In some embodiments of the present disclosure, the compound of general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1), or general formula (III-1C) or the pharmaceutically acceptable salt thereof is provided, wherein R^{1a} is selected from the group consisting of hydrogen atom, C₁₋₆ alkyl, and -C(O)R⁸, wherein R⁸ is C₁₋₆ alkyl; preferably, R^{1a} is methyl or acetyl.

In some embodiments of the present disclosure, the compound of general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1), or general formula (III-1C) or the pharmaceutically acceptable salt thereof is provided, wherein R^{1b} and R^{1c} or R^{1e} and R^{1f} are identical or different and are each independently selected from the group consisting of hydrogen atom, deuterium atom, halogen, and C₁₋₆ alkyl; preferably, R^{1b} and R^{1c} or R^{1e} and R^{1f} are identical or different and are each independently selected from the group consisting of hydrogen atom, deuterium atom, fluorine, and methyl.

In some embodiments of the present disclosure, the compound of general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1), or general formula (III-1C) or the pharmaceutically acceptable salt thereof is provided, wherein R^{1b} and R^{1c} or R^{1e} and R^{1f} are identical or different and are each independently selected from the group consisting of hydrogen atom, halogen, and C₁₋₆ alkyl; preferably, R^{1e} is hydrogen atom or halogen; R^{1f} is hydrogen atom or halogen.

In some embodiments of the present disclosure, the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1), or general formula (III-1C) or the pharmaceutically acceptable salt thereof is provided, wherein G is CR^{2a}; R^{2a} is as defined in general formula (I).

In some embodiments of the present disclosure, the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1), or general formula (III-1C) or the pharmaceutically acceptable salt thereof is provided, wherein R^{2a} is selected from the group consisting of hydrogen atom, halogen, and C₁₋₆ alkyl; preferably, R^{2a} is hydrogen atom.

In some embodiments of the present disclosure, the compound of general formula (I) or the pharmaceutically acceptable salt thereof is provided, wherein ring A is 3- to 8-membered cycloalkyl or 3- to 8-membered heterocyclyl; G is N atom or CR^{2a}; R^{2a} is selected from the group consisting of hydrogen atom, halogen, and C₁₋₆ alkyl; each R¹ is identical or different and is independently selected from the group consisting of hydrogen atom, deuterium atom, halogen, -C(O)R⁸, C₁₋₆ alkyl, C₁₋₆ alkoxy, and oxo; R⁸ is C₁₋₆ alkyl; n is 0 (i.e., R¹ is hydrogen atom), 1, 2, 3, or 4; each R² is identical or different and is independently selected from the group consisting of hydrogen atom, halogen, and C₁₋₆ alkyl; m is 0 (i.e., R² is hydrogen atom), 1, or 2; each R³ is identical or different and is independently selected from the group consisting of halogen, C₁₋₆ alkyl, C₁₋₆ alkoxy, and cyano; p is 1, 2, or 3; R⁴ is hydrogen atom or 3- to 8-membered heterocyclyl, wherein the 3- to 8-membered heterocyclyl is substituted with one or more identical or different substituents selected from the group consisting of hydroxy and carboxyl.

In some embodiments of the present disclosure, the compound of general formula (I) or the pharmaceutically acceptable salt thereof is provided, wherein ring A is 5- or 6-membered cycloalkyl or 5- or 6-membered heterocyclyl; G is CR^{2a}; R^{2a} is hydrogen atom; each R¹ is identical or different and is independently selected from the group consisting of hydrogen atom, deuterium atom, halogen, -C(O)R⁸, C₁₋₆ alkyl, and C₁₋₆ alkoxy; R⁸ is C₁₋₆ alkyl; n is 0 (i.e., R¹ is hydrogen atom), 1, 2, 3, or 4; each R² is hydrogen atom; each R³ is identical or different and is independently selected from the group consisting of halogen, C₁₋₆ alkyl, C₁₋₆ alkoxy, and cyano; p is 1, 2, or 3; R⁴ is hydrogen atom or 3- to 8-membered heterocyclyl, wherein the 3- to 8-membered heterocyclyl is substituted with one or more identical or different substituents selected from the group consisting of hydroxy and carboxyl.

In some embodiments of the present disclosure, the compound of general formula (II) or the pharmaceutically acceptable salt thereof is provided, wherein 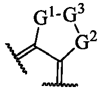 is selected from the group consisting of 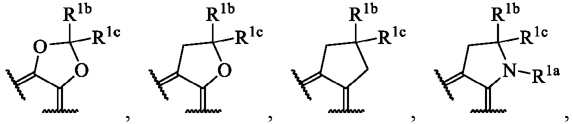 and 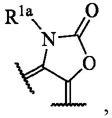 wherein R^{1a} is selected from the group consisting of hydrogen atom, C₁₋₆ alkyl, and -C(O)R⁸, and R⁸ is C₁₋₆ alkyl; R^{1b} and R^{1c} are identical or different and are each independently selected from the group consisting of hydrogen atom, deuterium atom, halogen, and C₁₋₆ alkyl; G is N atom or CR^{2a}; R^{2a} is hydrogen atom; m is 0; 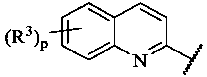 is 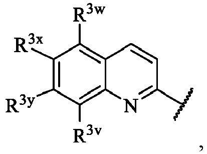 wherein R^{3v} is halogen, R^{3w}, R^{3x}, and R^{3y} are identical or different and are each independently selected from the group consisting of hydrogen atom, halogen, C₁₋₆ alkyl, C₁₋₆ alkoxy, cyano, 3- to 8-membered cycloalkyl, and 3- to 8-membered heterocyclyl, or R^{3w} and R^{3x}, together with the carbon atom to which they are attached, form 5- or 6-membered cycloalkyl; R⁴ is hydrogen atom or 3-to 8-membered heterocyclyl, wherein the 3- to 8-membered heterocyclyl is substituted with one or more identical or different substituents selected from the group consisting of hydroxy and carboxyl.

In some embodiments of the present disclosure, the compound of general formula (III) or the pharmaceutically acceptable salt thereof is provided, wherein 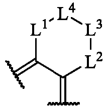 is 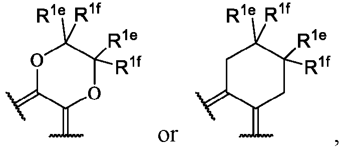 wherein R^{1e} and R^{1f} are identical or different and are each independently selected from the group consisting of hydrogen atom, deuterium atom, halogen, and C₁₋₆ alkyl; G is N atom or CR^{2a}; R^{2a} is hydrogen atom; m is 0; 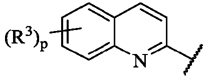 is 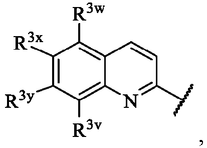 wherein R^{3v} is halogen, R^{3w}, R^{3x}, and R^{3y} are identical or different and are each independently selected from the group consisting of hydrogen atom, halogen, C₁₋₆ alkyl, C₁₋₆ alkoxy, cyano, 3- to 6-membered cycloalkyl, and 3- to 6-membered heterocyclyl; R⁴ is hydrogen atom or 3- to 8-membered heterocyclyl, wherein the 3- to 8-membered heterocyclyl is substituted with one or more identical or different substituents selected from the group consisting of hydroxy and carboxyl.

In some embodiments of the present disclosure, the compound of general formula (I-3) or general formula (I-4) or the pharmaceutically acceptable salt thereof is provided, wherein ring B is 5- or 6-membered cycloalkyl; ring A is 5- or 6-membered cycloalkyl or 5- or 6-membered heterocyclyl; G is CR^{2a}; R^{2a} is hydrogen atom; each R¹ is identical or different and is independently selected from the group consisting of hydrogen atom, deuterium atom, halogen, -C(O)R⁸, C₁₋₆ alkyl, and C₁₋₆ alkoxy; R⁸ is C₁₋₆ alkyl; n is 0 (i.e., R¹ is hydrogen atom), 1, 2, 3, or 4; R² is hydrogen atom; each R^{3a} is identical or different and is independently selected from the group consisting of halogen, C₁₋₆ alkyl, C₁₋₆ alkoxy, and cyano; r is 0, 1, or 2; R⁴ is hydrogen atom or 3- to 8-membered heterocyclyl, wherein the 3- to 8-membered heterocyclyl is substituted with one or more identical or different substituents selected from the group consisting of hydroxy and carboxyl.

**Table A. Typical compounds of the present disclosure include, but are not limited to:**

| Compound No. | Compound structure | Name |
|---|---|---|
| 1 | 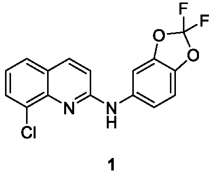 | 8-chloro-*N*-(2,2-difluorobenzo[*d*][1,3]dio xol-5-yl)quinolin-2-amine **1** |
| 2 | 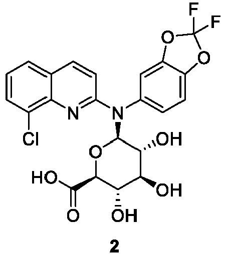 | (2*S*,3*S*,4*S*,5*R*,6*R*)-6-((8-chloroquinolin-2-yl)(2,2-difluorobenzo[*d*][1,3]dioxol-5-yl) amino)-3,4,5-trihydroxytetrahydro-2*H*-p yran-2-carboxylic acid **2** |
| | 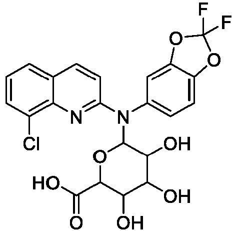 | 6-((8-chloroquinolin-2-yl)(2,2-difluorobe nzo[*d*][1,3]dioxol-5-yl)amino)-3,4,5-trih ydroxytetrahydro-2*H*-pyran-2-carboxylic acid |
| 3 | 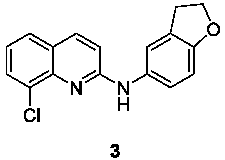 | 8-chloro-*N*-(2,3-dihydrobenzofuran-5-yl) quinolin-2-amine **3** |
| 4 | 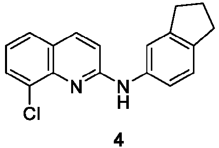 | 8-chloro-*N*-(2,3-dihydro-1*H*-inden-5-yl)q uinolin-2-amine **4** |
| 5 | 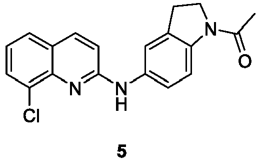 | 1-(5-((8-chloroquinolin-2-yl)amino)indol in-1-yl)ethan-1-one **5** |
| 6 | 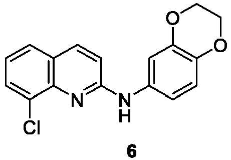 | 8-chloro-*N*-(2,3-dihydrobenzo[*b*][1,4]dio xin-6-yl)quinolin-2-amine **6** |
| 7 | 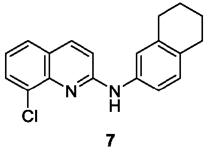 | 8-chloro-*N*-(5,6,7,8-tetrahydronaphthalen -2-yl)quinolin-2-amine **7** |
| 8 | 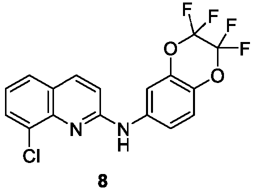 | 8-chloro-*N*-(2,2,3,3-tetrafluoro-2,3-dihyd robenzo[*b*][1,4]dioxin-6-yl)quinolin-2-a mine **8** |
| 9 | 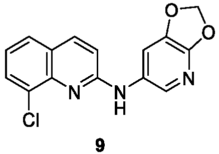 | *N*-(8-chloroquinolin-2-yl)-[1,3]dioxolo[4 ,5-*b*]pyridin-6-amine **9** |
| 10 | 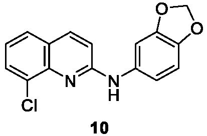 | *N*-(benzo[*d*][1,3]dioxol-5-yl)-8-chloroqui nolin-2-amine **10** |
| 11 | 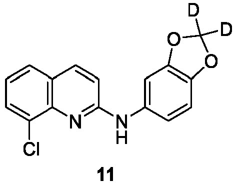 | *N*-(benzo[*d*][1,3]dioxol-5-yl-2,2-*d*₂)-8-ch loroquinolin-2-amine **11** |
| 12 | 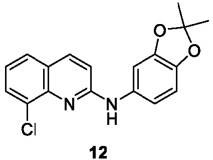 | 8-chloro-*N*-(2,2-dimethylbenzo[*d*][1,3]di oxol-5-yl)quinolin-2-amine **12** |
| 13 | 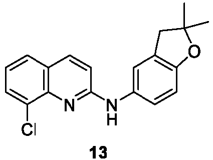 | 8-chloro-*N*-(2,2-dimethyl-2,3-dihydroben zofuran-5-yl)quinolin-2-amine **13** |
| 14 | 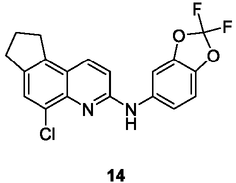 | 5-chloro-*N*-(2,2-difluorobenzo[*d*][1,3]dio xol-5-yl)-8,9-dihydro-7*H*-cyclopenta[*f*]q uinolin-3-amine **14** |
| 15 | 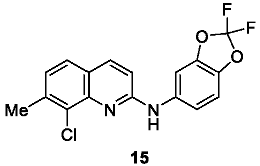 | 8-chloro-*N*-(2,2-difluorobenzo[*d*][1,3]dio xol-5-yl)-7-methylquinolin-2-amine **15** |
| 16 | 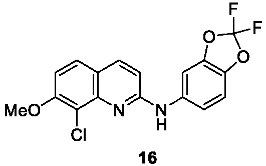 | 8-chloro-*N*-(2,2-difluorobenzo[*d*][1,3]dio xol-5-yl)-7-methoxyquinolin-2-amine **16** |
| 17 | 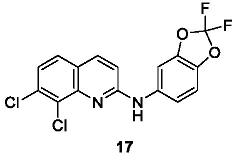 | 7,8-dichloro-*N*-(2,2-difluorobenzo[*d*][1,3 ]dioxol-5-yl)quinolin-2-amine **17** |
| 18 | 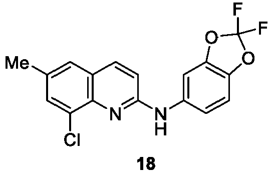 | 8-chloro-*N*-(2,2-difluorobenzo[*d*][1,3]dio xol-5-yl)-6-methylquinolin-2-amine **18** |
| 19 | 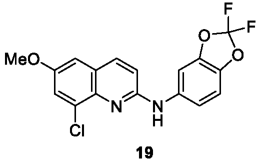 | 8-chloro-*N*-(2,2-difluorobenzo[*d*][1,3]dio xol-5-yl)-6-methoxyquinolin-2-amine **19** |
| 20 | 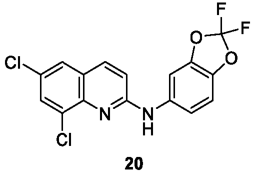 | 6,8-dichloro-*N*-(2,2-difluorobenzo[*d*][1,3 ]dioxol-5-yl)quinolin-2-amine **20** |
| 21 | 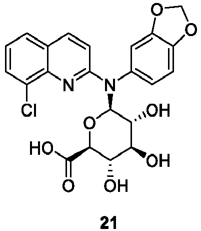 | (2*S*,3*S*,4*S*,5*R*,6*R*)-6-(benzo[*d*][1,3]dioxol -5-yl(8-chloroquinolin-2-yl)amino)-3,4,5 -trihydroxytetrahydro-2*H*-pyran-2-carbo xylic acid **21** |
| | 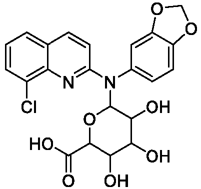 | 6-(benzo[*d*][1,3]dioxol-5-yl(8-chloroquin olin-2-yl)amino)-3,4,5-trihydroxytetrahy dro-2*H*-pyran-2-carboxylic acid |
| 22 | 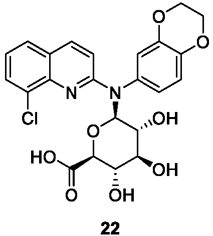 | (2*S*,3*S*,4*S*,5*R*,6*R*)-6-((8-chloroquinolin-2-yl)(2,3-dihydrobenzo[*b*][1,4]dioxin-6-yl) amino)-3,4,5-trihydroxytetrahydro-2*H*-p yran-2-carboxylic acid **22** |
| | 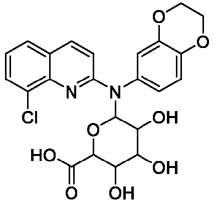 | 6-((8-chloroquinolin-2-yl)(2,3-dihydrobe nzo[*b*][1,4]dioxin-6-yl)amino)-3,4,5-trih ydroxytetrahydro-2*H*-pyran-2-carboxylic acid |
| 23 | 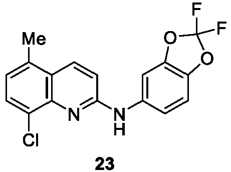 | 8-chloro-*N*-(2,2-difluorobenzo[*d*][1,3]dio xol-5-yl)-5-methylquinolin-2-amine **23** |
| 24 | 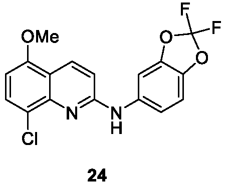 | 8-chloro-*N*-(2,2-difluorobenzo[*d*][1,3]dio xol-5-yl)-5-methoxyquinolin-2-amine **24** |
| 25 | 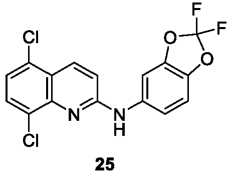 | 5,8-dichloro-*N*-(2,2-difluorobenzo[*d*][1,3 ]dioxol-5-yl)quinolin-2-amine **25** |
| 26 | 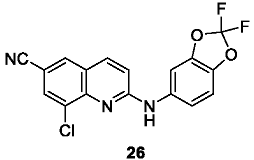 | 8-chloro-2-((2,2-difluorobenzo[*d*][1,3]di oxol-5-yl)amino)quinoline-6-carbonitrile **26** |
| 26e | 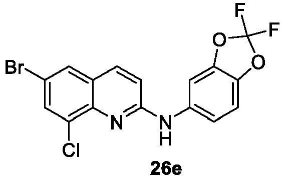 | 6-bromo-8-chloro-*N*-(2,2-difluorobenzo[ *d*][1,3]dioxol-5-yl)quinolin-2-amine **26e** |
| 27 | 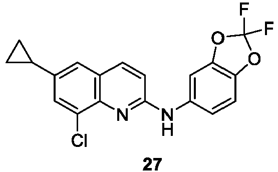 | 8-chloro-6-cyclopropyl-*N*-(2,2-difluorob enzo[*d*][1,3]dioxol-5-yl)quinolin-2-amin e**27** |
| 28 | 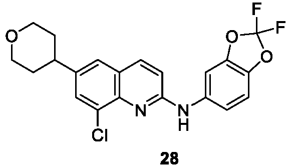 | 8-chloro-*N*-(2,2-difluorobenzo[*d*][1,3]dio xol-5-yl)-6-(tetrahydro-2*H*-pyran-4-yl)qu inolin-2-amine **28** |
| 28b | 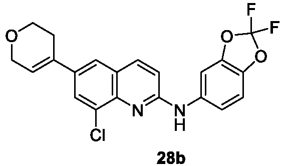 | 8-chloro-*N*-(2,2-difluorobenzo[d][1,3]dio xol-5-yl)-6-(3,6-dihydro-2*H*-pyran-4-yl) quinolin-2-amine **28b** |
| 29 | 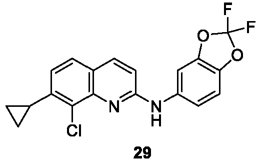 | 8-chloro-7-cyclopropyl-*N*-(2,2-difluorob enzo[*d*][1,3]dioxol-5-yl)quinolin-2-amin e**29** |
| 29e | 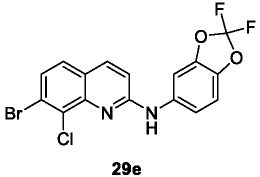 | 7-bromo-8-chloro-*N*-(2,2-difluorobenzo[ *d*][1,3]dioxol-5-yl)quinolin-2-amine **29e** |
| 30 | 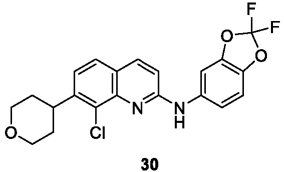 | 8-chloro-*N*-(2,2-difluorobenzo[*d*][1,3]dio xol-5-yl)-7-(tetrahydro-2*H*-pyran-4-yl)qu inolin-2-amine **30** |
| 30a | 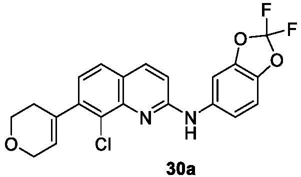 | 8-chloro-*N*-(2,2-difluorobenzo[*d*][1,3]dio xol-5-yl)-7-(3,6-dihydro-2*H*-pyran-4-yl) quinolin-2-amine **30a** |
| 31 | 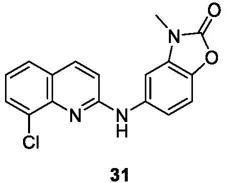 | 5-((8-chloroquinolin-2-yl)amino)-3-meth ylbenzo[*d*]oxazol-2(3*H*)-one **31** |

Another aspect of the present disclosure relates to a compound of general formula (I-1C) or (I-2C) or a salt thereof: 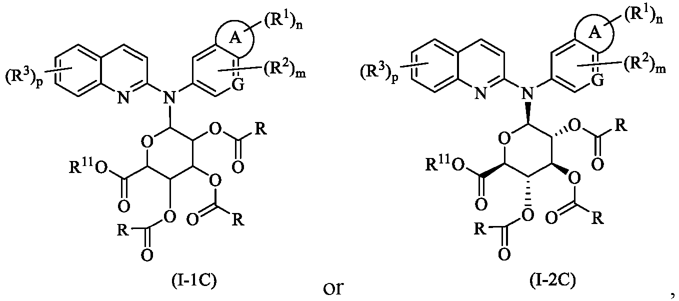 wherein
R is C₁₋₆ alkyl; R¹¹ is C₁₋₆ alkyl;
ring A, G, R¹ to R³, m, n, and p are as defined in general formula (I).

Another aspect of the present disclosure relates to a compound of general formula (IID-1A) or (IID-2A) or a salt thereof: 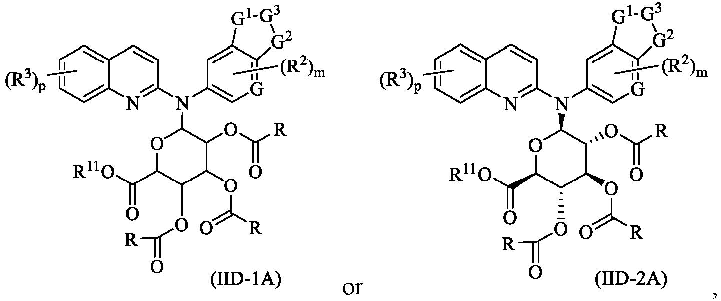 wherein
R is C₁₋₆ alkyl; R¹¹ is C₁₋₆ alkyl;
ring A, G, G¹, G², G³, R², R³, m, and p are as defined in general formula (II).

Another aspect of the present disclosure relates to a compound of general formula (IIID-1A) or (IIID-2A) or a salt thereof: 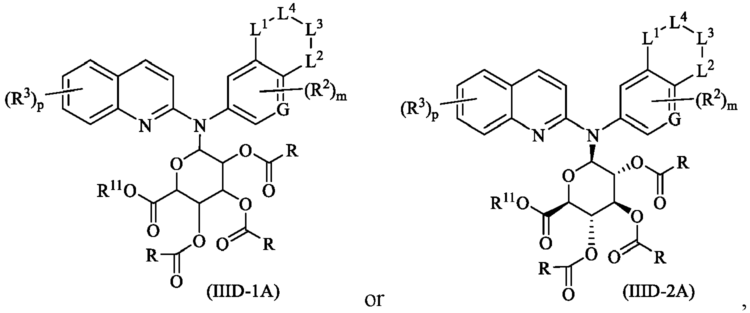 wherein
R is C₁₋₆ alkyl; R¹¹ is C₁₋₆ alkyl;
ring A, G, L¹, L², L³, L⁴, R², R³, m, and p are as defined in general formula (III).

In some embodiments of the present disclosure, the compound of general formula (I-1C), (I-2C), (IID-1A), (IID-2A), (IIID-1A), or (IIID-2A) or the pharmaceutically acceptable salt thereof is provided, wherein R is C₁₋₆ alkyl; preferably, R is methyl.

In some embodiments of the present disclosure, the compound of general formula (I-1C), (I-2C), (IID-1A), (IID-2A), (IIID-1A), or (IIID-2A) or the pharmaceutically acceptable salt thereof is provided, wherein R¹¹ is C₁₋₆ alkyl; preferably, R¹¹ is methyl.

**Table B. Typical intermediate compounds of the present disclosure include, but are not limited to:**

| Compound No. | Compound structure | Name |
|---|---|---|
| 2b | 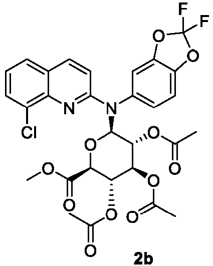 | (*2R*,3*R*,4*S*,5*S*,6*S*)-2-((8-chloroquinolin-2-yl) (2,2-difluorobenzo[*d*][1,3]dioxol-5-yl)amin o)-6-(methoxycarbonyl)tetrahydro-2*H*-pyra n-3,4,5-triyl triacetate **2b** |
| | 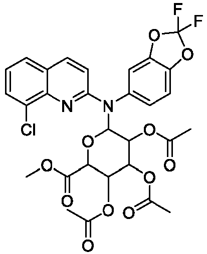 | 2-((8-chloroquinolin-2-yl)(2,2-difluorobenz o[*d*][1,3]dioxol-5-yl)amino)-6-(methoxycar bonyl)tetrahydro-2*H*-pyran-3,4,5-triyl triacetate |
| 21a | 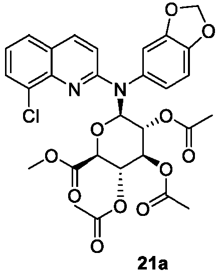 | (2*R*,3*R*,4*S*,5*S*,6*S*)-2-(benzo[*d*][1,3]dioxol-5-yl(8-chloroquinolin-2-yl)amino)-6-(methox ycarbonyl)tetrahydro-2*H*-pyran-3,4,5-triyl triacetate **21a** |
| | 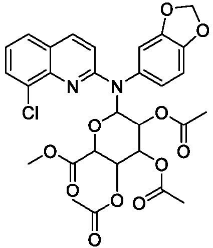 | 2-(benzo[*d*][1,3]dioxol-5-yl(8-chloroquinoli n-2-yl)amino)-6-(methoxycarbonyl)tetrahyd ro-2*H*-pyran-3,4,5-triyl triacetate |
| 22a | 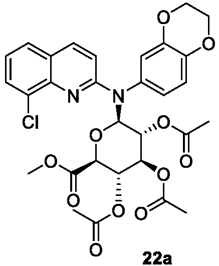 | (2*R*,3*R*,4*S*,5*S*,6*S*)-2-((8-chloroquinolin-2-yl) (2,3-dihydrobenzo[*b*][1,4]dioxin-6-yl)amino )-6-(methoxycarbonyl)tetrahydro-2*H*-pyran-3,4,5-triyl triacetate **22a** |
| | 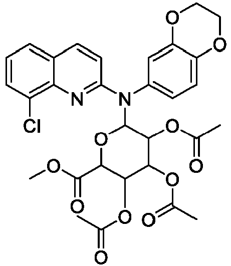 | 2-((8-chloroquinolin-2-yl)(2,3-dihydrobenz o[*b*][1,4]dioxin-6-yl)amino)-6-(methoxycar bonyl)tetrahydro-2*H*-pyran-3,4,5-triyl triacetate |

Another aspect of the present disclosure relates to a method for preparing a compound of general formula (I) or a pharmaceutically acceptable salt thereof, which comprises: 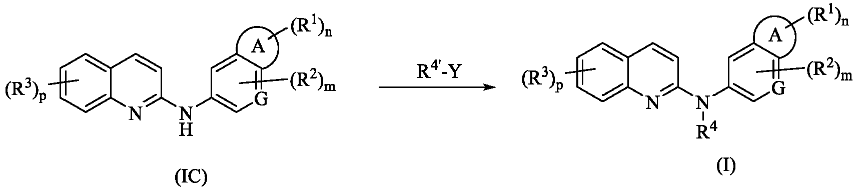
reacting a compound of general formula (IC) or a pharmaceutically acceptable salt thereof with an R^{4'}-Y compound, and then removing the protecting group on R^{4'} to give the compound of general formula (I) or the pharmaceutically acceptable salt thereof, wherein
Y is halogen, preferably Br atom;
R^{4'} is 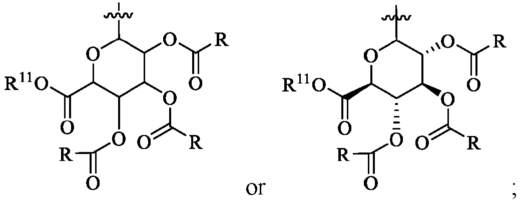
R is C₁₋₆ alkyl; R¹¹ is C₁₋₆ alkyl;
R⁴ is 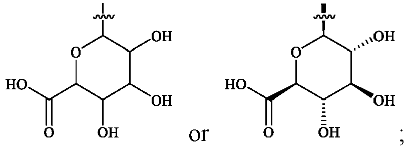
ring A, G, R¹ to R³, m, n, and p are as defined in general formula (I).

Another aspect of the present disclosure relates to a method for preparing a compound of general formula (IC) or a pharmaceutically acceptable salt thereof, which comprises: 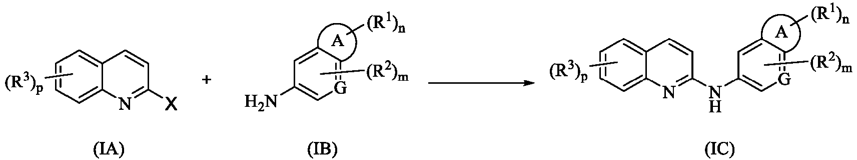
reacting a compound of general formula (IA) or a salt thereof with a compound of general formula (IB) or a salt thereof to give the compound of general formula (IC) or the pharmaceutically acceptable salt thereof,
wherein
X is halogen, preferably Cl atom;
ring A, G, R¹ to R³, m, n, and p are as defined in general formula (IC).

Another aspect of the present disclosure relates to a method for preparing a compound of general formula (I-1) or a pharmaceutically acceptable salt thereof, which comprises: 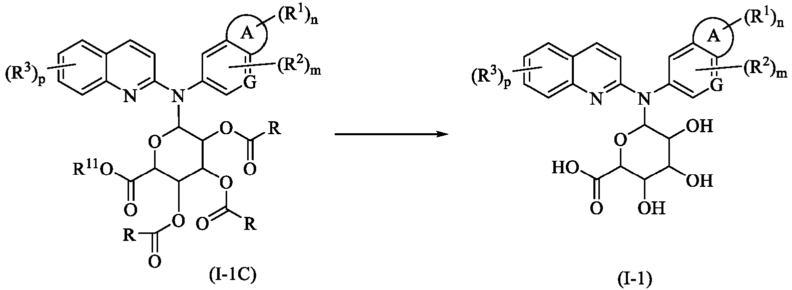
conducting an ester hydrolysis reaction of a compound of general formula (I-1C) or a salt thereof to give the compound of general formula (I-1) or the pharmaceutically acceptable salt thereof,
wherein
R and R¹¹ are as defined in general formula (I-1C);
ring A, G, R¹ to R³, m, n, and p are as defined in general formula (I-1).

Another aspect of the present disclosure relates to a method for preparing a compound of general formula (I-2) or a pharmaceutically acceptable salt thereof, which comprises: 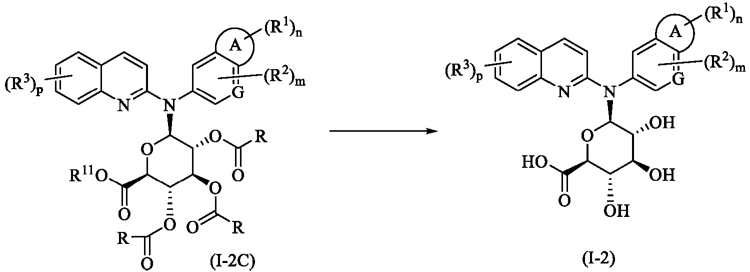
conducting an ester hydrolysis reaction of a compound of general formula (I-2C) or a salt thereof to give the compound of general formula (I-2) or the pharmaceutically acceptable salt thereof,
wherein
R and R¹¹ are as defined in general formula (I-2C);
ring A, G, R¹ to R³, m, n, and p are as defined in general formula (I-2).

Another aspect of the present disclosure relates to a method for preparing a compound of general formula (I-3) or a pharmaceutically acceptable salt thereof, which comprises: 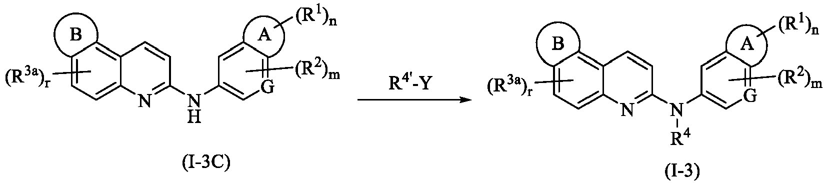
reacting a compound of general formula (I-3C) or a pharmaceutically acceptable salt thereof with an R^{4'}-Y compound, and then removing the protecting group on R^{4'} to give the compound of general formula (I-3) or the pharmaceutically acceptable salt thereof, wherein
Y is halogen, preferably Br atom;
R^{4'} is 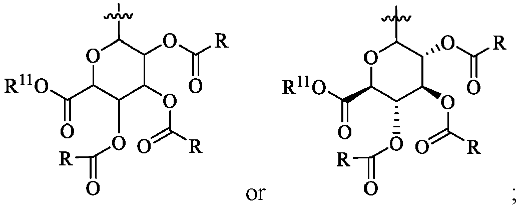
R is C₁₋₆ alkyl; R¹¹ is C₁₋₆ alkyl;
R⁴ is 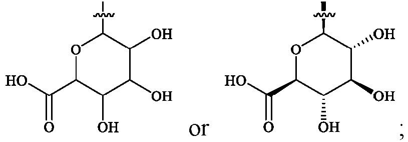
ring A, ring B, G, R¹, R², R^{3a}, m, n, and r are as defined in general formula (I-3).

Another aspect of the present disclosure relates to a method for preparing a compound of general formula (I-3C) or a pharmaceutically acceptable salt thereof, which comprises: 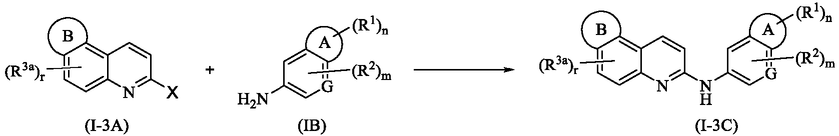
reacting a compound of general formula (I-3A) or a salt thereof with a compound of general formula (IB) or a salt thereof to give the compound of general formula (I-3C) or the pharmaceutically acceptable salt thereof,
wherein
X is halogen, preferably Cl atom;
ring A, ring B, G, R¹, R², R^{3a}, m, n, and r are as defined in general formula (I-3C).

Another aspect of the present disclosure relates to a method for preparing a compound of general formula (I-4) or a pharmaceutically acceptable salt thereof, which comprises: 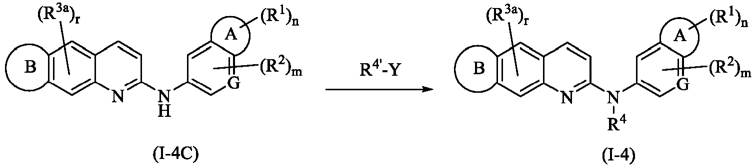
reacting a compound of general formula (I-4C) or a pharmaceutically acceptable salt thereof with an R^{4'}-Y compound, and then removing the protecting group on R^{4'} to give the compound of general formula (I-4) or the pharmaceutically acceptable salt thereof, wherein
Y is halogen, preferably Br atom;
R^{4'} is 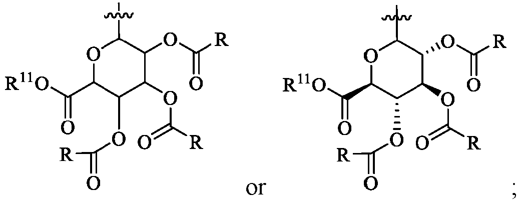
R is C₁₋₆ alkyl; R¹¹ is C₁₋₆ alkyl;
R⁴ is 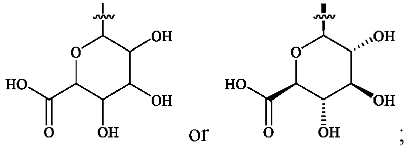
ring A, ring B, G, R¹, R², R^{3a}, m, n, and r are as defined in general formula (I-4). Another aspect of the present disclosure relates to a method for preparing a compound of general formula (I-4C) or a pharmaceutically acceptable salt thereof, which comprises: 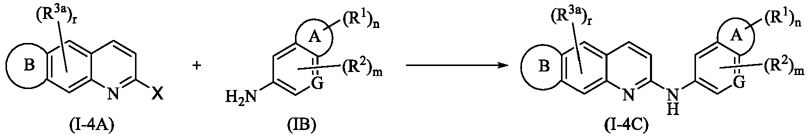
reacting a compound of general formula (I-4A) or a salt thereof with a compound of general formula (IB) or a salt thereof to give the compound of general formula (I-4C) or the pharmaceutically acceptable salt thereof,
wherein
X is halogen, preferably Cl atom;
ring A, ring B, G, R¹, R², R^{3a}, m, n, and r are as defined in general formula (I-4C).

Another aspect of the present disclosure relates to a method for preparing a compound of general formula (II) or a pharmaceutically acceptable salt thereof, which comprises: 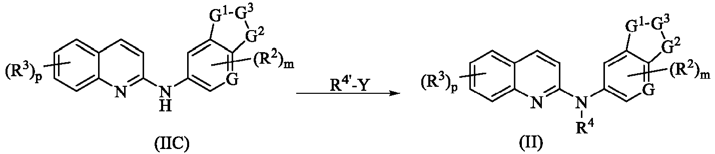
reacting a compound of general formula (IIC) or a pharmaceutically acceptable salt thereof with an R^{4'}-Y compound, and then removing the protecting group on R^{4'} to give the compound of general formula (II) or the pharmaceutically acceptable salt thereof, wherein
Y is halogen, preferably Br atom;
R^{4'} is 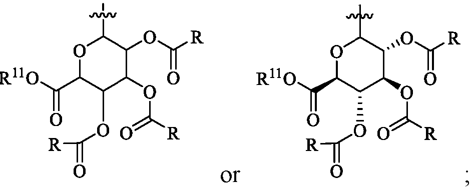
R is C₁₋₆ alkyl; R¹¹ is C₁₋₆ alkyl;
R⁴ is 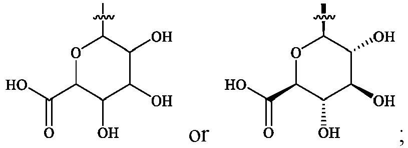
G, G¹, G², G³, R², R³, m, and p are as defined in general formula (II).

Another aspect of the present disclosure relates to a method for preparing a compound of general formula (IIC) or a pharmaceutically acceptable salt thereof, which comprises: 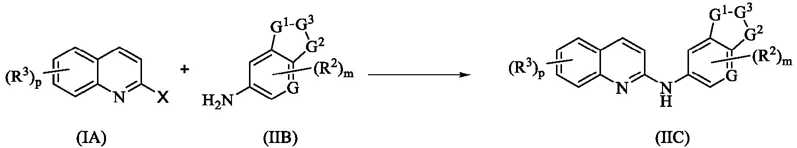
reacting a compound of general formula (IA) or a salt thereof with a compound of general formula (IIB) or a salt thereof to give the compound of general formula (IIC) or the pharmaceutically acceptable salt thereof,
wherein
X is halogen, preferably Cl atom;
G, G¹, G², G³, R², R³, m, and p are as defined in general formula (IIC).

Another aspect of the present disclosure relates to a method for preparing a compound of general formula (IID-1) or a pharmaceutically acceptable salt thereof, which comprises: 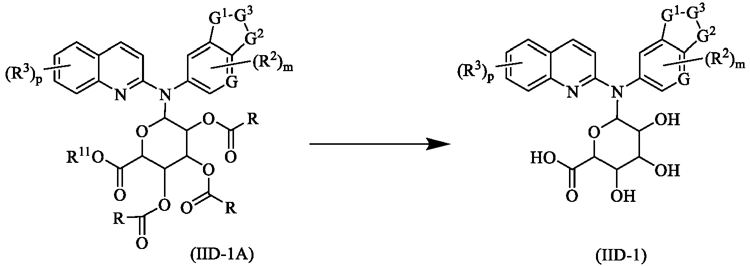
conducting an ester hydrolysis reaction of a compound of general formula (IID-1A) or a salt thereof to give the compound of general formula (IID-1) or the pharmaceutically acceptable salt thereof,
wherein
R is C₁₋₆ alkyl; R¹¹ is C₁₋₆ alkyl;
G, G¹, G², G³, R², R³, m, and p are as defined in general formula (IID-1).

Another aspect of the present disclosure relates to a method for preparing a compound of general formula (IID-2) or a pharmaceutically acceptable salt thereof, which comprises: 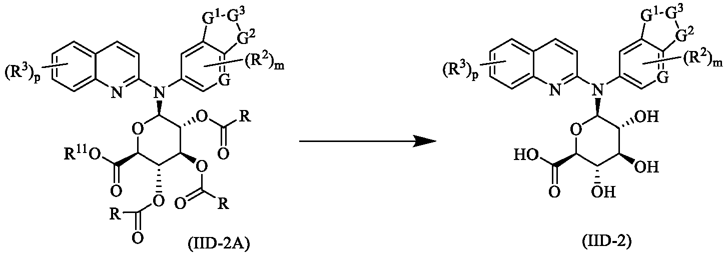
conducting an ester hydrolysis reaction of a compound of general formula (IID-2A) or a salt thereof to give the compound of general formula (IID-2) or the pharmaceutically acceptable salt thereof,
wherein
R is C₁₋₆ alkyl; R¹¹ is C₁₋₆ alkyl;
G, G¹, G², G³, R², R³, m, and p are as defined in general formula (IID-2).

Another aspect of the present disclosure relates to a method for preparing a compound of general formula (II-1) or a pharmaceutically acceptable salt thereof, which comprises: 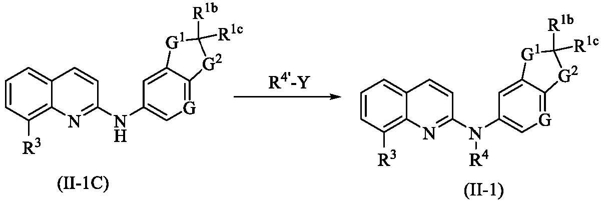
reacting a compound of general formula (II-1C) or a pharmaceutically acceptable salt thereof with an R^{4'}-Y compound, and then removing the protecting group on R^{4'} to give the compound of general formula (II-1) or the pharmaceutically acceptable salt thereof, wherein
Y is halogen, preferably Br atom;
R^{4'} is 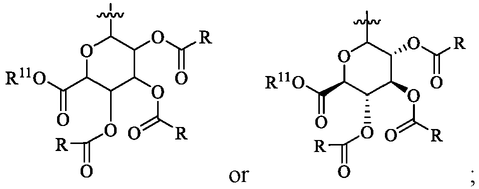
R is C₁₋₆ alkyl; R¹¹ is C₁₋₆ alkyl;
R⁴ is 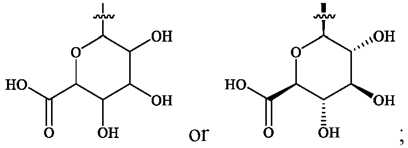
G, G¹, G², R^{1b}, R^{1c}, and R³ are as defined in general formula (II-1); preferably, R³ is halogen.

Another aspect of the present disclosure relates to a method for preparing a compound of general formula (II-1C) or a pharmaceutically acceptable salt thereof, which comprises: 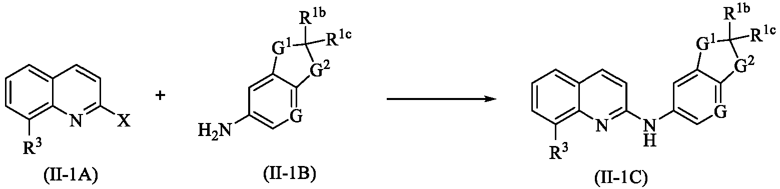
reacting a compound of general formula (II-1A) or a salt thereof with a compound of general formula (II-1B) or a salt thereof to give the compound of general formula (II-1C) or the pharmaceutically acceptable salt thereof,
wherein
X is halogen, preferably Cl atom;
G, G¹, G², R^{1b}, R^{1c}, and R³ are as defined in general formula (II-1C); preferably, R³ is halogen.

Another aspect of the present disclosure relates to a method for preparing a compound of general formula (III) or a pharmaceutically acceptable salt thereof, which comprises: 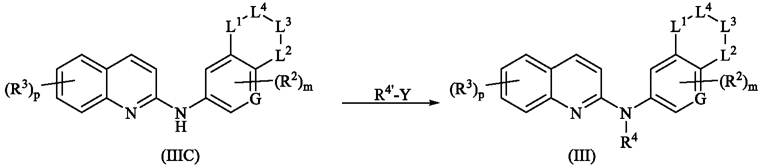
reacting a compound of general formula (IIIC) or a pharmaceutically acceptable salt thereof with an R^{4'}-Y compound, and then removing the protecting group on R^{4'} to give the compound of general formula (III) or the pharmaceutically acceptable salt thereof, wherein
Y is halogen, preferably Br atom;
R^{4'} is 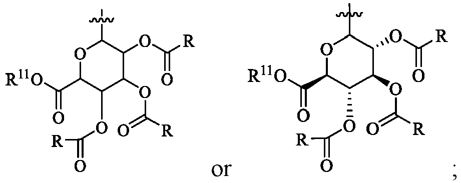
R is C₁₋₆ alkyl; R¹¹ is C₁₋₆ alkyl;
R⁴ is 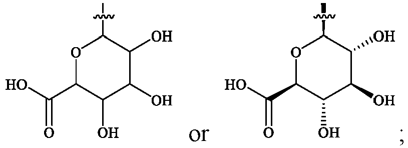
G, L¹, L², L³, L⁴, R², R³, m, and p are as defined in general formula (III).

Another aspect of the present disclosure relates to a method for preparing a compound of general formula (IIIC) or a pharmaceutically acceptable salt thereof, which comprises: 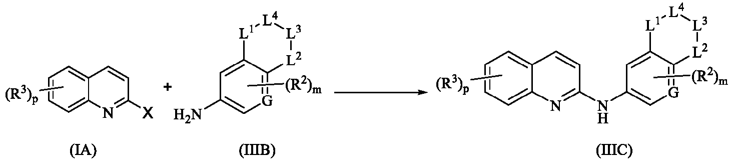
reacting a compound of general formula (IA) or a salt thereof with a compound of general formula (IIIB) or a salt thereof to give the compound of general formula (IIIC) or the pharmaceutically acceptable salt thereof,
wherein
X is halogen, preferably Cl atom;
G, L¹, L², L³, L⁴, R², R³, m, and p are as defined in general formula (IIIC).

Another aspect of the present disclosure relates to a method for preparing a compound of general formula (IIID-1) or a pharmaceutically acceptable salt thereof, which comprises: 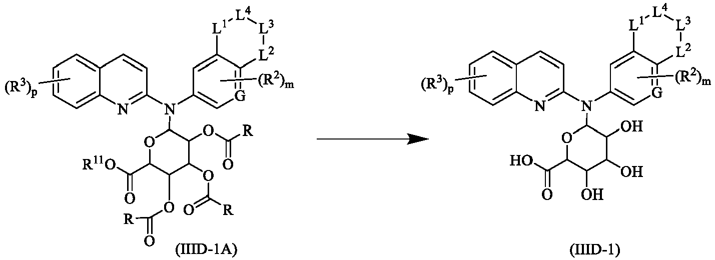
conducting an ester hydrolysis reaction of a compound of general formula (IIID-1A) or a salt thereof to give the compound of general formula (IIID-1) or the pharmaceutically acceptable salt thereof,
wherein
R is C₁₋₆ alkyl; R¹¹ is C₁₋₆ alkyl;
G, L¹, L², L³, L⁴, R², R³, m, and p are as defined in general formula (IIID-1).

Another aspect of the present disclosure relates to a method for preparing a compound of general formula (IIID-2) or a pharmaceutically acceptable salt thereof, which comprises: 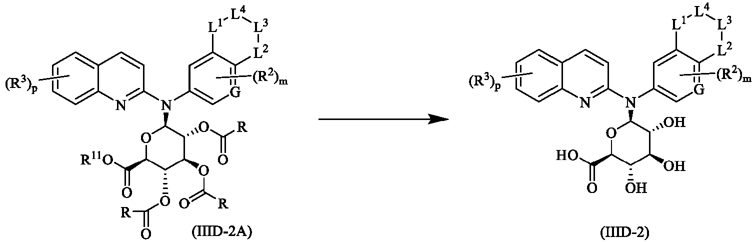
conducting an ester hydrolysis reaction of a compound of general formula (IIID-2A) or a salt thereof to give the compound of general formula (IIID-2) or the pharmaceutically acceptable salt thereof,
wherein
R is C₁₋₆ alkyl; R¹¹ is C₁₋₆ alkyl;
G, L¹, L², L³, L⁴, R², R³, m, and p are as defined in general formula (IIID-2).

Another aspect of the present disclosure relates to a method for preparing a compound of general formula (III-1) or a pharmaceutically acceptable salt thereof, which comprises: 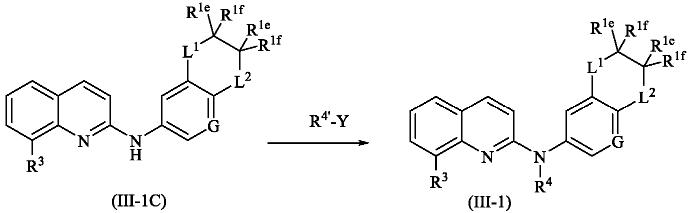
reacting a compound of general formula (III-1C) or a pharmaceutically acceptable salt thereof with an R^{4'}-Y compound, and then removing the protecting group on R^{4'} to give the compound of general formula (III-1) or the pharmaceutically acceptable salt thereof, wherein
Y is halogen, preferably Br atom;
R^{4'} is 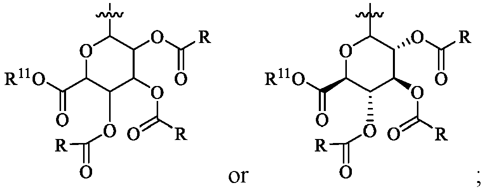
R is C₁₋₆ alkyl; R¹¹ is C₁₋₆ alkyl;
R⁴ is 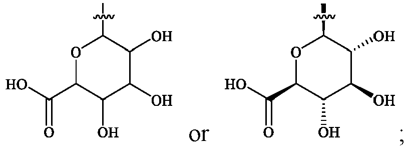
G, L¹, L², R^{1e}, R^{1f}, and R³ are as defined in general formula (III-1); preferably, R³ is halogen.

Another aspect of the present disclosure relates to a method for preparing a compound of general formula (III-1C) or a pharmaceutically acceptable salt thereof, which comprises: 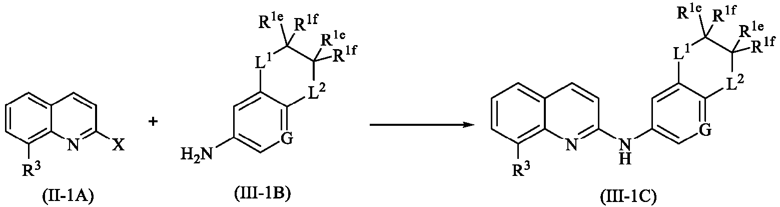
reacting a compound of general formula (II-1A) or a salt thereof with a compound of general formula (III-1B) or a salt thereof to give the compound of general formula (III-1C) or the pharmaceutically acceptable salt thereof,
wherein
X is halogen, preferably Cl atom;
G, L¹, L², R^{1e}, R^{1f}, and R³ are as defined in general formula (III-1C); preferably, R³ is halogen.

Another aspect of the present disclosure relates to a pharmaceutical composition, comprising a therapeutically effective amount of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) of the present disclosure and a compound shown in Table A or a pharmaceutically acceptable salt thereof, and one or more pharmaceutically acceptable carriers, diluents or excipients.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in preparing a medicament for regulating the level of a miRNA, wherein preferably, the miRNA is miR-124.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in preparing a medicament for treating a disease or condition that is ameliorated by regulating the level of a miRNA.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in preparing a medicament for treating and/or preventing a disease or condition, wherein the disease or condition is selected from the group consisting of a viral infection, an inflammation, and a cancer.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in preparing a medicament for treating and/or preventing AIDS or an AIDS-related condition or human immunodeficiency virus (HIV).

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in preparing a medicament for treating and/or preventing a disease or condition, wherein the disease or condition is an inflammation selected from the group consisting of an autoimmune-related inflammatory disease, an inflammatory disease in the central nervous system (CNS), an inflammatory disease in the joints, an inflammatory disease in the digestive tract, an inflammatory disease in the skin, other inflammatory diseases related to epithelial cells, a cancer-related inflammation, an irritation-related inflammation, and an injury-related inflammation.

The present disclosure further relates to a compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or a pharmaceutical composition comprising same for use as a medicament for treating and/or preventing a disease or condition, wherein the disease or condition is selected from the group consisting of a viral infection, an inflammation, and a cancer, wherein the inflammation is selected from the group consisting of inflammatory bowel disease, rheumatoid arthritis, multiple sclerosis, Alzheimer's disease, Parkinson's disease, osteoarthritis, atherosclerosis, ankylosing spondylitis, psoriasis, dermatitis, systemic lupus erythematosus, Sjogren's syndrome, bronchitis, asthma, and a colon cancer-related inflammation; preferably, the inflammation is inflammatory bowel disease.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in preparing a medicament for treating and/or preventing a disease or condition, wherein the disease or condition is a cancer selected from the group consisting of leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous carcinoma, papillary carcinoma, cystadenocarcinoma, medullary carcinoma, bronchogenic carcinoma, liver cancer, cholangiocarcinoma, choriocarcinoma, seminoma, embryonal carcinoma, Wilms' tumor, cervical cancer, uterine cancer, testicular cancer, lung cancer, bladder cancer, neuroglioma, medulloblastoma, craniopharyngioma, ependymoma, pinealoma, hemangioblastoma, acoustic neuroma, schwannoma, neurofibroma, retinoblastoma, melanoma, skin cancer, kidney cancer, nasopharyngeal cancer, gastric cancer, esophageal cancer, head and neck cancer, colorectal cancer, small intestine cancer, gallbladder cancer, pediatric tumor, urothelial cancer, ureteral tumor, thyroid cancer, osteoma, neuroblastoma, brain tumor, and myeloma.

The present disclosure further relates to a method for regulating the level of a miRNA, comprising administering to a patient in need a therapeutically effective amount of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same, wherein preferably, the miRNA is miR-124.

The present disclosure further relates to a method for treating and/or preventing a disease or condition, comprising administering to a patient in need a therapeutically effective amount of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same, wherein the disease or condition is selected from the group consisting of a viral infection, an inflammation, and a cancer, wherein the inflammation is preferably selected from the group consisting of an autoimmune-related inflammatory disease, an inflammatory disease in the central nervous system (CNS), an inflammatory disease in the joints, an inflammatory disease in the digestive tract, an inflammatory disease in the skin, other inflammatory diseases related to epithelial cells, a cancer-related inflammation, an irritation-related inflammation, and an injury-related inflammation.

The present disclosure further relates to a compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or a pharmaceutical composition comprising same for use as a medicament for treating and/or preventing a disease or condition, wherein the disease or condition is selected from the group consisting of a viral infection, an inflammation, and a cancer, wherein the inflammation is selected from the group consisting of inflammatory bowel disease, rheumatoid arthritis, multiple sclerosis, Alzheimer's disease, Parkinson's disease, osteoarthritis, atherosclerosis, ankylosing spondylitis, psoriasis, dermatitis, systemic lupus erythematosus, Sjogren's syndrome, bronchitis, asthma, and a colon cancer-related inflammation; preferably, the inflammation is inflammatory bowel disease.

The present disclosure further relates to a method for treating and/or preventing a disease or condition, comprising administering to a patient in need a therapeutically effective amount of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same, wherein the disease or condition is selected from the group consisting of a viral infection, an inflammation, and a cancer, wherein the cancer is selected from the group consisting of leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous carcinoma, papillary carcinoma, cystadenocarcinoma, medullary carcinoma, bronchogenic carcinoma, liver cancer, cholangiocarcinoma, choriocarcinoma, seminoma, embryonal carcinoma, Wilms' tumor, cervical cancer, uterine cancer, testicular cancer, lung cancer, bladder cancer, neuroglioma, medulloblastoma, craniopharyngioma, ependymoma, pinealoma, hemangioblastoma, acoustic neuroma, schwannoma, neurofibroma, retinoblastoma, melanoma, skin cancer, kidney cancer, nasopharyngeal cancer, gastric cancer, esophageal cancer, head and neck cancer, colorectal cancer, rectal cancer, small intestine cancer, gallbladder cancer, pediatric tumor, urothelial cancer, ureteral tumor, thyroid cancer, osteoma, neuroblastoma, brain tumor, and myeloma.

The present disclosure further relates to a method for treating and/or preventing a disease or condition, comprising administering to a patient in need a therapeutically effective amount of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same, wherein the disease or condition is selected from the group consisting of AIDS or an AIDS-related condition and human immunodeficiency virus (HIV).

The present disclosure further relates to a compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or a pharmaceutical composition comprising same for use as a medicament.

The present disclosure further relates to a compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or a pharmaceutical composition comprising same for use in regulating miRNA, wherein preferably, the miRNA is miR-124.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in treating and/or preventing a disease or condition, wherein the disease or condition is selected from the group consisting of a viral infection, an inflammation, and a cancer, wherein the inflammation is preferably selected from the group consisting of an autoimmune-related inflammatory disease, an inflammatory disease in the central nervous system (CNS), an inflammatory disease in the joints, an inflammatory disease in the digestive tract, an inflammatory disease in the skin, other inflammatory diseases related to epithelial cells, a cancer-related inflammation, an irritation-related inflammation, and an injury-related inflammation.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in treating and/or preventing a disease or condition, wherein the disease or condition is selected from the group consisting of a viral infection, an inflammation, and a cancer, wherein the inflammation is selected from the group consisting of inflammatory bowel disease, rheumatoid arthritis, multiple sclerosis, Alzheimer's disease, Parkinson's disease, osteoarthritis, atherosclerosis, ankylosing spondylitis, psoriasis, dermatitis, systemic lupus erythematosus, Sjogren's syndrome, bronchitis, asthma, and a colon cancer-related inflammation; preferably, the inflammation is inflammatory bowel disease.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in treating and/or preventing a disease or condition, wherein the disease or condition is selected from the group consisting of a viral infection, an inflammation, and a cancer, wherein the inflammation is inflammatory bowel disease, wherein the inflammatory bowel disease is ulcerative colitis (UC) or Crohn's disease (CD).

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in treating and/or preventing a disease or condition, wherein the disease or condition is selected from the group consisting of a viral infection, an inflammation, and a cancer, wherein the cancer is selected from the group consisting of leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous carcinoma, papillary carcinoma, cystadenocarcinoma, medullary carcinoma, bronchogenic carcinoma, liver cancer, cholangiocarcinoma, choriocarcinoma, seminoma, embryonal carcinoma, Wilms' tumor, cervical cancer, uterine cancer, testicular cancer, lung cancer, bladder cancer, neuroglioma, astrocytoma, medulloblastoma, craniopharyngioma, ependymoma, pinealoma, hemangioblastoma, acoustic neuroma, schwannoma, neurofibroma, retinoblastoma, melanoma, skin cancer, kidney cancer, nasopharyngeal cancer, gastric cancer, esophageal cancer, head and neck cancer, colorectal cancer, small intestine cancer, gallbladder cancer, pediatric tumor, urothelial cancer, ureteral tumor, thyroid cancer, osteoma, neuroblastoma, brain tumor, and myeloma.

The present disclosure further relates to use of the compound of general formula (I), general formula (IC), general formula (I-1), general formula (I-2), general formula (I-3), general formula (I-3C), general formula (I-4), general formula (I-4C), general formula (II), general formula (IIC), general formula (II-1), general formula (II-1C), general formula (III), general formula (IID-1), general formula (IID-2), general formula (IIID-1), general formula (IIID-2), general formula (IIIC), general formula (III-1C) or general formula (III-1) and a compound shown in Table A or a pharmaceutically acceptable salt thereof, or the pharmaceutical composition comprising same in treating and/or preventing AIDS or an AIDS-related condition or human immunodeficiency virus (HIV).

The disease or condition described herein is one that is treated and/or prevented by regulating the level of a miRNA, wherein preferably, the miRNA is miR-124.

Preferably, the viral infection described herein is a retroviral infection.

Preferably, the inflammatory bowel disease described herein is ulcerative colitis (UC) or Crohn's disease (CD).

Preferably, the lymphoma described herein is Hodgkin's disease or non-Hodgkin's lymphoma (e.g., mantle cell lymphoma, diffuse large B-cell lymphoma, follicular center lymphoma, marginal zone B-cell lymphoma, lymphoplasmacytic lymphoma, and peripheral T-cell lymphoma); the liver cancer is preferably hepatocellular carcinoma; the lung cancer (also known as bronchogenic lung cancer) is selected from the group consisting of non-small cell lung cancer (NSCLC) (e.g., squamous cell carcinoma) and small cell lung cancer (SCLC); the kidney cancer is selected from the group consisting of renal cell carcinoma, clear cell, and renal oncocytoma; the leukemia is selected from the group consisting of chronic lymphocytic leukemia (CLL), chronic myelogenous leukemia, acute lymphoblastic leukemia (ALL), T-cell acute lymphoblastic leukemia (T-ALL), chronic myelogenous leukemia (CML), and acute myelogenous leukemia (AML); the skin cancer is selected from the group consisting of malignant melanoma, squamous cell carcinoma, basal cell carcinoma, and angiosarcoma; the myeloma is preferably multiple myeloma; the colorectal cancer is preferably colon cancer or rectal cancer; and the neuroglioma (i.e., glioma or glioblastoma) is preferably selected from the group consisting of glioblastoma, astrocytoma, and oligodendroglioma.

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular, or subcutaneous), or administration by inhalation or insufflation. The compounds of the present disclosure may also be formulated into a dosage form, such as tablets, hard or soft capsules, aqueous or oily suspensions, emulsions, injections, dispersible powders or granules, suppositories, lozenges or syrups.

As a general guide, the active compound is preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 wt.% to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrant, a binder, and a lubricant. Such a tablet may be uncoated or may be coated by known techniques for masking the taste of the drug or delaying the disintegration and absorption of the drug in the gastrointestinal tract and thus enabling a sustained release of the drug over a longer period.

An oral formulation in a soft gelatin capsule where the active ingredient is mixed with an inert solid diluent or with a water-soluble carrier or oil vehicle may also be provided. An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more corrigents, and one or more sweeteners.

An oil suspension may be formulated by suspending the active ingredient in a vegetable oil, or in a mineral oil. The oil suspension may contain a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative, and an antioxidant. Such a formulation may also comprise a palliative, a preservative, a colorant, and an antioxidant.

The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives. As is well known to those skilled in the art, the dose of the drug administered depends on a variety of factors, including but not limited to: the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of administration, the daily dose of the compound, or the type of pharmaceutically acceptable salts, can be verified according to conventional treatment regimens.

### Description of the terms

Unless otherwise stated, the terms used in the specification and claims have the following meanings.

The term "alkyl" refers to a saturated linear or branched aliphatic hydrocarbon group, which is a linear or branched group containing 1 to 20 carbon atoms, preferably alkyl containing 1 to 12 (e.g., 1, 2, 3, 4, 5, 6, 7, 8, 9, 10, 11, and 12) carbon atoms (i.e., C₁₋₁₂ alkyl), and more preferably alkyl containing 1 to 6 carbon atoms (i.e., C₁₋₆ alkyl). Non-limiting examples of alkyl include: methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, various side-chain isomers thereof, and the like. Alkyl may be substituted or unsubstituted. When substituted, it may be substituted at any accessible point of attachment, and the substituent is preferably selected from one or more of deuterium atom, halogen, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "alkylene" refers to a saturated linear or branched aliphatic hydrocarbon group which is a residue derived from the parent alkane by removal of two hydrogen atoms from the same carbon atom or two different carbon atoms. Alkylene is a linear or branched group containing 1 to 20 carbon atoms. Alkylene preferably has 1 to 12 (e.g., 1, 2, 3, 4, 5, 6, 7, 8, 9, 10, 11 and 12) carbon atoms (i.e., C₁₋₁₂ alkylene), and more preferably has 1 to 6 carbon atoms (i.e., C₁₋₆ alkylene). Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. Alkylene may be substituted or unsubstituted. When substituted, it may be substituted at any available point of attachment, and the substituent is preferably one or more substituents independently and optionally selected from the group consisting of alkenyl, alkynyl, alkoxy, haloalkoxy, cycloalkyloxy, heterocyclyloxy, alkylthio, alkylamino, halogen, sulfhydryl, hydroxy, nitro, cyano, cycloalkyl, heterocyclyl, aryl, heteroaryl, cycloalkoxy, heterocyclooxy, cycloalkylthio, heterocyclylthio, and oxo.

The term "alkenyl" refers to an alkyl compound having at least one carbon-carbon double bond in the molecule, wherein the alkyl is as defined above. Alkenyl preferably contains 2 to 12 (e.g., 2, 3, 4, 5, 6, 7, 8, 9, 10, 11, and 12) carbon atoms and more preferably contains 2 to 6 carbon atoms (i.e., C₂₋₆ alkenyl). Alkenyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "alkynyl" refers to an alkyl compound having at least one carbon-carbon triple bond in the molecule, wherein the alkyl is as defined above. Alkynyl preferably has 2 to 12 (e.g., 2, 3, 4, 5, 6, 7, 8, 9, 10, 11 and 12) carbon atoms (i.e., C₂₋₁₂ alkynyl), and more preferably has 2 to 6 carbon atoms (i.e., C₂₋₆ alkynyl). Alkynyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 12 (e.g., 3, 4, 5, 6, 7, 8, 9, 10, 11, and 12) carbon atoms (i.e., 3- to 12-membered cycloalkyl), preferably 3 to 8 carbon atoms (i.e., 3- to 8-membered cycloalkyl), more preferably 3 to 6 carbon atoms (i.e., 3- to 6-membered cycloalkyl), and most preferably 5 or 6 carbon atoms (i.e., 5- or 6-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl and bridged cycloalkyl.

The term "spiro cycloalkyl" refers to a 5- to 20-membered polycyclic group in which monocyclic rings share one carbon atom (referred to as the spiro atom), and it may contain one or more double bonds. It is preferably 6- to 14-membered, and more preferably 7- to 10-membered (e.g., 7-membered, 8-membered, 9-membered, or 10-membered). According to the number of the spiro atoms shared among the rings, spiro cycloalkyl may be monospiro cycloalkyl or polyspiro cycloalkyl (e.g., bispiro cycloalkyl), preferably monospiro cycloalkyl and bispiro cycloalkyl, and more preferably 3-membered/5-membered, 3-membered/6-membered, 4-membered/4-membered, 4-membered/5-membered, 4-membered/6-membered, 5-membered/5-membered, 5-membered/6-membered, 6-membered/4-membered, 6-membered/5-membered, or 6-membered/6-membered monospiro cycloalkyl. Non-limiting examples of spirocycloalkyl include:

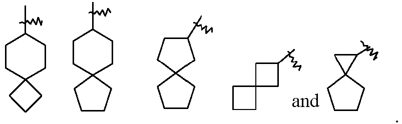

The term "fused cycloalkyl" refers to a 5- to 20-membered all-carbon polycyclic group in which each ring shares a pair of adjacent carbon atoms with the other rings in the system, wherein one or more of the rings may contain one or more double bonds. It is preferably 6- to 14-membered, and more preferably 7- to 10-membered (e.g., 7-membered, 8-membered, 9-membered, or 10-membered). According to the number of constituent rings, the fused cycloalkyl may be bicyclic, tricyclic, tetracyclic, etc., preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, 6-membered/6-membered, 6-membered/7-membered, 7-membered/5-membered or 7-membered/6-membered bicyclic alkyl. Non-limiting examples of fused cycloalkyl include:

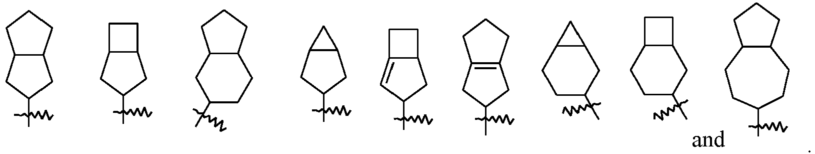

The term "bridged cycloalkyl" refers to a 5- to 20-membered all-carbon polycyclic group in which any two rings share two carbon atoms that are not directly connected, and it may contain one or more double bonds. It is preferably 6- to 14-membered, and more preferably 7- to 10-membered (e.g., 7-membered, 8-membered, 9-membered, or 10-membered). According to the number of constituent rings, bridged cycloalkyl may be polycyclic, e.g., bicyclic, tricyclic or tetracyclic, preferably bicyclic, tricyclic or tetracyclic bridged cycloalkyl, and more preferably bicyclic or tricyclic bridged cycloalkyl. Non-limiting examples of bridged cycloalkyl include:

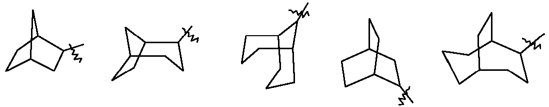

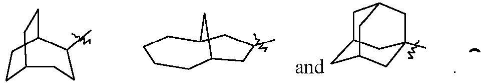

The cycloalkyl ring includes those in which the cycloalkyl described above (including monocyclic, spiro, fused and bridged rings) is fused to an aryl, heteroaryl, or heterocycloalkyl ring, wherein the ring connected to the parent structure is cycloalkyl.

Non-limiting examples include 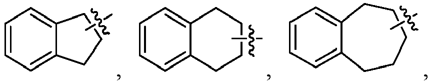 and the like, and preferably

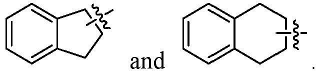

Cycloalkyl may be substituted or unsubstituted. When substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "alkoxy" refers to -O-(alkyl), wherein the alkyl is as defined above.

Non-limiting examples of alkoxy include: methoxy, ethoxy, propoxy, and butoxy. Alkoxy may be optionally substituted or unsubstituted. When it is substituted, the substituent is preferably selected from one or more of deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heterocyclyl" refers to a saturated or partially unsaturated monocyclic or polycyclic substituent containing 3 to 20 ring atoms, wherein one or more of the ring atoms are a heteroatom selected from the group consisting of nitrogen, oxygen and sulfur, the sulfur optionally being substituted with oxo (i.e., form sulfoxide or sulfone), but excluding a cyclic portion of -O-O-, -O-S- or -S-S-; and the remaining ring atoms are carbon. Preferably, heterocyclyl contains 3 to 12 (e.g., 3, 4, 5, 6, 7, 8, 9, 10, 11 and 12) ring atoms, of which 1-4 (e.g., 1, 2, 3 and 4) are heteroatoms (i.e., 3- to 12-membered heterocyclyl); more preferably, heterocyclyl contains 3 to 8 ring atoms (e.g., 3, 4, 5, 6, 7 and 8), of which 1-3 are heteroatoms (e.g., 1, 2 and 3) (i.e., 3- to 8-membered heterocyclyl); more preferably, heterocyclyl contains 3 to 6 ring atoms, of which 1-3 are heteroatoms (i.e., 3- to 6-membered heterocyclyl); most preferably, heterocyclyl contains 5 or 6 ring atoms, of which 1-3 are heteroatoms (i.e., 5- or 6-membered heterocyclyl). Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen, and sulfur, and the sulfur may optionally be substituted with oxo (i.e., to form sulfoxide or sulfone); the other ring atoms are carbon. It may have one or more double bonds. It is preferably 6- to 14-membered, and more preferably 7- to 10-membered (e.g., 7-membered, 8-membered, 9-membered or 10-membered). According to the number of spiro atoms shared among the rings, the spiro heterocyclyl may be monospiro heterocyclyl or polyspiro heterocyclyl (e.g., bispiro heterocyclyl), preferably monospiro heterocyclyl and bispiro heterocyclyl, and more preferably 3-membered/5-membered, 3-membered/6-membered, 4-membered/4-membered, 4-membered/5-membered, 4-membered/6-membered, 5-membered/5-membered, 5-membered/6-membered, or 6-membered/6-membered monospiro heterocyclyl. Non-limiting examples of spiro heterocyclyl include:

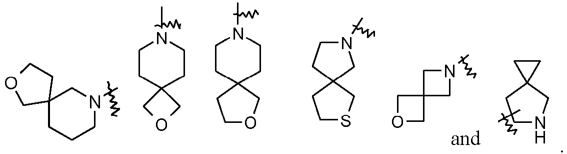

The term "fused heterocyclyl" refers to a 5- to 20-membered polycyclic heterocyclyl group in which each ring shares a pair of adjacent atoms with the other rings in the system, wherein one or more of the rings may contain one or more double bonds, wherein one or more of the ring atoms is a heteroatom selected from the group consisting of nitrogen, oxygen and sulfur, the sulfur optionally being substituted with oxo (i.e., form sulfoxide or sulfone); and the remaining ring atoms are carbon. It is preferably 6- to 14-membered, and more preferably 7- to 10-membered (e.g., 7-membered, 8-membered, 9-membered, or 10-membered). According to the number of constituent rings, the fused heterocyclyl may be polycyclic, e.g., bicyclic, tricyclic or tetracyclic, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused heterocyclyl. Non-limiting examples of fused heterocyclyl include:

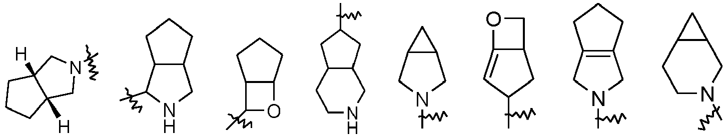

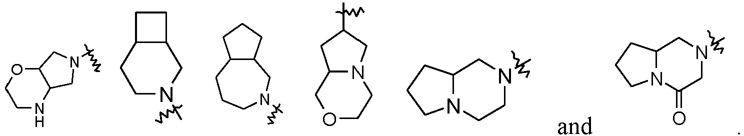

The term "bridged heterocyclyl" refers to a 5- to 14-membered polycyclic heterocyclyl group in which any two rings share two atoms that are not directly connected, and it may contain one or more double bonds, wherein one or more ring atoms are heteroatoms selected from the group consisting of nitrogen, oxygen, and sulfur, and the sulfur may optionally be substituted with oxo (i.e., to form sulfoxide or sulfone); the other ring atoms are carbon. It is preferably 6- to 14-membered, and more preferably 7-to 10-membered (e.g., 7-membered, 8-membered, 9-membered, or 10-membered). According to the number of constituent rings, the bridged heterocyclyl may be polycyclic, e.g., bicyclic, tricyclic, or tetracyclic, preferably bicyclic, tricyclic, or tetracyclic bridged heterocyclyl, and more preferably bicyclic or tricyclic bridged heterocyclyl. Non-limiting examples of bridged heterocyclyl include:

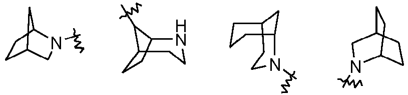

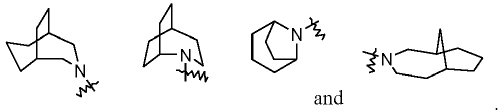

The heterocyclyl ring includes those in which the heterocyclyl described above (including monocyclic, spiro, fused and bridged ones) is fused to an aryl, heteroaryl or cycloalkyl ring, wherein the ring connected to the parent structure is heterocyclyl; its non-limiting examples include: 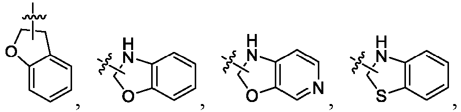 and the like.

Heterocyclyl may be substituted or unsubstituted. When substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "aryl" refers to a 6- to 14-membered, preferably 6- to 10-membered, all-carbon monocyclic or fused polycyclic (in which the rings share a pair of adjacent carbon atoms) group having a conjugated π-electron system, e.g., phenyl and naphthyl. The aryl ring includes those in which the aryl ring described above is fused to a heteroaryl, heterocyclyl or cycloalkyl ring, wherein the ring connected to the parent structure is the aryl ring; its non-limiting examples include: 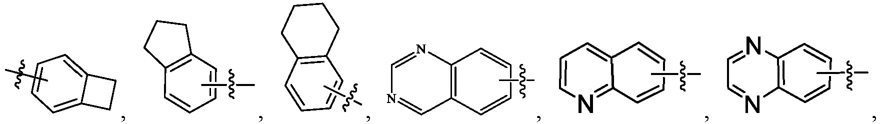 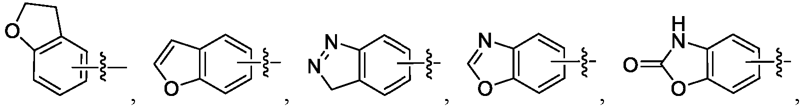 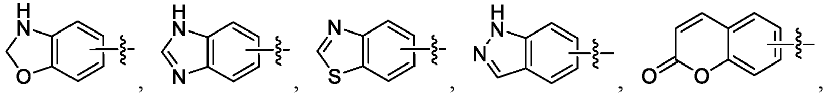 and

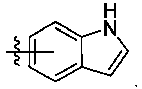

Aryl may be substituted or unsubstituted. When substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur and nitrogen. The heteroaryl is preferably 5- to 10-membered (e.g., 5-membered, 6-membered, 7-membered, 8-membered, 9-membered, or 10-membered) and more preferably 5-membered or 6-membered, e.g., furyl, thienyl, pyridinyl, pyrrolyl, *N*-alkylpyrrolyl, pyrimidinyl, pyrazinyl, pyridazinyl, imidazolyl, pyrazolyl, triazolyl, and tetrazolyl. The heteroaryl ring includes those in which the heteroaryl described above is fused to an aryl, heterocyclyl or cycloalkyl ring, wherein the ring connected to the parent structure is the heteroaryl ring; its non-limiting examples include:

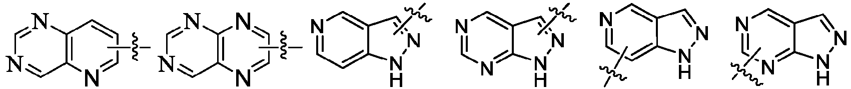

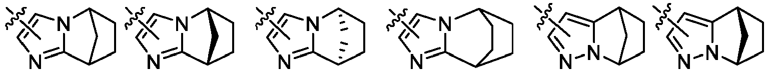

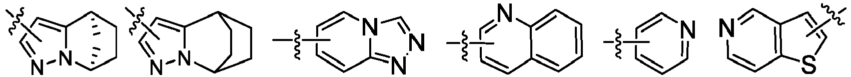

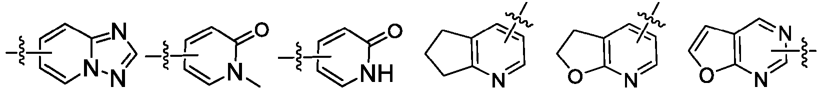

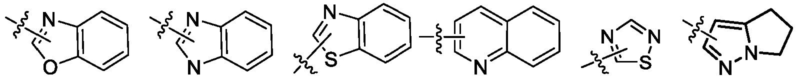

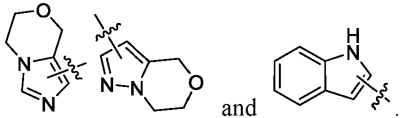

Heteroaryl may be substituted or unsubstituted. When substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The cycloalkyl, heterocyclyl, aryl and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene", or "heteroarylene".

The term "amino protecting group" refers to a group that can be easily removed and is intended to protect an amino group from being changed when reactions are taking place elsewhere in the molecule. Non-limiting examples include (trimethylsilyl)ethoxymethyl, tetrahydropyranyl, *tert*-butoxycarbonyl, acetyl, benzyl, allyl, *p*-methoxybenzyl, and the like. Those groups may be optionally substituted with 1 to 3 substituents selected from the group consisting of halogen, alkoxy, and nitro.

The term "hydroxy protecting group" refers to a hydroxy derivative that is commonly used to block or protect hydroxy while reactions are taking place on other functional groups of the compound. As an example, preferably, the hydroxy protecting group may be, for example: triethylsilyl, triisopropylsilyl, *tert*-butyldimethylsilyl (TBS), *tert*-butyldiphenylsilyl, methyl, *tert-*butyl*,* allyl, benzyl, methoxymethyl (MOM), ethoxyethyl, formyl, acetyl, benzoyl, *p*-nitrobenzoyl, and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O- or (cycloalkyl)C(O)O-, wherein the alkyl and cycloalkyl are as defined above.

The compounds disclosed herein include isotopic derivatives thereof. The term "isotopic derivative" refers to compounds that differ in structure only by having one or more enriched isotopic atoms. For example, compounds with the structure disclosed herein having "deuterium" or "tritium" in place of hydrogen, or ¹⁸F-fluorine labeling (¹⁸F isotope) in place of fluorine, or ¹¹C-, ¹³C- or ¹⁴C-enriched carbon (¹¹C-, ¹³C- or ¹⁴C-carbon labeling; ¹¹C-, ¹³C- or ¹⁴C-isotope) in place of a carbon atom are within the scope of the present disclosure. Such a compound can be used as an analytical tool or a probe in, for example, a biological assay, or may be used as a tracer for *in vivo* diagnostic imaging of disease, or as a tracer in a pharmacodynamic, pharmacokinetic, or receptor study. The deuterated forms of the compound mean that each available hydrogen atom connected to a carbon atom may be independently replaced with a deuterium atom. Those skilled in the art can synthesize the compounds in deuterated form by reference to the relevant literature. Commercially available deuterated starting materials can be used in preparing the deuterated compounds, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. Deuterides can generally retain comparable activity to non-deuterated compounds and can achieve better metabolic stability when deuterated at certain specific sites, thereby achieving certain therapeutic advantages. Compared with non-deuterated drugs, deuterated drugs have the advantages of reduced toxic and side effects, increased drug stability, enhanced curative effect, prolonged biological half-lives, and the like. All isotopic variations of the compounds of the present disclosure, whether radioactive or not, are intended to be included within the scope of the present disclosure. Each available hydrogen atom linked to a carbon atom may be independently replaced with a deuterium atom, wherein replacement of deuterium may be partial or complete, and replacement of partial deuterium refers to replacement of at least one hydrogen atom with at least one deuterium atom. When a position is specifically assigned deuterium (D), the position should be construed as deuterium with an abundance that is at least 1000 times greater than the natural abundance of deuterium (which is 0.015%) (i.e., at least 15% deuterium incorporation). The deuterium of the compounds in the examples with an abundance greater than the natural abundance of deuterium may be deuterium with an abundance that is at least 1000 times greater (i.e., at least 15% deuterium incorporation), at least 2000 times greater (i.e., at least 30% deuterium incorporation), at least 3000 times greater (i.e., at least 45% deuterium incorporation), at least 3340 times greater (i.e., at least 50.1% deuterium incorporation), at least 3500 times greater (i.e., at least 52.5% deuterium incorporation), at least 4000 times greater (i.e., at least 60% deuterium incorporation), at least 4500 times greater (i.e., at least 67.5% deuterium incorporation), at least 5000 times greater (i.e., at least 75% deuterium incorporation), at least 5500 times greater (i.e., at least 82.5% deuterium incorporation), at least 6000 times greater (i.e., at least 90% deuterium incorporation), at least 6333.3 times greater (i.e., at least 95% deuterium incorporation), at least 6466.7 times greater (i.e., at least 97% deuterium incorporation), at least 6600 times greater (i.e., at least 99% deuterium incorporation), or at least 6633.3 times greater (i.e., at least 99.5% deuterium incorporation), or deuterium with a higher abundance.

The compounds of the present disclosure may exist in specific stereoisomeric forms. The term "stereoisomer" refers to isomers that are structurally identical but differ in the arrangement of the atoms in space. It encompasses cis and trans (or *Z* and *E*) isomers, (-)- and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. For all carbon-carbon double bonds, both *Z*- and *E*-forms are included, even if only one configuration is named. Optically active (-)- and (+)- isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or derivatization with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. Furthermore, separation of enantiomers and diastereomers is generally accomplished by chromatography.

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It encompasses all possible tautomers; that is, it is present in the form of a single isomer or in the form of a mixture of the tautomers in any ratio. Non-limiting examples include: keto-enol, imine-enamine, lactam-lactim, and the like. An example of lactam-lactim in equilibrium is shown below:

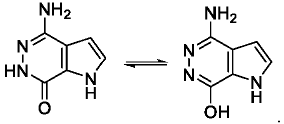

For example, reference to pyrazolyl is understood to include any one of the following two structures or a mixture of the two tautomers:

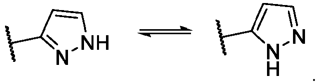

All tautomeric forms fall within the scope of the present disclosure, and the nomenclature of the compounds does not exclude any tautomers.

In the chemical structure of the compound of the present disclosure, a bond " 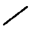 " represents an unspecified configuration; that is, if chiral isomers exist in the chemical structure, the bond " 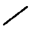 " may be " 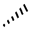 " or " 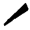 ", or contains both the configurations of " 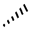 " and " 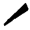 ".

"Optionally" or "optional" means that the event or circumstance subsequently described may, but does not necessarily, occur and that the description includes instances where the event or circumstance occurs or does not occur. For example, "C₁₋₆ alkyl that is optionally substituted with a halogen or cyano" means that the halogen or cyano may, but does not necessarily, exist, and the description includes the instance where alkyl is substituted with a halogen or cyano and the instance where alkyl is not substituted with a halogen or cyano.

"Substituted" means that one or more, preferably 1-5, more preferably 1-3 hydrogen atoms in the group are independently substituted with a corresponding number of substituents. Those skilled in the art can determine (experimentally or theoretically) possible or impossible substitution without undue effort. For example, it may be unstable when amino or hydroxy having free hydrogen is bound to a carbon atom having an unsaturated (e.g., olefinic) bond.

The term "pharmaceutical composition" refers to a mixture containing one or more of the compounds described herein or a pharmaceutically acceptable salt or pro-drug thereof, and other chemical components, and other components, for example, pharmaceutically acceptable carriers and excipients. The pharmaceutical composition is intended to promote the administration to an organism, so as to facilitate the absorption of the active ingredient, thereby exerting biological activity.

"Pharmaceutically acceptable salt" refers to a salt of the compound of the present disclosure, which may be selected from the group consisting of inorganic and organic salts. The salts are safe and effective for use in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of the drug or agent sufficient to achieve, or at least partially achieve, the desired effect. The determination of the effective amount varies from person to person. It depends on the age and general condition of a subject, as well as the particular active substance used. The appropriate effective amount in a case may be determined by those skilled in the art in the light of routine tests.

The term "pharmaceutically acceptable" used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

### Synthesis Methods for Compounds of the Present Disclosure

To achieve the purpose of the present disclosure, the following technical schemes are adopted in the present disclosure:

### Scheme 1

A method for preparing the compound of general formula (I) or the pharmaceutically acceptable salt thereof of the present disclosure, which comprises the following steps: 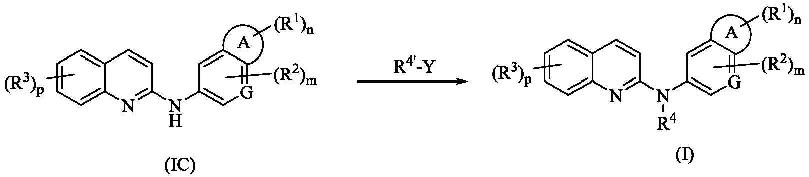
conducting a nucleophilic substitution reaction of a compound of general formula (IC) or a pharmaceutically acceptable salt thereof with an R^{4'}-Y compound under an alkaline condition, and then removing the protecting group on R^{4'} under an alkaline condition to give the compound of general formula (I) or the pharmaceutically acceptable salt thereof,
wherein
Y is halogen, preferably Br atom;
R^{4'} is 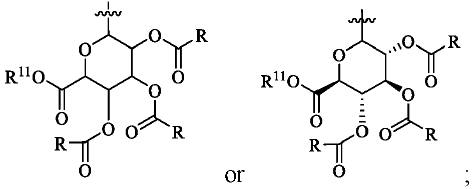
R is C₁₋₆ alkyl; R¹¹ is C₁₋₆ alkyl;
R⁴ is 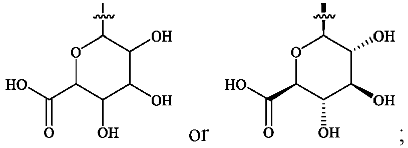
ring A, G, R¹ to R³, m, n, and p are as defined in general formula (I).

### Scheme 2

A method for preparing the compound of general formula (IC) or the pharmaceutically acceptable salt thereof of the present disclosure, which comprises the following step: 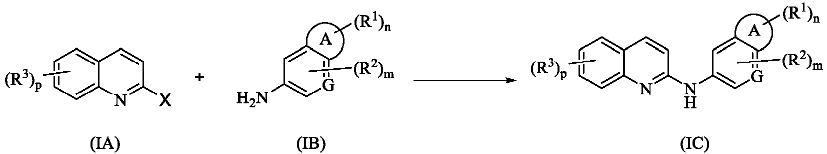
conducting an aromatic nucleophilic substitution reaction of a compound of general formula (IA) or a salt thereof with a compound of general formula (IB) or a salt thereof optionally under an alkaline condition or an acidic condition, or conducting a coupling reaction optionally under an alkaline condition or in the presence of a catalyst to give the compound of general formula (IC) or the pharmaceutically acceptable salt thereof,
wherein
X is halogen, preferably Cl atom;
ring A, G, R¹ to R³, m, n, and p are as defined in general formula (IC).

### Scheme 3

A method for preparing the compound of general formula (I-1) or the pharmaceutically acceptable salt thereof of the present disclosure, which comprises the following step: 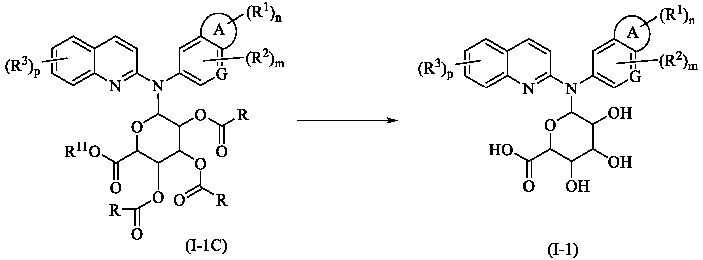
conducting an ester hydrolysis reaction of a compound of general formula (I-1C) or a salt thereof under an alkaline condition to give the compound of general formula (I-1) or the pharmaceutically acceptable salt thereof,
wherein
R and R¹¹ are as defined in general formula (I-1C);
ring A, G, R¹ to R³, m, n, and p are as defined in general formula (I-1).

### Scheme 4

A method for preparing the compound of general formula (I-2) or the pharmaceutically acceptable salt thereof of the present disclosure, which comprises the following step: 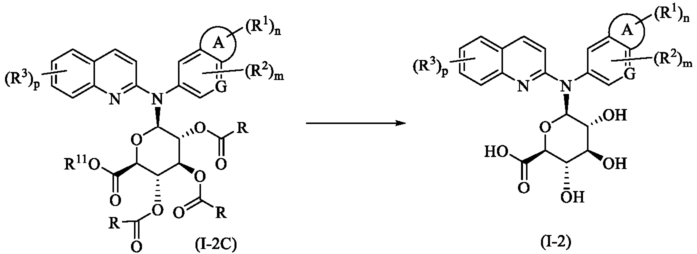
conducting an ester hydrolysis reaction of a compound of general formula (I-2C) or a salt thereof under an alkaline condition to give the compound of general formula (I-2) or the pharmaceutically acceptable salt thereof,
wherein
R is C₁₋₆ alkyl; R¹¹ is C₁₋₆ alkyl;
ring A, G, R¹ to R³, m, n, and p are as defined in general formula (I-2).

### Scheme 5

A method for preparing the compound of general formula (I-3) or the pharmaceutically acceptable salt thereof of the present disclosure, which comprises the following steps: 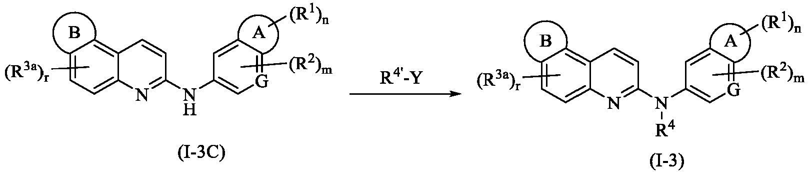
conducting a nucleophilic substitution reaction of a compound of general formula (I-3C) or a pharmaceutically acceptable salt thereof with an R^{4'}-Y compound under an alkaline condition, and then removing the protecting group on R^{4'} under an alkaline condition to give the compound of general formula (I-3) or the pharmaceutically acceptable salt thereof,
wherein
Y is halogen, preferably Br atom;
R^{4'} is 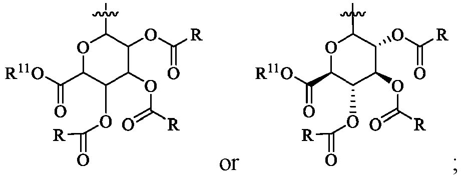
R is C₁₋₆ alkyl; R¹¹ is C₁₋₆ alkyl;
R⁴ is 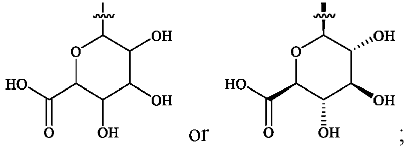
ring A, ring B, G, R¹, R², R^{3a}, n, m, and r are as defined in general formula (I-3).

### Scheme 6

A method for preparing the compound of general formula (I-3C) or the pharmaceutically acceptable salt thereof of the present disclosure, which comprises the following step: 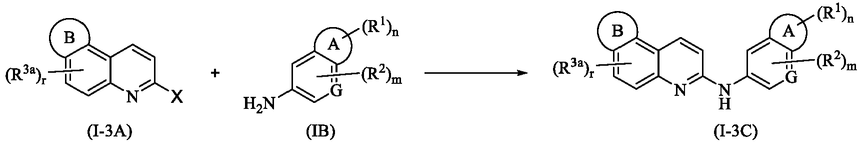
conducting a nucleophilic substitution reaction of a compound of general formula (I-3A) or a salt thereof with a compound of general formula (IB) or a salt thereof optionally under an alkaline condition or an acidic condition, or conducting a coupling reaction optionally under an alkaline condition or in the presence of a catalyst to give the compound of general formula (I-3C) or the pharmaceutically acceptable salt thereof, wherein
X is halogen, preferably Cl atom;
ring A, ring B, G, R¹, R², R^{3a}, n, m, and r are as defined in general formula (I-3C).

### Scheme 7

A method for preparing the compound of general formula (I-4) or the pharmaceutically acceptable salt thereof of the present disclosure, which comprises the following steps: 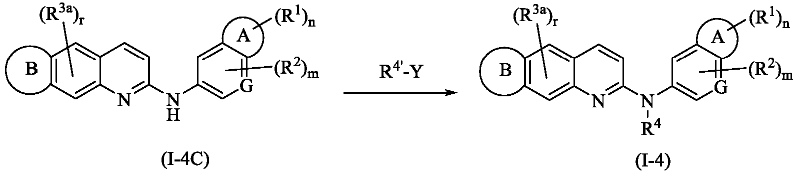
conducting a nucleophilic substitution reaction of a compound of general formula (I-4C) or a pharmaceutically acceptable salt thereof with an R^{4'}-Y compound under an alkaline condition, and then removing the protecting group on R^{4'} under an alkaline condition to give the compound of general formula (I-4) or the pharmaceutically acceptable salt thereof,
wherein
Y is halogen, preferably Br atom;
R^{4'} is 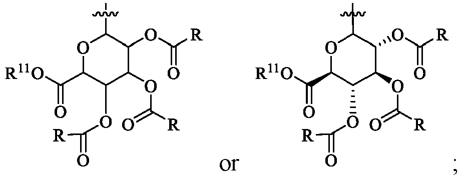
R is C₁₋₆ alkyl; R¹¹ is C₁₋₆ alkyl;
R⁴ is 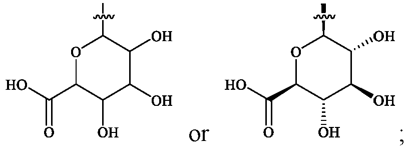
ring A, ring B, G, R¹, R², R^{3a}, n, m, and r are as defined in general formula (I-4).

### Scheme 8

A method for preparing the compound of general formula (I-4C) or the pharmaceutically acceptable salt thereof of the present disclosure, which comprises the following step: 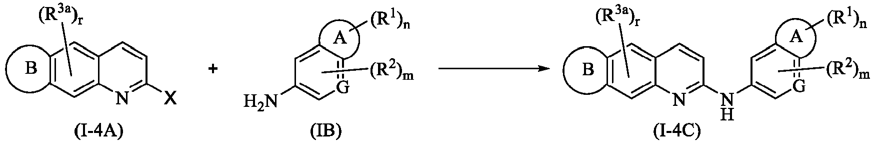
conducting a nucleophilic substitution reaction of a compound of general formula (I-4A) or a salt thereof with a compound of general formula (IB) or a salt thereof optionally under an alkaline condition or an acidic condition, or conducting a coupling reaction optionally under an alkaline condition or in the presence of a catalyst to give the compound of general formula (I-4C) or the pharmaceutically acceptable salt thereof, wherein
X is halogen, preferably Cl atom;
ring A, ring B, G, R¹, R², R^{3a}, n, m, and r are as defined in general formula (I-4C).

### Scheme 9

A method for preparing the compound of general formula (II) or the pharmaceutically acceptable salt thereof of the present disclosure, which comprises the following steps: 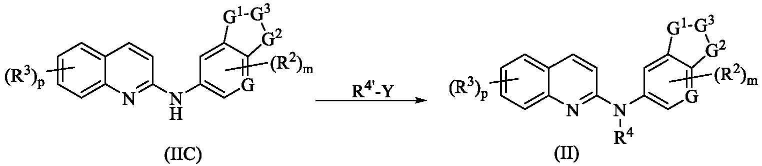
conducting a nucleophilic substitution reaction of a compound of general formula (IIC) or a pharmaceutically acceptable salt thereof with an R^{4'}-Y compound under an alkaline condition, and then removing the protecting group on R^{4'} under an alkaline condition to give the compound of general formula (II) or the pharmaceutically acceptable salt thereof,
wherein
Y is halogen, preferably Br atom;
R^{4'} is 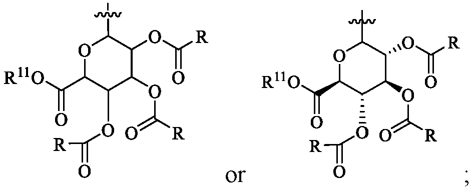
R is C₁₋₆ alkyl; R¹¹ is C₁₋₆ alkyl;
R⁴ is 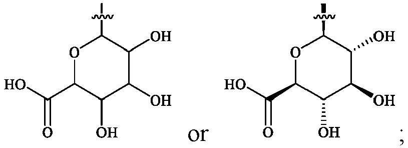
G, G¹, G², G³, R², R³, m, and p are as defined in general formula (II).

### Scheme 10

A method for preparing the compound of general formula (IIC) or the pharmaceutically acceptable salt thereof of the present disclosure, which comprises the following step: 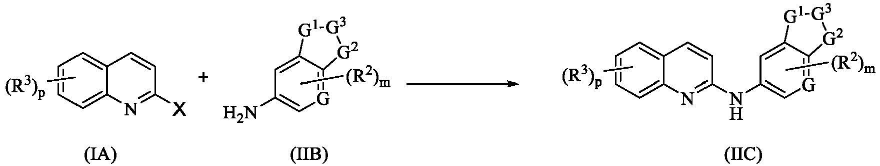
conducting a nucleophilic substitution reaction of a compound of general formula (IA) or a salt thereof with a compound of general formula (IIB) or a salt thereof optionally under an alkaline condition or an acidic condition, or conducting a coupling reaction optionally under an alkaline condition or in the presence of a catalyst to give the compound of general formula (IIC) or the pharmaceutically acceptable salt thereof,
wherein
X is halogen, preferably Cl atom;
G, G¹, G², G³, R², R³, m, and p are as defined in general formula (IIC).

### Scheme 11

A method for preparing the compound of general formula (IID-1) or the pharmaceutically acceptable salt thereof of the present disclosure, which comprises the following step: 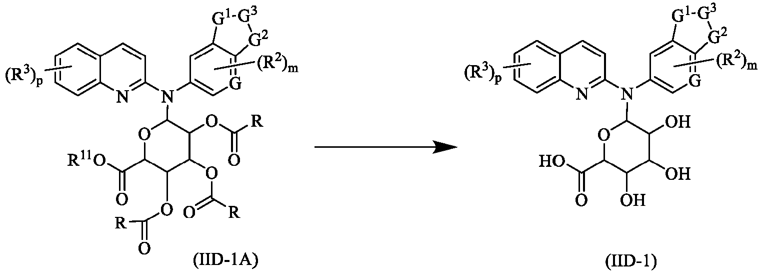
conducting an ester hydrolysis reaction of a compound of general formula (IID-1A) or a salt thereof under an alkaline condition to give the compound of general formula (IID-1) or the pharmaceutically acceptable salt thereof,
wherein
R is C₁₋₆ alkyl; R¹¹ is C₁₋₆ alkyl;
G, G¹, G², G³, R², R³, m, and p are as defined in general formula (IID-1).

### Scheme 12

A method for preparing the compound of general formula (IID-2) or the pharmaceutically acceptable salt thereof of the present disclosure, which comprises the following step: 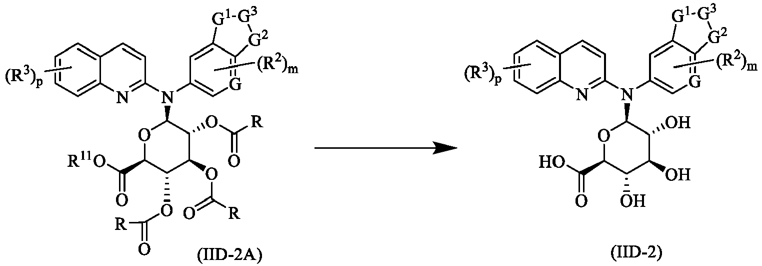
conducting an ester hydrolysis reaction of a compound of general formula (IID-2A) or a salt thereof under an alkaline condition to give the compound of general formula (IID-2) or the pharmaceutically acceptable salt thereof,
wherein
R is C₁₋₆ alkyl; R¹¹ is C₁₋₆ alkyl;
G, G¹, G², G³, R², R³, m, and p are as defined in general formula (IID-2).

### Scheme 13

A method for preparing the compound of general formula (II-1) or the pharmaceutically acceptable salt thereof of the present disclosure, which comprises the following steps: 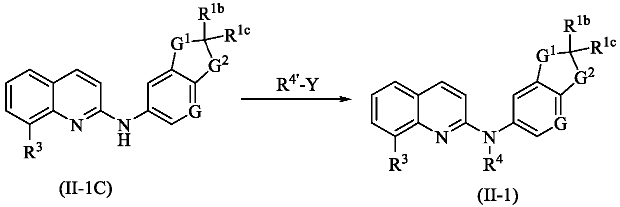
conducting a nucleophilic substitution reaction of a compound of general formula (II-1C) or a pharmaceutically acceptable salt thereof with an R^{4'}-Y compound under an alkaline condition, and then removing the protecting group on R^{4'} under an alkaline condition to give the compound of general formula (II-1) or the pharmaceutically acceptable salt thereof,
wherein
Y is halogen, preferably Br atom;
R^{4'} is 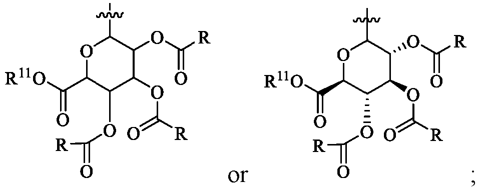
R is C₁₋₆ alkyl; R¹¹ is C₁₋₆ alkyl;
R⁴ is 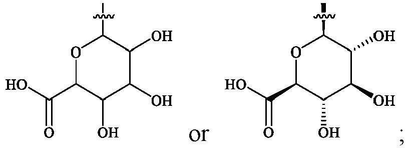
G, G¹, G², R^{1b}, R^{1c}, and R³ are as defined in general formula (II-1); preferably, R³ is halogen.

### Scheme 14

A method for preparing the compound of general formula (II-1C) or the pharmaceutically acceptable salt thereof of the present disclosure, which comprises the following step: 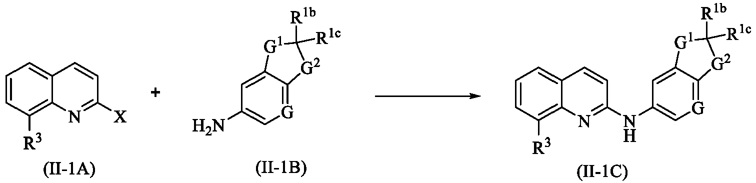
conducting a nucleophilic substitution reaction of a compound of general formula (II-1A) or a salt thereof with a compound of general formula (II-1B) or a salt thereof optionally under an alkaline condition or an acidic condition, or conducting a coupling reaction optionally under an alkaline condition or in the presence of a catalyst to give the compound of general formula (II-1C) or the pharmaceutically acceptable salt thereof,
wherein
X is halogen, preferably Cl atom;
G, G¹, G², R^{1b}, R^{1c}, and R³ are as defined in general formula (II-1C); preferably, R³ is halogen.

### Scheme 15

A method for preparing the compound of general formula (III) or the pharmaceutically acceptable salt thereof of the present disclosure, which comprises the following steps: 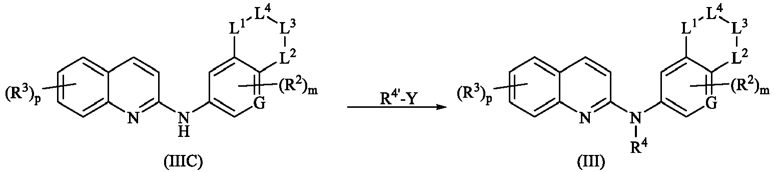
conducting a nucleophilic substitution reaction of a compound of general formula (IIIC) or a pharmaceutically acceptable salt thereof with an R^{4'}-Y compound under an alkaline condition, and then removing the protecting group on R^{4'} under an alkaline condition to give the compound of general formula (III) or the pharmaceutically acceptable salt thereof,
wherein
Y is halogen, preferably Br atom;
R^{4'} is 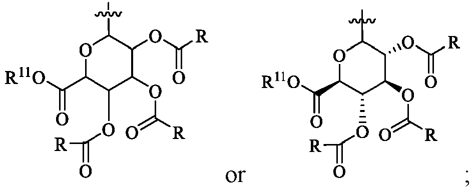
R is C₁₋₆ alkyl; R¹¹ is C₁₋₆ alkyl;
R⁴ is 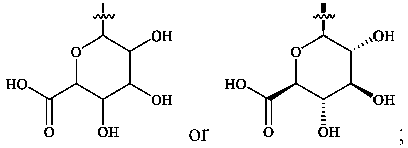
G, L¹, L², L³, L⁴, R², R³, m, and p are as defined in general formula (III).

### Scheme 16

A method for preparing the compound of general formula (IIIC) or the pharmaceutically acceptable salt thereof of the present disclosure, which comprises the following steps: 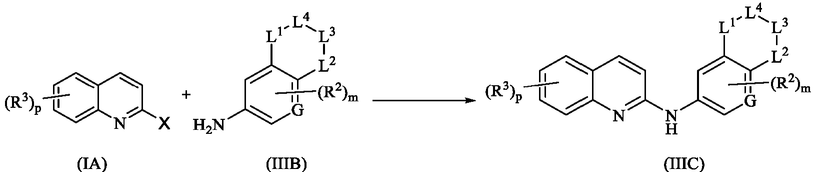
conducting a nucleophilic substitution reaction of a compound of general formula (IA) or a salt thereof with a compound of general formula (IIIB) or a salt thereof optionally under an alkaline condition or an acidic condition, or conducting a coupling reaction optionally under an alkaline condition or in the presence of a catalyst to give the compound of general formula (IIIC) or the pharmaceutically acceptable salt thereof,
wherein
X is halogen, preferably Cl atom;
G, L¹, L², L³, L⁴, R², R³, m, and p are as defined in general formula (IIIC).

### Scheme 17

A method for preparing the compound of general formula (IIID-1) or the pharmaceutically acceptable salt thereof of the present disclosure, which comprises the following step: 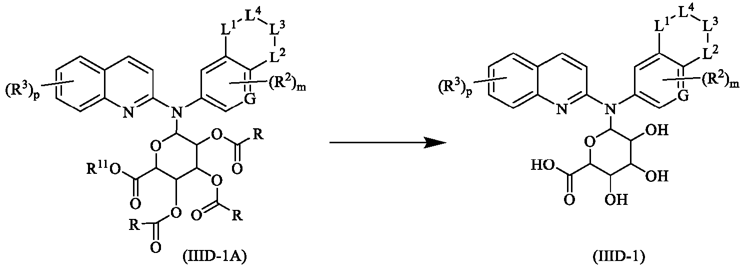
conducting an ester hydrolysis reaction of a compound of general formula (IIID-1A) or a salt thereof under an alkaline condition to give the compound of general formula (IIID-1) or the pharmaceutically acceptable salt thereof,
wherein
R is C₁₋₆ alkyl; R¹¹ is C₁₋₆ alkyl;
G, L¹, L², L³, L⁴, R², R³, m, and p are as defined in general formula (IIID-1).

### Scheme 18

A method for preparing the compound of general formula (IIID-2) or the pharmaceutically acceptable salt thereof of the present disclosure, which comprises the following step: 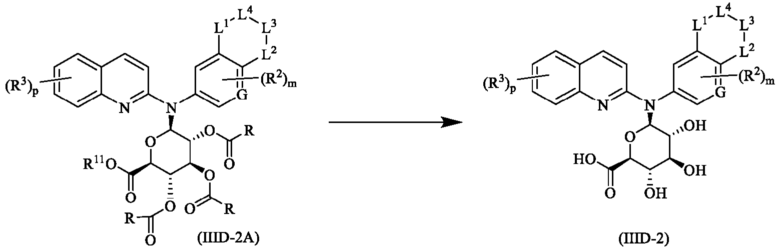
conducting an ester hydrolysis reaction of a compound of general formula (IIID-2A) or a salt thereof under an alkaline condition to give the compound of general formula (IIID-2) or the pharmaceutically acceptable salt thereof,
wherein
R is C₁₋₆ alkyl; R¹¹ is C₁₋₆ alkyl;
G, L¹, L², L³, L⁴, R², R³, m, and p are as defined in general formula (IIID-2).

### Scheme 19

A method for preparing the compound of general formula (III-1) or the pharmaceutically acceptable salt thereof of the present disclosure, which comprises the following steps: 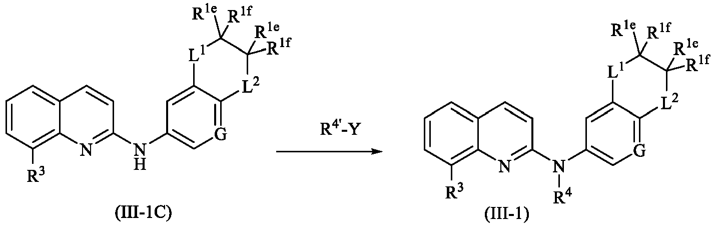
conducting a nucleophilic substitution reaction of a compound of general formula (III-1C) or a pharmaceutically acceptable salt thereof with an R^{4'}-Y compound under an alkaline condition, and then removing the protecting group on R^{4'} under an alkaline condition to give the compound of general formula (III-1) or the pharmaceutically acceptable salt thereof,
wherein
Y is halogen, preferably Br atom;
R^{4'} is 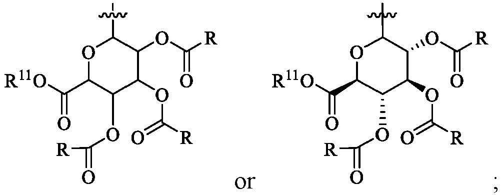
R is C₁₋₆ alkyl; R¹¹ is C₁₋₆ alkyl;
R⁴ is 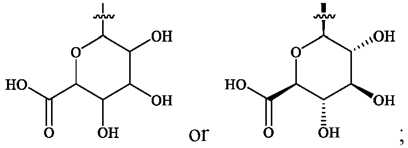
G, L¹, L², R^{1e}, R^{1f}, and R³ are as defined in general formula (III-1); preferably, R³ is halogen.

### Scheme 20

A method for preparing the compound of general formula (III-1C) or the pharmaceutically acceptable salt thereof of the present disclosure, which comprises the following step: 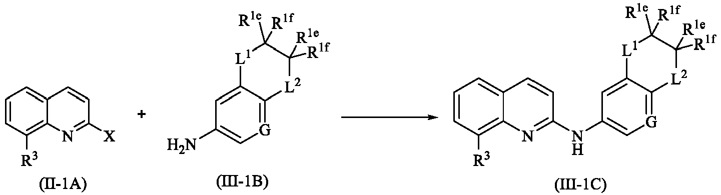
conducting a nucleophilic substitution reaction of a compound of general formula (II-1A) or a salt thereof with a compound of general formula (III-1B) or a salt thereof optionally under an alkaline condition or an acidic condition, or conducting a coupling reaction optionally under an alkaline condition or in the presence of a catalyst to give the compound of general formula (III-1C) or the pharmaceutically acceptable salt thereof,
wherein
X is halogen, preferably Cl atom;
G, L¹, L², R^{1e}, R^{1f}, and R³ are as defined in general formula (III-1C); preferably, R³ is halogen.

The reagents providing the alkaline conditions in the above synthesis schemes include organic bases and inorganic bases, wherein the organic bases include, but are not limited to, triethylamine, pyridine, *N,N*-diisopropylethylamine, *n*-butyllithium, lithium diisopropylamide, sodium acetate, potassium acetate, sodium *tert*-butoxide, potassium *tert*-butoxide, or 1,8-diazabicycloundec-7-ene, and the inorganic bases include, but are not limited to, sodium hydride, potassium phosphate, sodium carbonate, potassium carbonate, cesium carbonate, cadmium carbonate, sodium hydroxide, lithium hydroxide monohydrate, lithium hydroxide, and potassium hydroxide; preferably, the reagent providing the alkaline condition is selected from the group consisting of lithium hydroxide monohydrate, potassium carbonate, cesium carbonate, and cadmium carbonate; the reagents providing the alkaline conditions in schemes 2, 6, 8, 10, 14, 16, and 20 are more preferably potassium carbonate or cesium carbonate; the reagents providing the alkaline conditions in the nucleophilic substitution reactions in schemes 1, 5, 7, 9, 13, 15, and 19 are more preferably cadmium carbonate.

The reagents providing the alkaline conditions in schemes 3, 4, 11, 12, 17, and 18 are preferably lithium hydroxide monohydrate, and more preferably lithium hydroxide monohydrate and hydrogen peroxide.

The reagents providing the alkaline conditions in the reactions of removing the protecting group on R^{4'} in schemes 1, 5, 7, 9, 13, 15, and 19 are preferably lithium hydroxide monohydrate, and more preferably lithium hydroxide monohydrate and hydrogen peroxide.

The reagents providing the acidic conditions in the above synthesis schemes include, but are not limited to, mellitic acid, nitrogen sulfur squaric acid, trichloroacetic acid, trinitrobenzenesulfonic acid, trifluoromethanesulfonic acid, and trifluoroacetic acid; preferably, the reagent providing the acidic condition is trifluoroacetic acid.

The ester hydrolysis reactions involved in the above synthesis schemes are preferably conducted under the conditions of lithium hydroxide monohydrate and hydrogen peroxide.

The catalysts in the above synthesis schemes include, but are not limited to, tetrakis(triphenylphosphine)palladium, palladium dichloride, palladium acetate, methanesulfonato(2-dicyclohexylphosphino-3,6-dimethoxy-2',4',6'-triisopropoxy-1,1'-bi phenyl)(2'-amino-1,1'-biphenyl-2-yl)palladium(II), [1,1'-bis(dibenzylphosphino)ferrocene]palladium(II) dichloride, [1,1'-bis(diphenylphosphino)ferrocene]palladium(II) dichloride, [1,1'-bis(diphenylphosphino)ferrocene]palladium(II) dichloride dichloromethane complex, tris(dibenzylideneacetone)dipalladium(0), and 4,5-bis(diphenylphosphino)-9,9-dimethylxanthene; preferably, the catalyst is selected from the group consisting of methanesulfonato(2-dicyclohexylphosphino-3,6-dimethoxy-2',4',6'-triisopropoxy-1,1'-bi phenyl)(2'-amino-1,1'-biphenyl-2-yl)palladium(II), tris(dibenzylideneacetone)dipalladium(0), and 4,5-bis(diphenylphosphino)-9,9-dimethylxanthene.

The reaction of the above steps is preferably conducted in a solvent including, but not limited to: pyridine, ethylene glycol dimethyl ether, acetic acid, methanol, ethanol, acetonitrile, *n*-butanol, toluene, tetrahydrofuran, dichloromethane, petroleum ether, ethyl acetate, *n*-hexane, dimethyl sulfoxide, 1,4-dioxane, water, *N,N*-dimethylformamide, *N,N*-dimethylacetamide, 1,2-dibromoethane, and a mixture thereof.

### BRIEF DESCRIPTION OF THE DRAWINGS

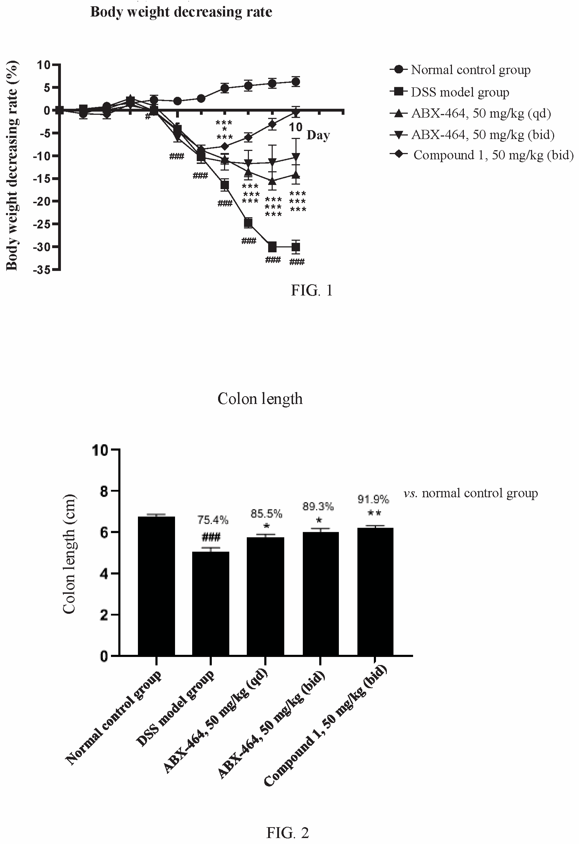

FIG. 1 shows the effect of compound **1** disclosed herein on the body weight of dextran sulfate sodium salt (DSS)-induced UC mice, wherein, #P < 0.05 indicates a significant difference between the model group and the normal control group, ##P < 0.01 indicates a highly significant difference between the model group and the normal control group, and ###P < 0.001 indicates a very highly significant difference between the model group and the normal control group; *P < 0.05 indicates that a significant difference between the administration group and the model group, **P < 0.01 indicates a highly significant difference between the administration group and the model group, and ***P < 0.001 indicates a very highly significant difference between the administration group and the model group.
FIG. 2 shows the effect of compound **1** disclosed herein on the colon length of dextran sulfate sodium salt (DSS)-induced UC mice, wherein, #P < 0.05 indicates a significant difference between the model group and the normal control group, ##P < 0.01 indicates a highly significant difference between the model group and the normal control group, and ###P < 0.001 indicates a very highly significant difference between the model group and the normal control group; *P < 0.05 indicates that a significant difference between the administration group and the model group, **P < 0.01 indicates a highly significant difference between the administration group and the model group, and ***P < 0.001 indicates a very highly significant difference between the administration group and the model group.

### DETAILED DESCRIPTION

The present disclosure is further described below with reference to examples, which, however, are not intended to limit the present disclosure.

### Examples

The structures of compounds were determined by nuclear magnetic resonance (NMR) spectroscopy and/or mass spectrometry (MS). NMR shifts (δ) are given in 10⁻⁶ (ppm).

NMR analysis was performed on a Bruker AVANCE-400 nuclear magnetic resonance instrument, with deuterated dimethyl sulfoxide (DMSO-*d₆*), deuterated chloroform (CDCl₃) and deuterated methanol (CD₃OD) as solvents and tetramethylsilane (TMS) as an internal standard.

MS analyses were performed on an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector) and THERMO Ultimate 3000-Q Exactive (manufacturer: THERMO, MS model: THERMO Q Exactive).

High performance liquid chromatography (HPLC) analyses were performed on Agilent HPLC 1200DAD, Agilent HPLC 1200VWD, and Waters HPLC e2695-2489 high performance liquid chromatographs.

Chiral HPLC analyses were performed on an Agilent 1260 DAD high performance liquid chromatograph.

Preparative high performance liquid chromatography used Waters 2545-2767, Waters 2767-SQ Detecor2, Shimadzu LC-20AP and Gilson GX-281 preparative chromatographs.

Preparative chiral chromatography used a Shimadzu LC-20AP preparative chromatograph.

The CombiFlash preparative flash chromatograph used was CombiFlash Rf200 (TELEDYNE ISCO).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Silica gel column chromatography generally used 200- to 300-mesh silica gel (Huanghai, Yantai) as the carrier.

The mean inhibition of kinase and the IC₅₀ value were determined on a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were performed using a Parr 3916EKX hydrogenator and a Qinglan QL-500 hydrogenator, or an HC2-SS hydrogenator.

Hydrogenation reactions generally involved 3 cycles of vacuumization and hydrogen purging.

Microwave reactions were performed on a CEM Discover-S 908860 microwave reactor. In the examples, a solution was an aqueous solution unless otherwise specified.

In the examples, reactions were conducted at room temperature, i.e., 20 °C to 30 °C, unless otherwise specified.

The monitoring of the reaction progress in the examples is conducted by thin-layer chromatography (TLC). The developing solvent for reactions, the eluent system for column chromatography purification and the developing solvent system for thin-layer chromatography include: A: dichloromethane/methanol system, and B: *n*-hexane/ethyl acetate system. The volume ratio of the solvents was adjusted according to the polarity of the compound, or by adding a small amount of basic or acidic reagents such as triethylamine and acetic acid.

### Example 1

### 8-Chloro-N-(2,2-difluorobenzo[d][1,3]dioxol-5-yl)quinolin-2-amine 1

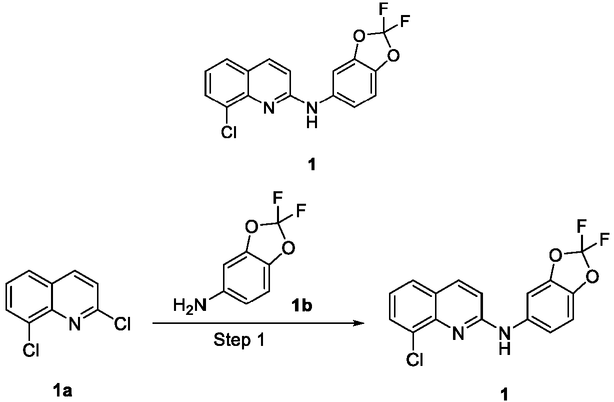

2,8-Dichloroquinoline **1a** (100 mg, 0.51 mmol, Bide Pharmatech Ltd.) and 5-amino-2,2-difluoro-1,3-benzo[1,3]dioxolane **1b** (105 mg, 0.61 mmol, Shanghai Haohong Scientific Co., Ltd.) were dissolved in isopropanol (1 mL), and the resulting solution was heated to 90 °C and allowed to react for 12 h. The reaction solution was cooled to room temperature, filtered, and then separated and purified by preparative high performance liquid chromatography (Waters 2767-SQ Detecor2, elution system: a 0.1% aqueous formic acid solution and acetonitrile, gradient of acetonitrile: 65%-85%, flow rate: 30 mL/min) to give the title compound **1** (150 mg, yield: 89%).

MS m/z (ESI): 335.0 [M+1].

¹H NMR (500 MHz, DMSO-*d₆*) δ 9.99 (s, 1H), 8.88 (d, 1H), 8.17 (d, 1H), 7.81 (dd, 1H), 7.77 (dd, 1H), 7.50 (dd, 1H), 7.39 (d, 1H), 7.32 (t, 1H), 7.14 (d, 1H).

### Example 2

### (2S,3S,4S,5R,6R)-6-((8-Chloroquinolin-2-yl)(2,2-difluorobenzo[d][1,3]dioxol-5-yl)amin o)-3,4,5-trihydroxytetrahydro-2H-pyran-2-carboxylic acid 2

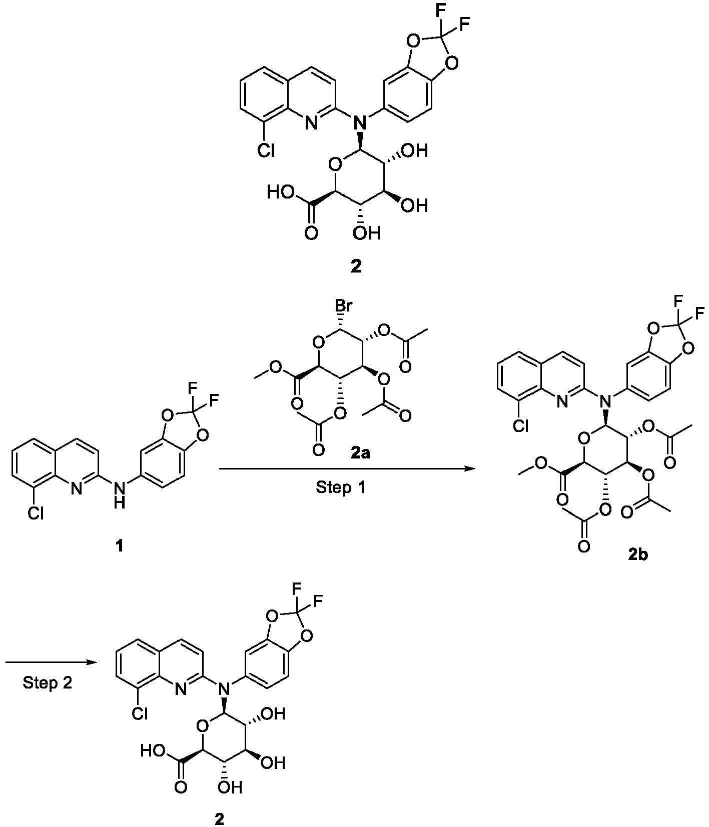

### Step 1

### (2R,3R,4S,5S,6S)-2-((8-chloroquinolin-2-yl)(2,2-difluorobenzo[d][1,3]dioxol-5-yl)amin o)-6-(methoxycarbonyl)tetrahydro-2H-pyran-3,4,5-triyl triacetate 2b

Compound 1 (500 mg, 1.49 mmol) was dissolved in toluene (30 mL), and cadmium carbonate (155 mg, 0.90 mmol) was added. The resulting mixture was heated to 140 °C for water separation reaction for 12 h, and then methyl 1-bromo-1-deoxy-2,3,4-tri-O-acetyl-α-*D*-glucuronate **2a** (1.05 g, 2.64 mmol, Accela ChemBio Inc.), followed by water separation reaction at 140 °C for 24 h. The reaction mixture was cooled to room temperature, concentrated under reduced pressure to remove toluene, and purified by column chromatography with eluent system B to give the title compound **2b** (340 mg, yield: 35%).

MS m/z (ESI): 651.0 [M+1].

### Step 2

### (2S,3S,4S,5R,6R)-6-((8-Chloroquinolin-2-yl)(2,2-difluorobenzo[d][1,3]dioxol-5-yl)amin o)-3,4,5-trihydroxytetrahydro-2H-pyran-2-carboxylic acid 2

Lithium hydroxide monohydrate (450 mg, 10.74 mmol) was dissolved in water (5 mL), hydrogen peroxide (1 mL) was added. The resulting solution was stirred at room temperature for 10 min, then added to a solution of compound **2b** (340 mg, 0.52 mmol) in tetrahydrofuran (15 mL), and stirred at room temperature for another 2 h. The reaction was quenched with a saturated sodium thiosulfate solution (20 mL), and then the mixture was adjusted to pH 3 with a 1 N hydrochloric acid solution, extracted with ethyl acetate (50 mL × 3), and washed with a saturated sodium chloride solution (100 mL). The organic phase was concentrated under reduced pressure, and then separated and purified by preparative high performance liquid chromatography (Waters 2767-SQ Detecor2, elution system: a 10 mmol/L aqueous ammonium bicarbonate solution and acetonitrile, gradient of acetonitrile: 25%-45%, flow rate: 30 mL/min) to give the title compound **2** (95 mg, yield: 36%).

MS m/z (ESI): 511.0 [M+1].

¹H NMR (500 MHz, DMSO-*d*₆) δ 8.06 (d, 1H), 7.79 (d, 1H), 7.75 (d, 1H), 7.58 (d, 1H), 7.42 (d, 1H), 7.34-7.22 (m, 2H), 6.60 (d, 1H), 6.30 (d, 1H), 5.05-4.82 (m, 2H), 3.55-3.36 (m, 4H), 2.98 (t, 1H).

### Example 3

### 8-Chloro-N-(2,3-dihydrobenzofuran-5-yl)quinolin-2-amine 3

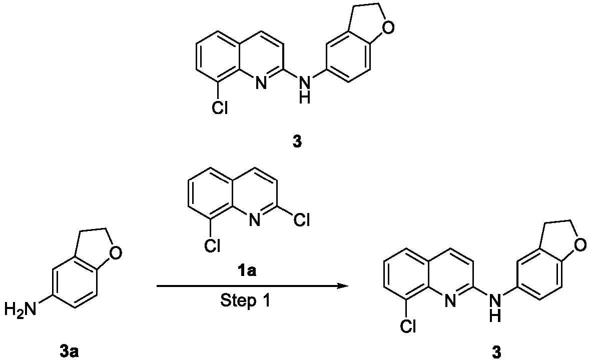

Compound **1a** (100 mg, 0.51 mmol, Bide Pharmatech Ltd.), 5-amino-2,3-dihydrobenzofuran **3a** (105 mg, 0.61 mmol, Bide Pharmatech Ltd.), and trifluoroacetic acid (0.15 mL, 2.0 mmol) were added to isopropanol (1 mL), and the resulting mixture was heated to 90 °C and allowed to react for 12 h. The reaction mixture was cooled to room temperature, filtered, and then separated and purified by preparative high performance liquid chromatography (Waters 2767-SQ Detecor2, elution system: a 0.1% aqueous formic acid solution and acetonitrile, gradient of acetonitrile: 45%-65%, flow rate: 30 mL/min) to give the title compound **3** (115 mg, yield: 77%).

MS m/z (ESI): 297.0 [M+1].

¹H NMR (500 MHz, DMSO-*d*₆) δ 9.51 (s, 1H), 8.20 (d, 1H), 8.05 (d, 1H), 7.80-7.65 (m, 3H), 7.23 (t, 1H), 7.07 (d, 1H), 6.75 (d, 1H), 4.52 (t, 2H), 3.22 (t, 2H).

### Example 4

### 8-Chloro-N-(2,3-dihydro-1H-inden-5-yl)quinolin-2-amine

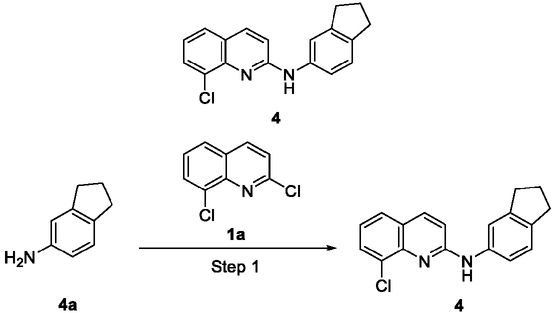

5-Indanamine **4a** (168 mg, 1.26 mmol, TCI), compound **1a** (100 mg, 0.51 mmol, Shanghai Haohong Scientific Co., Ltd.), and trifluoroacetic acid (173 mg, 1.51 mmol) were dissolved in isopropanol (3 mL), and the resulting solution was heated to 100 °C and allowed to react for 12 h. The reaction solution was cooled to room temperature, filtered, and then separated and purified by preparative high performance liquid chromatography (Waters 2767-SQ Detecor2, elution system: a 10 mmol/L aqueous ammonium bicarbonate solution and acetonitrile, gradient of acetonitrile: 65%-85%, flow rate: 30 mL/min) to give the title compound **4** (129 mg, yield: 87%).

MS m/z (ESI): 295.1 [M+1].

¹H NMR (500 MHz, CDCl₃) 7.90 (d, 1H), 7.69 (dd, 1H), 7.63 (s, 1H), 7.57 dd, 1H), 7.34 (d, 1H), 7.25 (d, 1H), 7.21 (t, 1H), 7.01 (d, 1H), 6.96 (s, 1H), 3.04-2.90 (m, 4H), 2.17-2.09 (m, 2H).

### Example 5

### 1-(5-((8-Chloroquinolin-2-yl)amino)indolin-1-yl)ethan-1-one 5

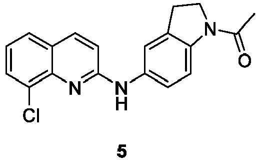

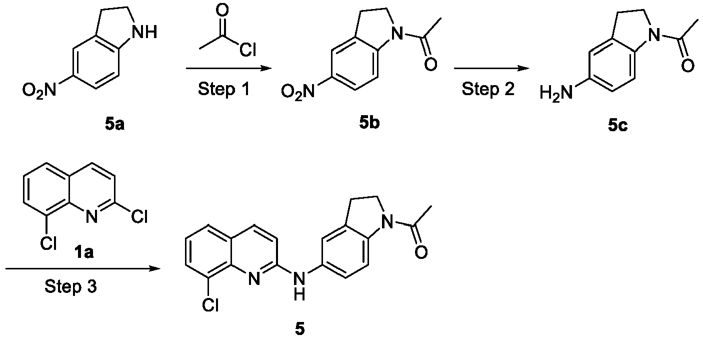

### Step 1

### 1-(5-Nitroindolin-1-yl)ethan-1-one 5b

5-Nitroindoline **5a** (2.0 g, 12.19 mmol; Bide Pharmatech Ltd.) and triethylamine (1.6 g, 15.85 mmol) were added to 40 mL of dichloromethane, and acetyl chloride (1.24 g, 15.85 mmol) was slowly added at 0 °C. The resulting mixture was then naturally warmed to room temperature and was allowed to react for 2 h. 30 mL of water was added to the system, and the resulting mixture was extracted with dichloromethane (50 mL × 3). The organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **5b** (1.8 g, yield: 72%).

MS m/z (ESI): 207.1 [M+1].

### Step 2

### 1-(5-Aminoindolin-1-yl)ethan-1-one 5c

Compound **5b** (0.8 g, 3.88 mmol) was added to 10 mL of methanol, and palladium on carbon (10%, 0.2 g) was added. The resulting mixture was purged with hydrogen three times to expel the air and allowed to react under a hydrogen atmosphere for 2 h. The palladium on carbon was removed by filtration, and the filtrate was concentrated under reduced pressure to give the title compound **5c** (crude product, 0.5 g), which was directly used in the next step.

MS m/z (ESI): 177.1 [M+1].

### Step 3

### 1-(5-((8-Chloroquinolin-2-yl)amino)indolin-1-yl)ethan-1-one 5

Compound **5c** (100 mg, 0.57 mmol), 2,8-dichloroquinoline **1a** (112 mg, 0.57 mmol, Bide Pharmatech Ltd.), and trifluoroacetic acid (64.72 mg, 0.57 mmol) were dissolved in isopropanol (1 mL), and then the resulting solution was heated to 90 °C and allowed to react for 12 h. The reaction solution was cooled to room temperature and concentrated under reduced pressure to remove the solvent, and the residue was purified by thin-layer chromatography (TLC) with developing solvent system B to give the title product **5** (48 mg, yield: 25%).

MS m/z (ESI): 338.1 [M+1].

¹H NMR (400 MHz, DMSO-*d₆*) δ 9.76 (s, 1H), 8.27 (s, 1H), 8.10 (d, 1H), 8.01 (d, 1H), 7.89-7.65 (m, 3H), 7.26 (t, 1H), 7.12 (d, 1H), 4.11 (t, 2H), 3.19 (t, 2H), 2.15 (s, 3H).

### Example 6

### 8-Chloro-N-(2,3-dihydrobenzo[b][1,4]dioxin-6-yl)quinolin-2-amine 6

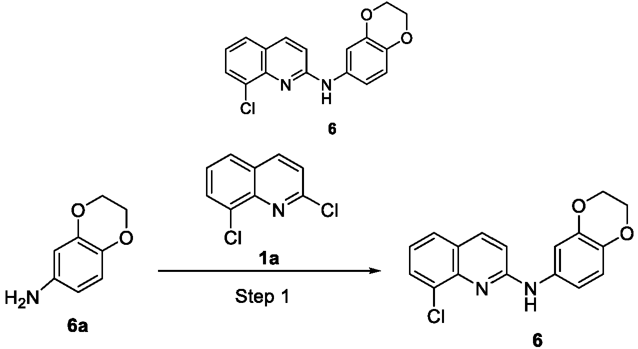

6-Amino-1,4-benzodioxane **6a** (95 mg, 0.63 mmol, TCI) and 2,8-dichloroquinoline **1a** (50 mg, 0.25 mmol, Bide Pharmatech Ltd.) was dissolved in isopropanol (3 mL). The reaction solution was heated to 100 °C, allowed to react for 12 h, then cooled to room temperature, filtered, and then separated and purified by preparative high performance liquid chromatography (Waters 2767-SQ Detecor2, elution system: a 10 mmol/L aqueous ammonium bicarbonate solution and acetonitrile, gradient of acetonitrile: 58%-78%, flow rate: 30 mL/min) to give the title compound **6** (63 mg, yield: 80%).

MS m/z (ESI): 313.1 [M+1].

¹H NMR (500 MHz, CDCl₃) 7.90 (d, 1H), 7.73 (dd, 1H), 7.57 (dd, 1H), 7.40 (s, 1H), 7.21 (t, 1H), 7.04 (dd, 1H), 6.96 (d, 1H), 6.90 (d, 1H), 6.83 (s, 1H), 4.35-4.26 (m, 4H).

### Example 7

### 8-Chloro-N-(5,6,7,8-tetrahydronaphthalen-2-yl)quinolin-2-amine 7

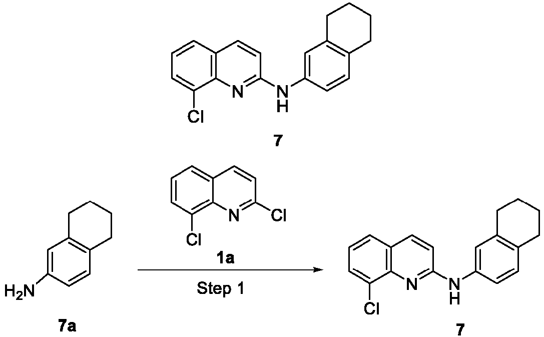

5,6,7,8-Tetrahydro-2-naphthylamine **7a** (147 mg, 0.51 mmol, TCI), 2,8-dichloroquinoline **1a** (100 mg, 0.51 mmol, Bide Pharmatech Ltd.), and trifluoroacetic acid (173 mg, 1.51 mmol) were dissolved in isopropanol (3 mL), and the resulting solution was heated to 100 °C and allowed to react for 12 h. The reaction solution was cooled to room temperature, filtered, and then separated and purified by preparative high performance liquid chromatography (Waters 2767-SQ Detecor2, elution system: a 10 mmol/L aqueous ammonium bicarbonate solution and acetonitrile, gradient of acetonitrile: 60%-80%, flow rate: 30 mL/min) to give the title compound **7** (136 mg, yield: 87%).

MS m/z (ESI): 309.1 [M+1].

¹H NMR (500 MHz, CDCl₃) 7.88 (d, 1H), 7.70 (d, 1H), 7.60-7.47 (m, 2H), 7.29 (dd, 1H), 7.18 (t, 1H), 7.07 (d, 1H), 6.96 (d, 1H), 6.86 (s, 1H), 2.88-2.68 (m, 4H), 1.90-1.74 (m, 4H).

### Example 8

### 8-Chloro-N-(2,2,3,3-tetrafluoro-2,3-dihydrobenzo[b][1,4]dioxin-6-yl)quinolin-2-amine 8

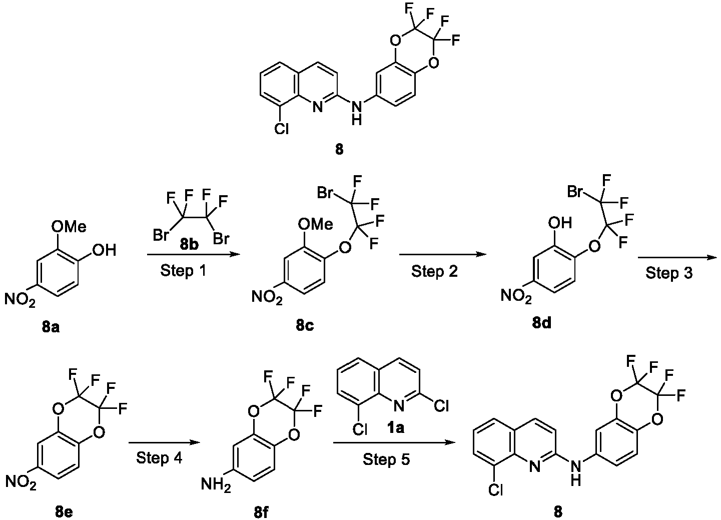

### Step 1

### 1-(2-Bromo-1,1,2,2-tetrafluoroethoxy)-2-methoxy-4-nitrobenzene 8c

2-Methoxy-4-nitrophenol **8a** (2.0 g, 11.82 mmol, Bide Pharmatech Ltd.), cesium carbonate (5.8 g, 17.74 mmol), 1,2-dibromotetrafluoroethane **8b** (6.2 g, 23.65 mmol, Shanghai Titan Scientific Co., Ltd.), and 1-propanethiol (0.45 g, 5.91 mmol, TCI) were dissolved in 30 mL of dimethyl sulfoxide, and the resulting solution was heated to 100 °C and allowed to react for 12 h. The reaction solution was cooled to room temperature, and 50 mL of a 2 N sodium hydroxide solution was added. The resulting mixture was extracted with diethyl ether (50 mL × 3), and the organic phases were combined, then sequentially washed with a 2 N sodium hydroxide solution (50 mL × 3) and a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **8c** (2.6 g, yield: 64%).

### Step 2

### 2-(2-Bromo-1,1,2,2-tetrafluoroethoxy)-5-nitrophenol 8d

Compound **8c** (2.6 g, 7.56 mmol) was added to 46 mL of acetic acid, and a hydrobromic acid solution (40% by mass, 19 mL) was added. The resulting mixture was heated to 120 °C, allowed to react for 30 h, and then cooled to room temperature. 50 mL of water was added, and the mixture was extracted with dichloromethane (50 mL × 3). The organic phase was dried over anhydrous sodium sulfate and filtered. The filtrate was concentrated under reduced pressure, and the resulting residue was purified by silica gel column chromatography with eluent system B to give the title compound **8d** (2.0 g, yield: 79%).

MS m/z (ESI): 331.9 [M-1].

### Step 3

### 2,2,3,3-Tetrafluoro-6-nitro-2,3-dihydrobenzo[b][1,4]dioxine 8e

Compound **8d** (200 mg, 0.60 mmol) was dissolved in methanol, followed by addition of potassium hydroxide (37 mg, 0.66 mmol) and reaction for 1 h. The solvent was removed under reduced pressure, and 4 mL of sulfolane was added. The resulting mixture was heated to 140 °C, allowed to react for 8 h, and then cooled to room temperature. 20 mL of water was added, and the mixture was extracted with ethyl acetate (50 mL × 3), washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **8e** (98 mg, yield: 65%).

### Step 4

### 2,2,3,3-Tetrafluoro-2,3-dihydrobenzo[b][1,4]dioxin-6-amine 8f

Compound **8e** (95 mg, 0.38 mmol) was dissolved in 3 mL of methanol, followed by addition of platinum dioxide. The resulting mixture was purged with hydrogen three times to expel the air and allowed to react under a hydrogen atmosphere for 4 h. The reaction mixture was filtered, the filtrate was concentrated under reduced pressure, and the resulting residue was purified by silica gel column chromatography with eluent system B to give the title compound **8f** (60 mg, yield: 72%).

MS m/z (ESI): 224.0 [M+1].

### Step 5

### 8-Chloro-N-(2,2,3,3-tetrafluoro-2,3-dihydrobenzo[b][1,4]dioxin-6-yl)quinolin-2-amine 8

Compound **8f** (60 mg, 0.27 mmol), 2,8-dichloroquinoline **1a** (50 mg, 0.25 mmol, Bide Pharmatech Ltd.), potassium carbonate (32 mg, 0.30 mmol), 4,5-bis(diphenylphosphino)-9,9-dimethylxanthene (52 mg, 0.1 mmol), and tris(dibenzylideneacetone)dipalladium(0) (28 mg, 0.03 mmol) were dissolved in 3 mL of 1,4-dioxane, and the reaction solution was heated to 100 °C, allowed to react for 12 h, and then cooled to room temperature. 30 mL of water was added, and the resulting mixture was extracted with ethyl acetate (50 mL × 3), washed with a saturated sodium chloride solution (50 mL × 2), dried over anhydrous sodium sulfate, and filtered. The filtrate was concentrated under reduced pressure, and the residue was purified by preparative high performance liquid chromatography (Waters 2767-SQ Detecor2, elution system: a 10 mmol/L aqueous ammonium bicarbonate solution and acetonitrile, gradient of acetonitrile: 80%-95%, flow rate: 30 mL/min) to give the title compound **8** (23 mg, yield: 24%).

MS m/z (ESI): 385.0 [M+1].

¹H NMR (500 MHz, CDCl₃) 8.34 (s, 1H), 7.93 (d, 1H), 7.75 (d, 1H), 7.57 (d, 1H), 7.34 (dd, 1H), 7.25 (t, 1H), 7.09 (d, 1H), 6.96-6.76 (m, 2H).

### Example 9

### N-(8-Chloroquinolin-2-yl)-[1,3]dioxolo[4,5-b]pyridin-6-amine 9

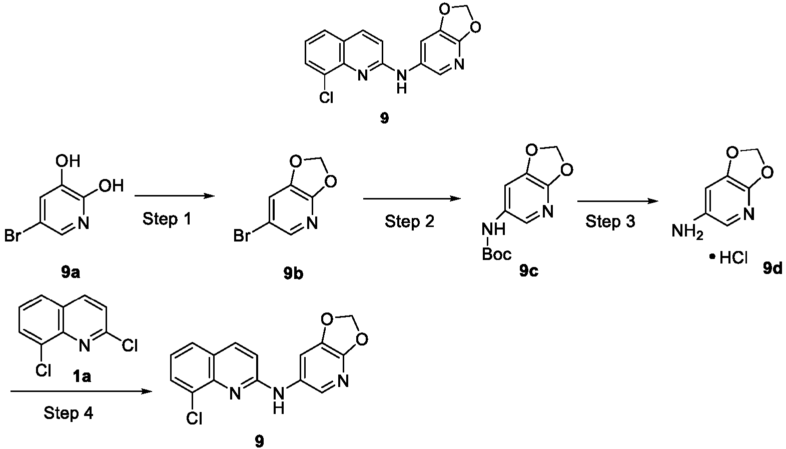

### Step 1

### 6-Bromo-[1,3]dioxolo[4,5-b]pyridine 9b

2,3-Dihydroxy-5-bromopyridine **9a** (2.0 g, 10.53 mmol, Bide Pharmatech Ltd.), anhydrous potassium carbonate (4.36 g, 31.55 mmol), dibromomethane (2.2 g, 12.65 mmol) were dissolved in 30 mL of N-methylpyrrolidone, and the resulting solution was heated to 100 °C, allowed to react for 12 h, then cooled to room temperature, and poured into 30 mL of water. The resulting mixture was extracted with ethyl acetate (50 mL × 3), and the organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **9b** (300 mg, yield: 14%).

MS m/z (ESI): 201.9 [M+1], 203.9 [M+3].

### Step 2

### tert-Butyl [1,3]dioxolo[4,5-b]pyridin-6-ylcarbamate 9c

Compound **9b** (100 mg, 0.49 mmol) and *tert*-butyl carbamate (104 mg, 0.89 mmol) were dissolved in 8 mL of 1,4-dioxane, then tris(dibenzylideneacetone)dipalladium(0) (45 mg, 0.049 mmol), 2-dicyclohexylphosphino-2',4',6'-triisopropylbiphenyl (47 mg, 0.098 mmol) and sodium *tert*-butoxide (85 mg, 0.89 mmol) were added under a nitrogen atmosphere, and the resulting mixture was heated to 105 °C and allowed to react for 20 h. The reaction mixture was cooled to room temperature, and 20 mL of water was added. The resulting mixture was extracted with ethyl acetate (20 mL × 3) and washed with a saturated sodium chloride solution (30 mL). The organic phase was dried over anhydrous sodium sulfate and filtered. The filtrate was concentrated under reduced pressure, and the resulting residue was purified by silica gel column chromatography with eluent system B to give the title compound **9c** (50 mg, yield: 42%).

MS m/z (ESI): 239.1 [M+1].

### Step 3

### [1,3]Dioxolo[4,5-b]pyridin-6-amine hydrochloride 9d

Compound **9c** (50 mg, 0.21 mmol) was dissolved in 5 mL of dichloromethane, and a 4 N solution of hydrogen chloride in dioxane (425 µL, 1.7 mmol) was added dropwise under an ice bath. After the addition was completed, the resulting mixture was naturally warmed to room temperature and was allowed to react at room temperature for 3 h. The reaction mixture was concentrated under reduced pressure to give the title compound **9d** (crude product, 36 mg), which was directly used in the next step.

MS m/z (ESI): 139.0 [M+1].

### Step 4

### N-(8-Chloroquinolin-2-yl)-[1,3]dioxolo[4,5-b]pyridin-6-amine 9

Compound **9d** (36 mg, 0.21 mmol), 2,8-dichloroquinoline **1a** (30 mg, 0.15 mmol, Bide Pharmatech Ltd.), cesium carbonate (148 mg, 0.45 mmol), and methanesulfonato(2-dicyclohexylphosphino-3,6-dimethoxy-2',4',6'-triisopropoxy-1,1'-bi phenyl)(2'-amino-1,1'-biphenyl-2-yl)palladium(II) (20 mg, 0.02 mmol) were dissolved in 5 mL of 1,4-dioxane, and the reaction solution was heated to 100 °C, allowed to react for 12 h, and then cooled to room temperature. 30 mL of water was added, and the resulting mixture was extracted with ethyl acetate (50 mL × 3), washed with a saturated sodium chloride solution (50 mL × 2), dried over anhydrous sodium sulfate, and filtered. The filtrate was concentrated under reduced pressure, and the residue was separated and purified by preparative high performance liquid chromatography (Waters 2767-SQ Detecor2, elution system: a one-thousandth aqueous trifluoroacetic acid solution and acetonitrile, gradient of acetonitrile: 50%-95%, flow rate: 30 mL/min) to give the title compound **9** (12 mg, yield: 26%).

MS m/z (ESI): 300.0 [M+1].

¹H NMR (500 MHz, DMSO-*d*₆) 9.80 (s, 1H), 8.46 (d, 1H), 8.18 (d, 1H), 8.13 (d, 1H), 7.78 (d, 1H), 7.74 (d, 1H), 7.28 (t, 1H), 7.10(d, 1H), 6.14 (s, 2H).

### Example 10

### N-(Benzo[d][1,3]dioxol-5-yl)-8-chloroquinolin-2-amine 10

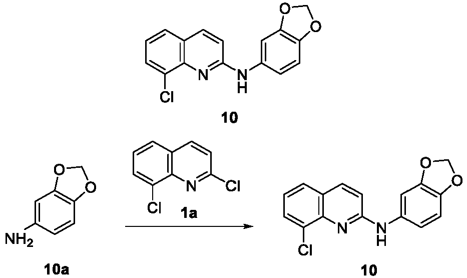

Compound benzo[*d*][1,3]dioxol-5-amine **10a** (693 mg, 5.05 mmol, Bide Pharmatech Ltd.), compound **1a** (1.0 g, 5.05 mmol), cesium carbonate (2.64 g, 8.10 mmol), 4,5-bis(diphenylphosphino)-9,9-dimethylxanthene (584 mg, 1.01 mmol), and tris(dibenzylideneacetone)dipalladium(0) (462 mg, 0.50 mol) were dissolved in 30 mL of 1,4-dioxane, and the reaction solution was heated to 105 °C, allowed to react for 12 h, and then cooled to room temperature. 25 mL of water was added, and the resulting mixture was extracted with ethyl acetate (25 mL × 3), washed with a saturated sodium chloride solution (25 mL × 2), dried over anhydrous sodium sulfate, and filtered. The filtrate was concentrated under reduced pressure, and the residue was purified by preparative high performance liquid chromatography (Waters-2545, elution system: a 10 mmol/L aqueous ammonium bicarbonate solution and acetonitrile, gradient of acetonitrile: 10%-26%, flow rate: 30 mL/min) to give the target compound **10** (750 mg, yield: 50%).

MS m/z (ESI): 299.0 [M+1].

¹H NMR (500 MHz, DMSO-*d*₆) 9.64 (s, 1H), 8.30 (d, 1H), 8.08 (d, 1H), 7.75 (dd, 1H), 7.70 (dd, 1H), 7.28 (dd, 1H), 7.25 (t, 1H), 7.07(d, 1H), 6.90(d, 1H), 5.99 (s, 2H).

### Example 11

### N-(Benzo[d][1,3]dioxol-5-yl-2,2-d₂)-8-chloroquinolin-2-amine 11

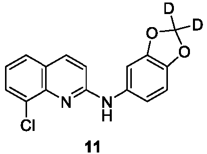

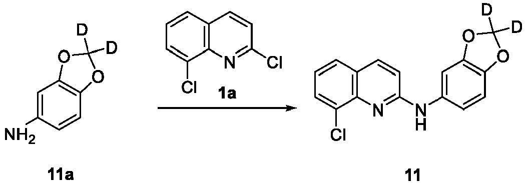

Compound benzo[*d*][1,3]dioxol-2,2-*d*₂-5-amine **11a** (170 mg, 1.21 mmol, prepared by the method disclosed in intermediate synthesis method 2 on page 41 of the specification in the patent application "WO2012037351A1"), compound **1a** (240 mg, 1.21 mmol), cesium carbonate (636 mg, 1.95 mmol), 4,5-bis(diphenylphosphino)-9,9-dimethylxanthene (141 mg, 0.24 mmol), and tris(dibenzylideneacetone)dipalladium(0) (111 mg, 0.12 mol) were dissolved in 10 mL of 1,4-dioxane, and the reaction solution was heated to 105 °C, allowed to react for 12 h, and then cooled to room temperature. 25 mL of water was added, and the resulting mixture was extracted with ethyl acetate (25 mL × 3), washed with a saturated sodium chloride solution (25 mL × 2), dried over anhydrous sodium sulfate, and filtered. The filtrate was concentrated under reduced pressure, and the residue was separated and purified by preparative high performance liquid chromatography (Waters-2767 Autopurification, elution system: a one-thousandth aqueous formic acid solution and acetonitrile, gradient of acetonitrile: 45%-95%, flow rate: 30 mL/min) to give the target compound **11** (100 mg, yield: 27%).

MS m/z (ESI): 300.9 [M+1].

¹H NMR (500 MHz, DMSO-*d*₆) 9.64 (s, 1H), 8.29 (d, 1H), 8.08 (d, 1H), 7.75 (dd, 1H), 7.71 (dd, 1H), 7.32-7.18 (m, 2H), 7.07 (d, 1H), 6.89 (d, 1H).

### Example 12

### 8-Chloro-N-(2,2-dimethylbenzo[d][1,3]dioxol-5-yl)quinolin-2-amine 12

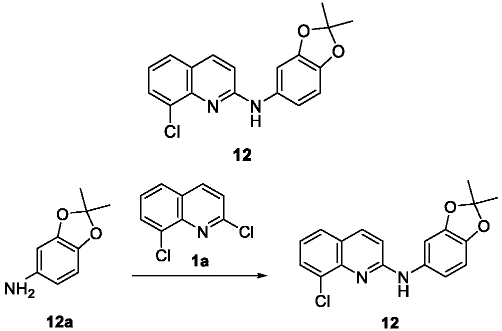

Compound 2,2-dimethylbenzo[*d*][1,3]dioxol-5-amine **12a** (141 mg, 0.85 mmol, Shanghai Haohong Scientific Co., Ltd.), compound **1a** (146 mg, 0.74 mmol), cesium carbonate (362 mg, 1.11 mmol), tris(dibenzylideneacetone)dipalladium(0) (68 mg, 0.074 mmol), and 4,5-bis(diphenylphosphino)-9,9-dimethylxanthene (86 mg, 0.15 mmol) were dissolved in 5 mL of 1,4-dioxane, and the reaction solution was heated to 105 °C, allowed to react for 12 h, and then cooled to room temperature. 30 mL of water was added, and the resulting mixture was extracted with ethyl acetate (50 mL × 3), washed with a saturated sodium chloride solution (50 mL × 2), dried over anhydrous sodium sulfate, and filtered. The filtrate was concentrated under reduced pressure, and separated and purified by preparative high performance liquid chromatography (Waters-2545, elution system: a 10 mmol/L aqueous ammonium bicarbonate solution and acetonitrile, gradient of acetonitrile: 60%-95%, flow rate: 30 mL/min) to give the target compound **12** (132 mg, yield: 55%).

MS m/z (ESI): 327.1 [M+1].

¹H NMR (500 MHz, DMSO-*d*₆) δ 9.59 (s, 1H), 8.18 (d, 1H), 8.06 (d, 1H), 7.74 (dd, 1H), 7.69 (dd, 1H), 7.30-7.17 (m, 2H), 7.06 (d, 1H), 6.79 (d, 1H), 1.65 (s, 6H).

### Example 13

### 8-Chloro-N-(2,2-dimethyl-2,3-dihydrobenzofuran-5-yl)quinolin-2-amine 13

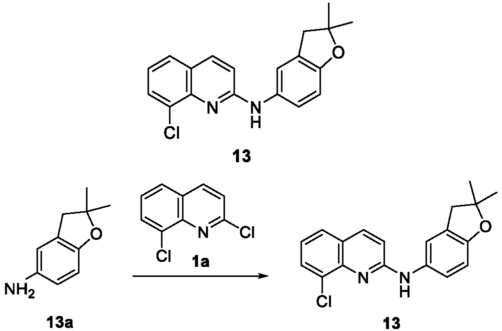

Compound 2,2-dimethyl-2,3-dihydrobenzofuran-5-amine **13a** (59 mg, 0.36 mmol, prepared by the method disclosed in Example 13 on page 72 of the specification in the patent application "WO2014169845"), compound **1a** (60 mg, 0.30 mmol), trifluoroacetic acid (62 mg, 0.54 mmol), and 2 mL of isopropanol were placed in a 25 mL sealed tube, and the reaction mixture was heated to 90 °C, allowed to react for 12 h, and then cooled to room temperature. 15 mL of water was added, and the resulting mixture was adjusted to about pH 8 with a saturated sodium bicarbonate solution, then extracted with ethyl acetate (25 mL × 3), washed with a saturated sodium chloride solution (25 mL × 2), dried over anhydrous sodium sulfate, and filtered. The filtrate was concentrated under reduced pressure, and the residue was separated and purified by preparative high performance liquid chromatography (Waters-2767 Autopurification, elution system: a one-thousandth aqueous formic acid solution and methanol, gradient of methanol: 60%-95%, flow rate: 30 mL/min) to give the target compound **13** (55 mg, yield: 56%).

MS m/z (ESI): 325.1 [M+1].

¹H NMR (500 MHz, DMSO-*d*₆) 9.47 (s, 1H), 8.18 (s, 1H), 8.04 (dd, 1H), 7.78-7.60 (m, 3H), 7.22 (td, 1H), 7.05 (dd, 1H), 6.67 (dd, 1H), 3.03 (s, 2H), 1.42 (s, 6H).

### Example 14

### 5-Chloro-N-(2,2-difluorobenzo[d][1,3]dioxol-5-yl)-8,9-dihydro-7H-cyclopenta[f]quinoli n-3-amine 14

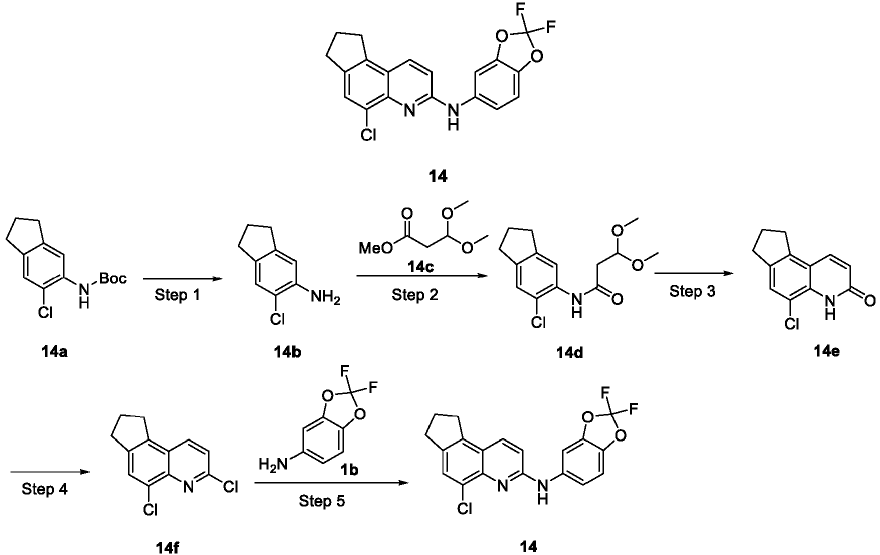

### Step 1

### 6-Chloro-2,3-dihydro-1H-inden-5-amine 14b

Dichloromethane (50 mL), *tert*-butyl (6-chloro-2,3-dihydro-1*H*-inden-5-yl)carbamate **14a** (3.4 g, 12.73 mmol, prepared by a well-known method *"*ACS Catalysis, 2018, 8, 4783-4788"), and trifluoroacetic acid (10 mL) were added sequentially to a 100 mL single-necked flask, and the resulting mixture was allowed to react at room temperature for 3 h, then concentrated under reduced pressure to remove the solvent, diluted with 100 mL of dichloromethane, and sequentially washed with a saturated sodium bicarbonate solution (50 mL × 2) and a saturated sodium chloride solution (50 mL × 2). The organic phase was dried over anhydrous sodium sulfate and filtered, and the filtrate was concentrated under reduced pressure to give the title compound **14b** (crude product, 1.55 g, yield: 73%), which was directly used in the next step.

MS m/z (ESI): 168.1 [M+1].

### Step 2

### N-(6-Chloro-2,3-dihydro-1H-inden-5-yl)-3,3-dimethoxypropanamide 14d

Tetrahydrofuran (16 mL), compound **14b** (1.55 g, 9.28 mmol), and methyl 3,3-dimethoxypropionate **14c** (1.65 g, 11.14 mmol, J&K Scientific) were added sequentially to a 100 mL three-necked flask, and then sodium bis(trimethylsilyl)amide (a 2 M solution in tetrahydrofuran, 6.96 mL, 13.92 mmol) was slowly added dropwise at 0 °C. The resulting mixture was naturally warmed to room temperature and was allowed to react for 12 h. 50 mL of a saturated ammonium bicarbonate solution was added to the system under an ice bath to quench the reaction, and the mixture was extracted with ethyl acetate (50 mL × 3). The organic phases were combined, washed with a saturated sodium chloride solution (50 mL × 3), dried, and filtered. The filtrate was concentrated under reduced pressure, and the resulting residue was purified by silica gel column chromatography with eluent system B to give the title compound **14d** (950 mg, yield: 36%).

### Step 3

### 5-Chloro-4,7,8,9-tetrahydro-3H-cyclopenta[f]quinolin-3-one 14e

Dichloromethane (10 mL) and compound **14d** (800 mg, 2.83 mmol) were added sequentially to a 50 mL single-necked flask, and concentrated sulfuric acid (2.27 mL, 42.40 mmol) was slowly added dropwise to the system at 0 °C. After the addition was completed, the resulting mixture was allowed to react at room temperature for 30 min and then concentrated under reduced pressure to remove the solvent. The residue was dropped into ice water, and a solid was precipitated. The mixture was filtered, and the solid was dried to give the title compound **14e** (500 mg, yield: 81%).

MS m/z (ESI): 220.0 [M+1].

### Step 4

### 3,5-Dichloro-8,9-dihydro-7H-cyclopenta[f]quinoline 14f

*N,N*-Dimethylformamide (2 mL) and compound **14e** (300 mg, 1.37 mmol) were added sequentially to a 50 mL single-necked flask. The system was heated to 95 °C, and then phosphorus oxychloride (0.1 mL, 1.10 mmol) was slowly added dropwise. The resulting mixture was allowed to react for 30 min, then concentrated under reduced pressure to remove the phosphorus oxychloride, diluted with 20 mL of ethyl acetate, and sequentially washed with a 0.2 N sodium hydroxide solution (30 mL × 2) and a saturated sodium chloride solution (30 mL × 3). The organic phase was dried over anhydrous sodium sulfate and filtered, and the filtrate was concentrated under reduced pressure to give the title compound **14f** (crude product, 0.25 g, yield: 78%), which was directly used in the next step.

MS m/z (ESI): 238.0 [M+1].

### Step 5

### 5-Chloro-N-(2,2-difluorobenzo[d][1,3]dioxol-5-yl)-8,9-dihydro-7H-cyclopenta[f]quinoli n-3-amine 14

Isopropanol (2 mL), compound **14f** (100 mg, 0.42 mmol), trifluoroacetic acid (47.89 mg, 0.42 mmol), and compound **1b** (73 mg, 0.42 mmol) were added sequentially to a 25 mL single-necked flask, and then the resulting mixture was heated to 85 °C, allowed to react for 16 h, then concentrated under reduced pressure to remove the solvent, diluted with 1 mL of methanol, and purified by thin-layer chromatography (TLC) with developing solvent system B to give the title compound **14** (78 mg, yield: 49%).

MS m/z (ESI): 375.0 [M+1].

¹H NMR (400 MHz, DMSO-*d₆*) δ 9.90 (s, 1H), 8.89 (d, 1H), 8.08 (d, 1H), 7.72 (s, 1H), 7.48-7.45 (m, 1H), 7.37 (d, 1H), 7.13 (d, 1H), 3.15 (t, 2H), 3.02 (t, 2H), 2.12-2.14 (m, 2H).

### Example 15

### 8-Chloro-N-(2,2-difluorobenzo[d][1,3]dioxol-5-yl)-7-methylquinolin-2-amine 15

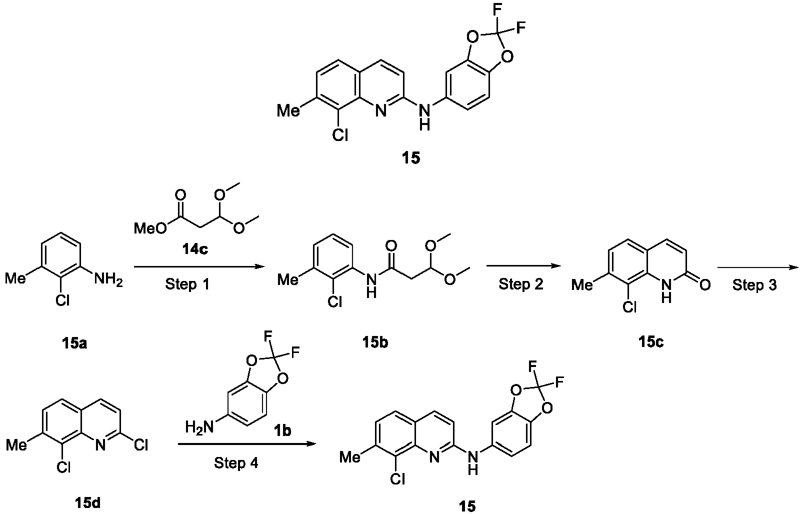

### Step 1

### N-(2-chloro-3-methylphenyl)-3,3-dimethoxypropanamide 15b

Tetrahydrofuran (25 mL), 2-chloro-3-methylaniline **15a** (1.5 g, 10.59 mmol, Bide Pharmatech Ltd.), and compound **14c** (1.88 g, 11.69 mmol) were added sequentially to a 100 mL three-necked flask. The system was cooled to 0 °C, and then sodium bis(trimethylsilyl)amide (a 2 M solution in tetrahydrofuran, 10.57 mL, 21.14 mmol) was slowly added dropwise. The system was then warmed to room temperature and allowed to react for 24 h. 50 mL of a saturated aqueous sodium bicarbonate solution was added to the system to quench the reaction, and the reaction mixture was concentrated under reduced pressure to remove most of the organic phases and then extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure to give the title compound **15b** (crude product, 2.70 g, yield: 98%), which was directly used in the next step.

### Step 2

### 8-Chloro-7-methylquinolin-2(1H)-one 15c

Dichloromethane (15 mL) and compound **15b** (2.70 g, 10.48 mmol) were added sequentially to a 100 mL single-necked flask, and concentrated sulfuric acid (8.37 mL, 157.11 mmol) was slowly added dropwise at 0 °C. After the addition was completed, the ice bath was removed, and the mixture was allowed to react for 16 h and then concentrated under reduced pressure to remove the solvent. The residue was dropped into ice water, and a solid was precipitated. The mixture was filtered, and the solid was dried to give the title compound **15c** (1.8 g, yield: 89%).

MS m/z (ESI): 194.0 [M+1].

### Step 3

### 2,8-Dichloro-7-methylquinoline 15d

Compound **15c** (800 mg, 4.67 mmol) and phosphorus oxychloride (3.16 g, 20.66 mmol) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was adjusted to about pH 8 with a saturated sodium bicarbonate solution and then extracted with ethyl acetate (50 mL × 3). The organic phase was washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **15d** (620 mg, yield: 71%).

MS m/z (ESI): 211.9 [M+1].

### Step 4

### 8-Chloro-N-(2,2-difluorobenzo[d][1,3]dioxol-5-yl)-7-methylquinolin-2-amine 15

Isopropanol (8 mL), compound **15d** (300 mg, 1.29 mmol), compound **1b** (268 mg, 1.54 mmol), and trifluoroacetic acid (260 mg, 2.28 mmol) were added sequentially to a 25 mL sealed tube. Then, the resulting mixture was heated to 95 °C, allowed to react for 16 h, then concentrated under reduced pressure to remove the solvent, diluted with 100 mL of ethyl acetate, and sequentially washed with a saturated sodium bicarbonate solution (50 mL × 2) and a saturated sodium chloride solution (50 mL × 2). The organic phase was dried over anhydrous sodium sulfate and filtered. The filtrate was concentrated under reduced pressure, and the residue was purified by preparative high performance liquid chromatography (Waters-2767 Autopurification, elution system: a one-thousandth aqueous formic acid solution and methanol, gradient of methanol: 65%-95%, flow rate: 30 mL/min) to give the title compound **15** (300 mg, yield: 73%).

MS m/z (ESI): 349.1 [M+1].

¹H NMR (500 MHz, DMSO-d₆) δ 9.93 (s, 1H), 8.90 (s, 1H), 8.11 (d, 1H), 7.65 (d, 1H), 7.49 (d, 1H), 7.37 (d, 1H), 7.30 (d, 1H), 7.07 (d, 1H), 2.55 (s, 3H).

### Example 16

### 8-Chloro-N-(2,2-difluorobenzo[d][1,3]dioxol-5-yl)-7-methoxyquinolin-2-amine 16

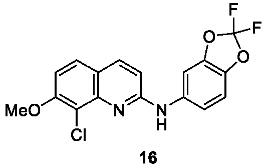

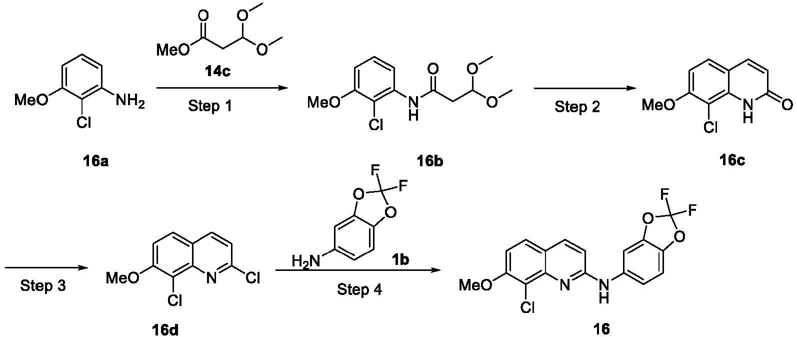

### Step 1

### N-(2-Chloro-3-methoxyphenyl)-3,3-dimethoxypropanamide 16b

Tetrahydrofuran (25 mL), 2-chloro-3-methoxyaniline **16a** (1.5 g, 9.51 mmol, Bide Pharmatech Ltd.), and compound **14c** (1.69 g, 11.40 mmol) were added sequentially to a 100 mL three-necked flask, and sodium bis(trimethylsilyl)amide (a 2 M solution in tetrahydrofuran, 9.51 mL, 19.03 mmol) was slowly added dropwise at 0 °C. The resulting mixture was naturally warmed to room temperature and was allowed to react for 24 h. 50 mL of a saturated sodium bicarbonate solution was added to the system to quench the reaction, and the reaction mixture was concentrated under reduced pressure to remove most of the organic phases and then extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure to give the title compound **16b** (crude product, 2.50 g, yield: 96%), which was directly used in the next step.

### Step 2

### 8-Chloro-7-methoxyquinolin-2(1H)-one 16c

Dichloromethane (10 mL) and compound **16b** (2.50 g, 9.13 mmol) were added sequentially to a 100 mL single-necked flask, and concentrated sulfuric acid (7.29 mL, 136.93 mmol) was slowly added dropwise at 0 °C. The ice bath was then removed, and the mixture was allowed to react for 16 h and then concentrated under reduced pressure to remove the solvent. The residue was dropped into ice water, and a solid was precipitated. The mixture was filtered, and the solid was dried to give the title compound **16c** (1.6 g, yield: 84%).

MS m/z (ESI): 210.0 [M+1].

### Step 3

### 2,8-Dichloro-7-methoxyquinoline 16d

Toluene (8 mL), compound **16c** (800 mg, 3.81 mmol), and phosphorus oxychloride (1.17 g, 7.63 mmol) were added sequentially to a 50 mL single-necked flask, and the system was heated to 100 °C and allowed to react for 3 h. The reaction mixture was cooled to room temperature, and 20 mL of ice water was added. The resulting mixture was adjusted to about pH 8 with saturated sodium bicarbonate and then extracted with ethyl acetate (50 mL × 3). The organic phase was washed with a saturated sodium chloride solution (50 mL × 2), dried over anhydrous sodium sulfate, filtered, and concentrated, and the resulting residue was purified by silica gel column chromatography with eluent system B to give the title compound **16d** (680 mg, yield: 78%).

MS m/z (ESI): 227.9 [M+1].

### Step 4

### 8-Chloro-N-(2,2-difluorobenzo[d][1,3]dioxol-5-yl)-7-methoxyquinolin-2-amine 16

Isopropanol (4 mL), compound **16d** (250 mg, 1.09 mmol), compound **1b** (227 mg, 1.31 mmol), and trifluoroacetic acid (224 mg, 1.96 mmol) were added sequentially to a 25 mL sealed tube, and then the resulting mixture was heated to 95 °C, allowed to react for 16 h, then concentrated under reduced pressure to remove the solvent, diluted with 100 mL of ethyl acetate, and sequentially washed with a saturated sodium bicarbonate solution (50 mL × 2) and a saturated sodium chloride solution (50 mL × 2). The organic phase was dried over anhydrous sodium sulfate, filtered, and concentrated, and the resulting residue was purified by silica gel column chromatography with eluent system B to give the target product **16** (280 mg, yield: 70%).

MS m/z (ESI): 365.1 [M+1].

¹H NMR (400 MHz, DMSO-d₆) δ 9.92 (s, 1H), 8.92 (s, 1H), 8.09 (d, 1H), 7.76 (d, 1H), 7.49 (d, 1H), 7.38 (d, 1H), 7.31 (d, 1H), 6.97 (d, 1H), 4.00 (s, 3H).

### Example 17

### 7,8-Dichloro-N-(2,2-difluorobenzo[d][1,3]dioxol-5-yl)quinolin-2-amine 17

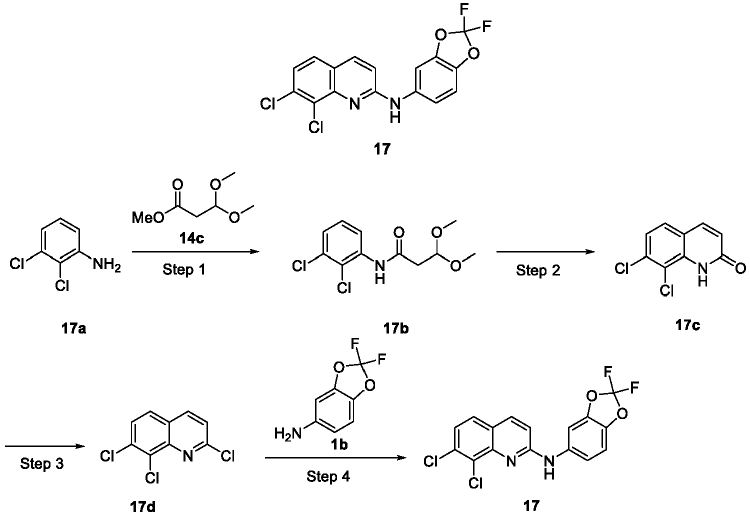

### Step 1

### N-(2,3-Dichlorophenyl)-3,3-dimethoxypropanamide 17b

Tetrahydrofuran (25 mL), 2,3-dichloroaniline **17a** (1.5 g, 9.26 mmol, J&K Scientific), and compound **14c** (1.65 g, 11.11 mmol) were added sequentially to a 100 mL three-necked flask, and sodium bis(trimethylsilyl)amide (a 2 M solution in tetrahydrofuran, 9.30 mL, 18.60 mmol) was slowly added dropwise at 0 °C. The system was then warmed to room temperature and allowed to react for 24 h. 50 mL of a saturated aqueous sodium bicarbonate solution was added to the system to quench the reaction, and the reaction mixture was concentrated under reduced pressure to remove most of the organic phases and then extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure to give the title compound **17b** (crude product, 2.50 g, yield: 97%), which was directly used in the next step.

### Step 2

### 7,8-Dichloroquinolin-2(1H)-one 17c

Dichloromethane (15 mL) and compound **17b** (2.50 g, 8.99 mmol) were added sequentially to a 100 mL single-necked flask, and concentrated sulfuric acid (7.18 mL, 134.79 mmol) was slowly added dropwise at 0 °C. The ice bath was then removed, and the mixture was allowed to react for 16 h and then concentrated under reduced pressure to remove the solvent. The residue was dropped into ice water, and a solid was precipitated. The mixture was filtered, and the solid was dried to give the title compound **17c** (1.70 g, yield: 89%).

MS m/z (ESI): 214.0 [M+1].

### Step 3

### 2,7,8-Trichloroquinoline 17d

Compound **17c** (1.0 g, 4.67 mmol) and phosphorus oxychloride (3.58 g, 23.36 mmol) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was adjusted to about pH 8 with saturated sodium bicarbonate and then extracted with ethyl acetate (50 mL × 3). The organic phase was washed with a saturated sodium chloride solution (50 mL × 2), dried over anhydrous sodium sulfate, filtered, and concentrated, and the resulting residue was purified by silica gel column chromatography with eluent system B to give the title compound **17d** (950 mg, yield: 87%).

MS m/z (ESI): 231.9 [M+1].

### Step 4

### 7,8-Dichloro-N-(2,2-difluorobenzo[d][1,3]dioxol-5-yl)quinolin-2-amine 17

Isopropanol (8 mL), compound **17d** (300 mg, 1.29 mmol), compound **1b** (268 mg, 1.54 mmol), and trifluoroacetic acid (260 mg, 2.28 mmol) were added sequentially to a 25 mL sealed reaction flask, and then the resulting mixture was heated to 95 °C, allowed to react for 16 h, then concentrated under reduced pressure to remove the solvent, diluted with 100 mL of ethyl acetate, and sequentially washed with a saturated sodium bicarbonate solution (50 mL × 2) and a saturated sodium chloride solution (50 mL × 2). The organic phase was dried over anhydrous sodium sulfate, filtered, and concentrated, and the resulting residue was purified by silica gel column chromatography with eluent system A to give the target product **17** (300 mg, yield: 63%).

MS m/z (ESI): 369.0 [M+1].

¹H NMR (400 MHz, DMSO-*d₆*) δ 10.06 (s, 1H), 8.76 (s, 1H), 8.16 (d, 1H), 7.76 (d, 1H), 7.54-7.45 (m, 2H), 7.38 (d, 1H), 7.12 (d, 1H).

### Example 18

### 8-Chloro-N-(2,2-difluorobenzo[d][1,3]dioxol-5-yl)-6-methylquinolin-2-amine 18

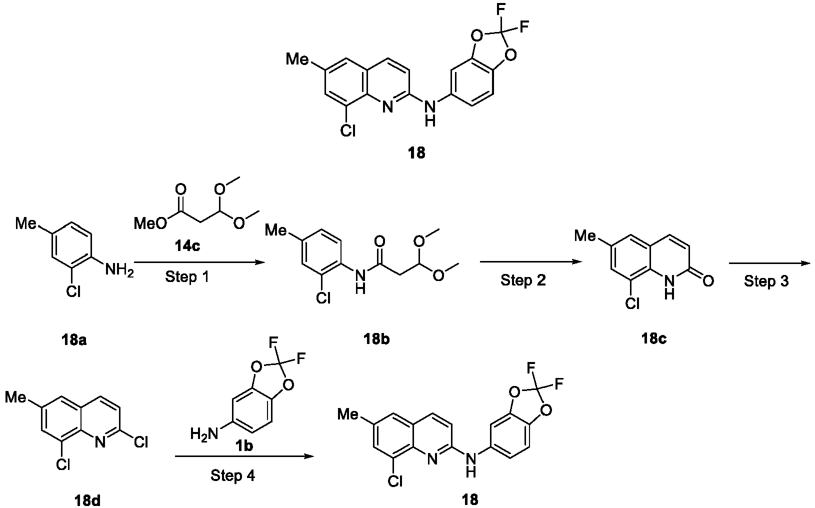

### Step 1

### N-(2-Chloro-4-methylphenyl)-3,3-dimethoxypropanamide 18b

Tetrahydrofuran (25 mL), 2-chloro-4-methylaniline **18a** (1 g, 7.06 mmol, Bide Pharmatech Ltd.), and compound **14c** (1.26 g, 8.47 mmol) were added sequentially to a 100 mL three-necked flask, and sodium bis(trimethylsilyl)amide (a 2 M solution in tetrahydrofuran, 5.30 mL, 10.60 mmol) was slowly added dropwise at 0 °C. The system was then warmed to room temperature and allowed to react for 24 h. 50 mL of a saturated aqueous sodium bicarbonate solution was added to the system to quench the reaction, and the reaction mixture was concentrated under reduced pressure to remove most of the organic phases and then extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **18b** (1.50 g, yield: 82%).

### Step 2

### 8-Chloro-6-methylquinolin-2(1H)-one 18c

Dichloromethane (2 mL) and compound **18b** (1.50 g, 5.82 mmol) were added sequentially to a 100 mL single-necked flask, and concentrated sulfuric acid (4.7 mL, 87.31 mmol) was slowly added dropwise at 0 °C. The ice bath was then removed, and the mixture was allowed to react for 4 h and then concentrated under reduced pressure to remove the solvent. The residue was dropped into ice water, and a solid was precipitated. The mixture was filtered, and the solid was dried to give the title compound **18c** (0.8 g, yield: 71%).

MS m/z (ESI): 194.0 [M+1].

### Step 3

### 2,8-Dichloro-6-methylquinoline 18d

Compound **18c** (0.5 g, 2.58 mmol) and phosphorus oxychloride (2 mL) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was extracted with ethyl acetate (50 mL × 3), and the organic phase was washed with a saturated sodium chloride solution (50 mL × 2), dried over anhydrous sodium sulfate, filtered, and concentrated. The resulting residue was purified by silica gel column chromatography with eluent system B to give the title compound **18d** (0.3 g, yield: 55%).

MS m/z (ESI): 212.0 [M+1].

### Step 4

### 8-Chloro-N-(2,2-difluorobenzo[d][1,3]dioxol-5-yl)-6-methylquinolin-2-amine 18

Isopropanol (3 mL), compound **18d** (50 mg, 0.24 mmol), compound **1b** (102 mg, 0.59 mmol), and trifluoroacetic acid (67 mg, 0.59 mmol) were added sequentially to a 25 mL sealed tube, and then the resulting mixture was heated to 95 °C, allowed to react for 16 h, and then cooled to room temperature. 25 mL of water was added, and the mixture was extracted with ethyl acetate (25 mL × 3), washed with a saturated sodium chloride solution (25 mL × 2), dried over anhydrous sodium sulfate, and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 70%-90%, flow rate: 30 mL/min) to give the title compound **18** (55 mg, yield: 67%).

MS m/z (ESI): 349.1 [M+1].

¹H NMR (500 MHz, CDCl₃) 8.26 (s, 1H), 7.87 (d, 1H), 7.60 (s, 1H), 7.35 (s, 1H), 7.14 (m, 1H), 7.02 (d, 1H), 6.85 (d, 1H), 6.79 (s, 1H), 2.46 (s, 3H).

### Example 19

### 8-Chloro-N-(2,2-difluorobenzo[d][1,3]dioxol-5-yl)-6-methoxyquinolin-2-amine 19

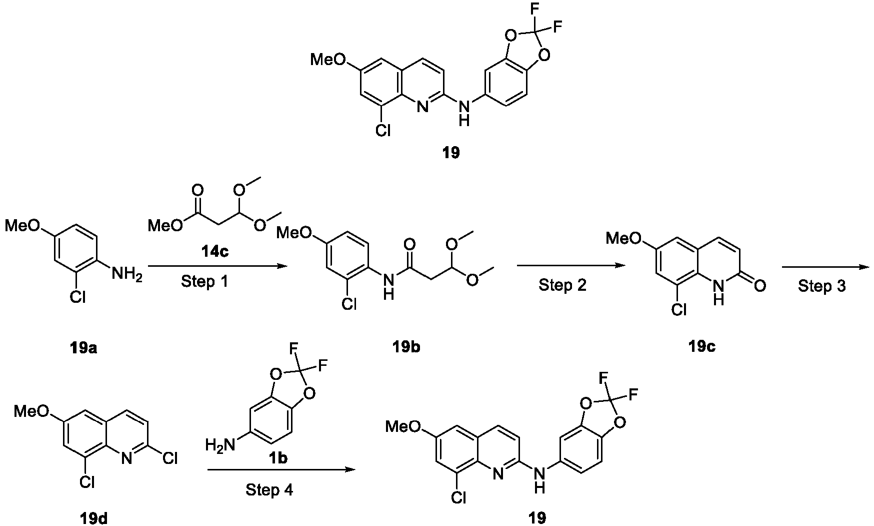

### Step 1

### N-(2-Chloro-4-methoxyphenyl)-3,3-dimethoxypropanamide 19b

Tetrahydrofuran (25 mL), 2-chloro-4-methoxyaniline **19a** (1 g, 6.35 mmol, Bide Pharmatech Ltd.), and compound **14c** (1.13 g, 7.61 mmol) were added sequentially to a 100 mL three-necked flask, and sodium bis(trimethylsilyl)amide (a 2 M solution in tetrahydrofuran, 4.76 mL, 9.52 mmol) was slowly added dropwise at 0 °C. The system was then warmed to room temperature and allowed to react for 24 h. 50 mL of a saturated aqueous sodium bicarbonate solution was added to the system to quench the reaction, and the reaction mixture was concentrated under reduced pressure to remove most of the organic phases and then extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **19b** (1.20 g, yield: 69%).

### Step 2

### 8-Chloro-6-methoxyquinolin-2(1H)-one 19c

Dichloromethane (2 mL) and compound **19b** (1.20 g, 4.38 mmol) were added sequentially to a 100 mL single-necked flask, and concentrated sulfuric acid (3.52 mL, 65.76 mmol) was slowly added dropwise at 0 °C. The ice bath was then removed, and the mixture was allowed to react for 4 h and then concentrated under reduced pressure to remove the solvent. The residue was dropped into ice water, and a solid was precipitated. The mixture was filtered, and the solid was dried to give the title compound **19c** (0.6 g, yield: 65%).

MS m/z (ESI): 210.0 [M+1].

### Step 3

### 2,8-Dichloro-6-methoxyquinoline 19d

Compound **19c** (500 mg, 2.39 mmol) and phosphorus oxychloride (2.5 mL) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was extracted with ethyl acetate (50 mL × 3), and the organic phase was washed with a saturated sodium chloride solution (50 mL × 2), dried over anhydrous sodium sulfate, filtered, and concentrated. The resulting residue was purified by silica gel column chromatography with eluent system B to give the title compound **19d** (75 mg, yield: 14%).

MS m/z (ESI): 228.0 [M+1].

### Step 4

### 8-Chloro-N-(2,2-difluorobenzo[d][1,3]dioxol-5-yl)-6-methoxyquinolin-2-amine 19

Isopropanol (3 mL), compound **19d** (75 mg, 0.33 mmol), compound **1b** (142 mg, 0.82 mmol), and trifluoroacetic acid (94 mg, 0.82 mmol) were added sequentially to a 25 mL sealed reaction flask, and then the resulting mixture was heated to 95 °C, allowed to react for 16 h, and then cooled to room temperature. 25 mL of water was added, and the mixture was extracted with ethyl acetate (25 mL × 3), washed with a saturated sodium chloride solution (25 mL × 2), dried over anhydrous sodium sulfate, and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 65%-85%, flow rate: 30 mL/min) to give the title compound **19** (55 mg, yield: 67%).

MS m/z (ESI): 365.1 [M+1].

¹H NMR (500 MHz, CDCl₃) 8.22 (s, 1H), 7.85 (d, 1H), 7.45 (s, 1H), 7.11 (s, 1H), 7.00 (d, 1H), 6.94 (s, 1H), 6.85 (d, 1H), 6.73 (s, 1H), 3.89 (s, 3H).

### Example 20

### 6,8-Dichloro-N-(2,2-difluorobenzo[d][1,3]dioxol-5-yl)quinolin-2-amine 20

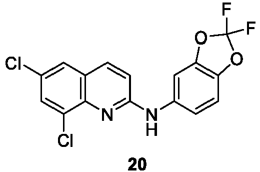

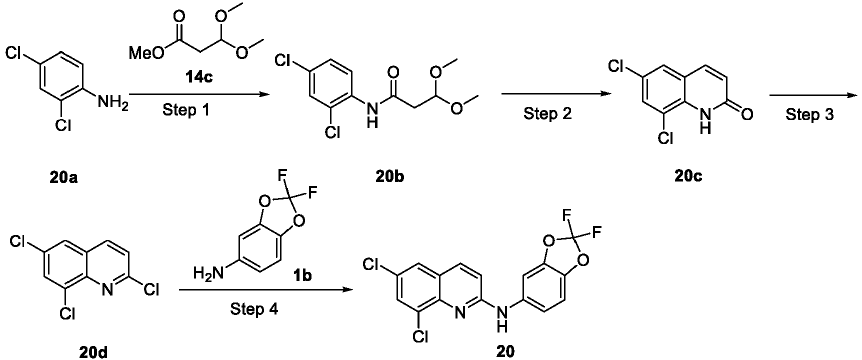

### Step 1

### N-(2,4-Dichlorophenyl)-3,3-dimethoxypropanamide 20b

Tetrahydrofuran (25 mL), 2,4-dichloroaniline **20a** (1.0 g, 6.17 mmol, J&K Scientific), and compound **14c** (1.10 g, 7.41 mmol) were added sequentially to a 100 mL three-necked flask, and sodium bis(trimethylsilyl)amide (a 2 M solution in tetrahydrofuran, 4.63 mL, 9.26 mmol) was slowly added dropwise at 0 °C. The system was then warmed to room temperature and allowed to react for 24 h. 50 mL of a saturated aqueous sodium bicarbonate solution was added to the system to quench the reaction, and the reaction mixture was concentrated under reduced pressure to remove most of the organic phases and then extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **20b** (1.40 g, yield: 82%).

### Step 2

### 6,8-Dichloroquinolin-2(1H)-one 20c

Dichloromethane (2 mL) and compound **20b** (1.20 g, 4.31 mmol) were added sequentially to a 100 mL single-necked flask, and concentrated sulfuric acid (3.52 mL, 65.76 mmol) was slowly added dropwise at 0 °C. The ice bath was then removed, and the mixture was heated to 90 °C, allowed to react for 4 h, and then concentrated under reduced pressure to remove the solvent. The residue was dropped into ice water, and a solid was precipitated. The mixture was filtered, and the solid was dried to give the title compound **20c** (0.5 g, yield: 54%).

MS m/z (ESI): 213.9 [M+1].

### Step 3

### 2,6,8-Trichloroquinoline 20d

Compound **20c** (300 mg, 1.40 mmol) and phosphorus oxychloride (2 mL) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was extracted with ethyl acetate (50 mL × 3), and the organic phase was washed with a saturated sodium chloride solution (50 mL × 2), dried over anhydrous sodium sulfate, filtered, and concentrated. The resulting residue was purified by silica gel column chromatography with eluent system B to give the title compound **20d** (240 mg, yield: 74%).

MS m/z (ESI): 231.9 [M+1].

### Step 4

### 6,8-Dichloro-N-(2,2-difluorobenzo[d][1,3]dioxol-5-yl)quinolin-2-amine 20

Isopropanol (3 mL), compound **20d** (100 mg, 0.43 mmol), compound **1b** (82 mg, 0.47 mmol), and trifluoroacetic acid (123 mg, 1.08 mmol) were added sequentially to a 25 mL sealed tube, and then the resulting mixture was heated to 95 °C, allowed to react for 16 h, and then cooled to room temperature. 25 mL of water was added, and the mixture was extracted with ethyl acetate (25 mL × 3), washed with a saturated sodium chloride solution (25 mL × 2), dried over anhydrous sodium sulfate, and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 80%-95%, flow rate: 30 mL/min) to give the title compound **20** (55 mg, yield: 63%).

MS m/z (ESI): 369.0 [M+1].

¹H NMR (500 MHz, CDCl₃) 8.23 (s, 1H), 7.86 (d, 1H), 7.72 (s, 1H), 7.56 (s, 1H), 7.14 (d, 1H), 7.03 (s, 1H), 6.94-6.72 (m, 2H).

### Example 21

### (2S,3S,4S,5R,6R)-6-(Benzo[d][1,3]dioxol-5-yl(8-chloroquinolin-2-yl)amino)-3,4,5-trihy droxytetrahydro-2H-pyran-2-carboxylic acid 21

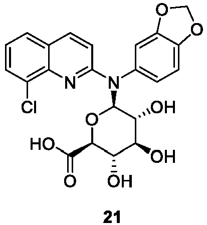

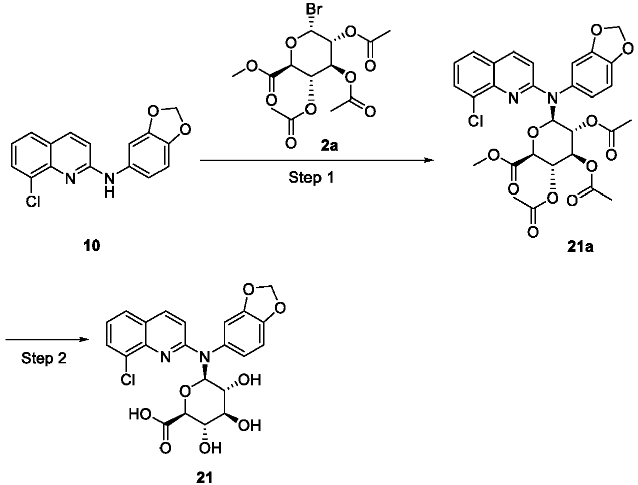

### Step 1

### (2R,3R,4S,5S,6S)-2-(Benzo[d][1,3]dioxol-5-yl(8-chloroquinolin-2-yl)amino)-6-(methox ycarbonyl)tetrahydro-2H-pyran-3,4,5-triyl triacetate 21a

Compound **10** (380 mg, 1.27 mmol) was dissolved in toluene (15 mL), and cadmium carbonate (131 mg, 0.76 mmol) was added. The resulting mixture was heated to 145 °C for water separation reaction for 12 h, and then compound **2a** (606 mg, 1.53 mmol) was added, followed by water separation reaction at 145 °C for 24 h. The reaction mixture was cooled to room temperature and concentrated under reduced pressure to remove the toluene, and the residue was purified by column chromatography with eluent system B to give the title compound **21a** (450 mg, yield: 58%).

MS m/z (ESI): 615.0 [M+1].

### Step 2

### (2S,3S,4S,5R,6R)-6-(Benzo[d][1,3]dioxol-5-yl(8-chloroquinolin-2-yl)amino)-3,4,5-trihy droxytetrahydro-2H-pyran-2-carboxylic acid 21

Lithium hydroxide monohydrate (645 mg, 15.35 mmol) was dissolved in water (4 mL), and a 30% hydrogen peroxide solution (1.83 mL) was added. The resulting solution was stirred at room temperature for 10 min, then added to a solution of compound **21a** (450 mg, 0.51 mmol) in tetrahydrofuran (16 mL), and stirred at room temperature for another 16 h. The reaction was quenched with a saturated sodium thiosulfate solution (20 mL), and then the mixture was adjusted to pH 4 with a 1 N hydrochloric acid solution, extracted with ethyl acetate (50 mL × 3), and washed with a saturated sodium chloride solution (100 mL). The organic phase was concentrated under reduced pressure, and then purified by preparative high performance liquid chromatography (Waters 2767-SQ Detecor2, elution system: a 10 mmol/L aqueous ammonium bicarbonate solution and acetonitrile, gradient of acetonitrile: 15%-95%, flow rate: 30 mL/min) to give the title compound **21** (85 mg, yield: 35%).

MS m/z (ESI): 475.1 [M+1].

¹H NMR (500 MHz, DMSO-*d*₆) δ 8.03 (d, 1H), 7.78 (d, 1H), 7.72 (d, 1H), 7.26 (t, 1H), 7.06 (d, 1H), 7.00-6.86 (m, 2H), 6.50 (d, 1H), 6.34 (d, 1H), 6.13 (d, 2H), 5.08-4.82 (m, 2H), 3.51 (d, 1H), 3.40-3.25 (m, 2H), 3.06 (t, 1H), 2.98-2.82 (m, 1H).

### Example 22

### (2S,3S,4S,5R,6R)-6-((8-Chloroquinolin-2-yl)(2,3-dihydrobenzo[b][1,4]dioxin-6-yl)amin o)-3,4,5-trihydroxytetrahydro-2H-pyran-2-carboxylic acid 22

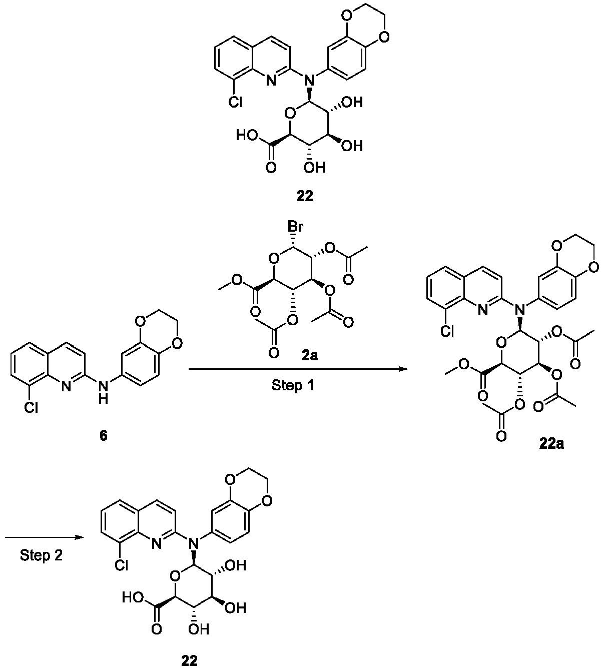

### Step 1

### (2R,3R,4S,5S,6S)-2-((8-Chloroquinolin-2-yl)(2,3-dihydrobenzo[b][1,4]dioxin-6-yl)amin o)-6-(methoxycarbonyl)tetrahydro-2H-pyran-3,4,5-triyl triacetate 22a

Compound **6** (500 mg, 1.60 mmol) was dissolved in toluene (30 mL), and cadmium carbonate (165 mg, 0.96 mmol) was added. The resulting mixture was heated to 140 °C for water separation reaction for 12 h, and then compound **2a** (1.90 g, 4.80 mmol) was added, followed by water separation reaction at 140 °C for 24 h. The reaction mixture was cooled to room temperature and concentrated under reduced pressure to remove the toluene, and the residue was purified by column chromatography with eluent system B to give the title compound **22a** (400 mg, yield: 40%).

MS m/z (ESI): 629.0 [M+1].

### Step 2

### (2S,3S,4S,5R,6R)-6-((8-Chloroquinolin-2-yl)(2,3-dihydrobenzo[b][1,4]dioxin-6-yl)amin o)-3,4,5-trihydroxytetrahydro-2H-pyran-2-carboxylic acid 22

Lithium hydroxide monohydrate (534 mg, 12.72 mmol) was dissolved in water (5 mL), and a 30% hydrogen peroxide solution (1.1 mL) was added. The resulting solution was stirred at room temperature for 10 min, then added to a solution of compound **22a** (400 mg, 0.64 mmol) in tetrahydrofuran (15 mL), and stirred at room temperature for another 2 h. The reaction was quenched with a saturated sodium thiosulfate solution (20 mL), and then the mixture was adjusted to pH 4 with a 1 N hydrochloric acid solution, extracted with ethyl acetate (50 mL × 3), and washed with a saturated sodium chloride solution (100 mL). The organic phase was concentrated under reduced pressure, and then purified by preparative high performance liquid chromatography (Waters 2767-SQ Detecor2, elution system: a 10 mmol/L aqueous ammonium bicarbonate solution and acetonitrile, gradient of acetonitrile: 20%-65%, flow rate: 30 mL/min) to give the title compound **22** (95 mg, yield: 36%).

MS m/z (ESI): 489.1 [M+1].

¹H NMR (500 MHz, DMSO-*d*₆) δ 8.01 (d, 1H), 7.77 (dd, 1H), 7.70 (dd, 1H), 7.25 (t, 1H), 6.99 (d, 1H), 6.94 (d, 1H), 6.89 (dd, 1H), 6.46 (d, 1H), 6.33 (d, 1H), 5.03-4.83 (m, 2H), 4.40-4.20 (m, 4H), 3.54 (d, 1H), 3.43-3.36 (m, 2H), 3.08 (t, 1H), 2.97-2.81 (m, 1H).

### Example 23

### 8-Chloro-N-(2,2-difluorobenzo[d][1,3]dioxol-5-yl)-5-methylquinolin-2-amine 23

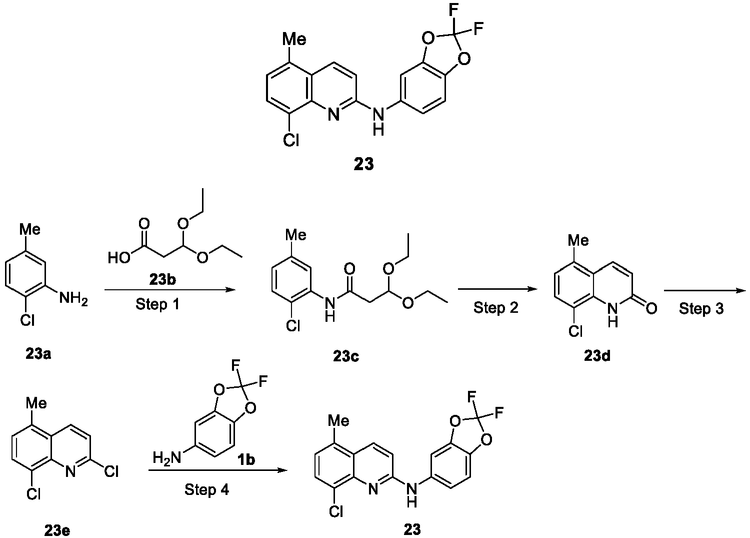

### Step 1

### N-(2-Chloro-5-methylphenyl)-3,3-diethoxypropanamide 23c

*N,N*-Dimethylformamide (5 mL), 2-chloro-5-methylaniline **23a** (0.5 g, 3.53 mmol, Bide Pharmatech Ltd.), and 3,3-diethoxypropanoic acid **23b** (0.63 g, 3.88 mmol, J&K Scientific) were added sequentially to a 100 mL three-necked flask, and 2-(7-azabenzotriazol)-*N*,*N*,*N*',*N*'-tetramethyluronium hexafluorophosphate (2.01 g, 5.29 mmol) and *N,N*-diisopropylethylamine (0.91 g, 7.06 mmol) were slowly added at 0 °C. The system was then warmed to room temperature and allowed to react for 24 h. 50 mL of a saturated sodium bicarbonate solution was added to the system to quench the reaction, and the reaction mixture was extracted with ethyl acetate (50 mL × 3). The organic phases were combined, washed with a saturated sodium chloride solution (50 mL × 3), dried over anhydrous sodium sulfate, and filtered. The filtrate was concentrated under reduced pressure to give the title compound **23c** (crude product, 1.0 g, yield: 99%), which was directly used in the next step.

### Step 2

### 8-Chloro-5-methylquinolin-2(1H)-one 23d

Dichloromethane (2 mL) and compound **23c** (1.00 g, 3.5 mmol) were added sequentially to a 100 mL single-necked flask, and concentrated sulfuric acid (3.35 mL, 62.98 mmol) was slowly added dropwise at 0 °C. The ice bath was then removed, and the mixture was allowed to react for 4 h and then concentrated under reduced pressure to remove the solvent. The residue was dropped into 50 mL of ice water, and the resulting mixture was extracted with ethyl acetate (50 mL × 5). The organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **23d** (0.26 g, yield: 38%).

MS m/z (ESI): 194.0 [M+1].

### Step 3

### 2,8-Dichloro-5-methylquinoline 23e

Compound **23d** (0.26 g, 1.34 mmol) and phosphorus oxychloride (2 mL) were added sequentially to a 50 mL single-necked flask, and the system was heated to 95 °C and allowed to react for 90 min. The reaction mixture was concentrated under reduced pressure to remove the phosphorus oxychloride, and ice water (50 mL) was added. The resulting mixture was extracted with ethyl acetate (50 mL × 3), and the organic phase was washed with a saturated sodium chloride solution (50 mL × 2), dried over anhydrous sodium sulfate, filtered, and concentrated to give the title compound **23e** (crude product, 0.28 g, yield: 99%), which was directly used in the next step.

MS m/z (ESI): 212.0 [M+1].

### Step 4

### 8-Chloro-N-(2,2-difluorobenzo[d][1,3]dioxol-5-yl)-5-methylquinolin-2-amine 23

Isopropanol (3 mL), compound **23e** (100 mg, 0.47 mmol), compound **1b** (97 mg, 0.56 mmol), and trifluoroacetic acid (161 mg, 1.41 mmol) were added sequentially to a 25 mL sealed tube, and then the resulting mixture was heated to 95 °C, allowed to react for 16 h, and then cooled to room temperature. 25 mL of water was added, and the mixture was extracted with ethyl acetate (25 mL × 3), washed with a saturated sodium chloride solution (25 mL × 2), dried over anhydrous sodium sulfate, and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 70%-90%, flow rate: 30 mL/min) to give the title compound **23** (80 mg, yield: 48%).

MS m/z (ESI): 349.1 [M+1].

¹H NMR (500 MHz, DMSO-*d₆*) δ 9.96 (s, 1H), 8.88 (d, 1H), 8.27 (d, 1H), 7.69 (d, 1H), 7.48 (dd, 1H), 7.37 (d, 1H), 7.19-7.12 (m, 2H), 2.57 (s, 3H).

### Example 24

### 8-Chloro-N-(2,2-difluorobenzo[d][1,3]dioxol-5-yl)-5-methoxyquinolin-2-amine 24

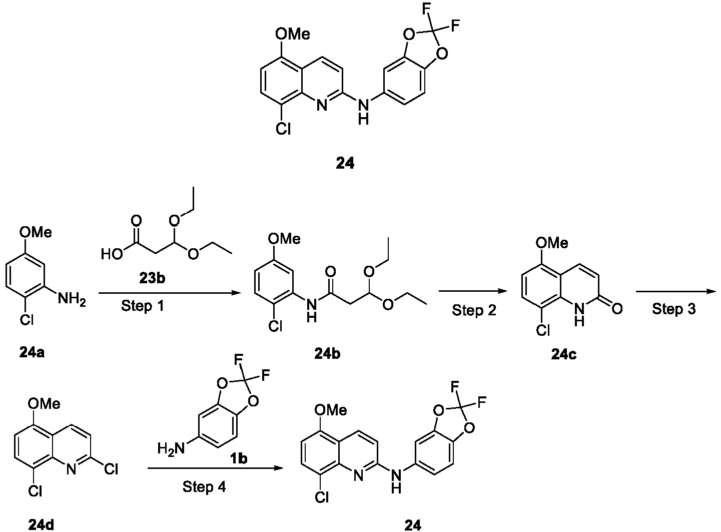

### Step 1

### N-(2-Chloro-5-methoxyphenyl)-3,3-diethoxypropanamide 24b

*N,N*-Dimethylformamide (5 mL), 2-chloro-5-methoxyaniline **24a** (0.5 g, 3.17 mmol, Bide Pharmatech Ltd.), and compound **23b** (0.63 g, 3.88 mmol) were added sequentially to a 100 mL three-necked flask, and 2-(7-azabenzotriazol)-*N*,*N*,*N'*,*N'*-tetramethyluronium hexafluorophosphate (2.01 g, 5.29 mmol) and *N,N*-diisopropylethylamine (0.91 g, 7.06 mmol) were slowly added at 0 °C. The system was then warmed to room temperature and allowed to react for 24 h. 50 mL of a saturated sodium bicarbonate solution was added to the system to quench the reaction, and the reaction mixture was extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure to give the title compound **24b** (crude product, 0.95 g, yield: 99%), which was directly used in the next step.

### Step 2

### 8-Chloro-5-methoxyquinolin-2(1H)-one 24c

Dichloromethane (2 mL) and compound **24b** (0.95 g, 3.14 mmol) were added sequentially to a 100 mL single-necked flask, and concentrated sulfuric acid (2.52 mL, 47.22 mmol) was slowly added dropwise at 0 °C. The ice bath was then removed, and the mixture was allowed to react for 4 h and then concentrated under reduced pressure to remove the solvent. The residue was dropped into 50 mL of ice water, and the resulting mixture was extracted with ethyl acetate (50 mL × 5). The organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **24c** (0.26 g, yield: 40%).

MS m/z (ESI): 210.0 [M+1].

### Step 3

### 2,8-Dichloro-5-methoxyquinoline 24d

Compound **24c** (365 mg, 1.26 mmol) and phosphorus oxychloride (2.5 mL) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was extracted with ethyl acetate (50 mL × 3), and the organic phase was washed with a saturated sodium chloride solution (50 mL × 2), dried over anhydrous sodium sulfate, filtered, and concentrated to give the title compound **24d** (crude product, 288 mg, yield: 99%), which was directly used in the next step.

MS m/z (ESI): 228.0 [M+1].

### Step 4

### 8-Chloro-N-(2,2-difluorobenzo[d][1,3]dioxol-5-yl)-5-methoxyquinolin-2-amine 24

Isopropanol (3 mL), compound **24d** (288 mg, 1.26 mmol), compound **1b** (218 mg, 1.26 mmol), and trifluoroacetic acid (431 mg, 3.78 mmol) were added sequentially to a 25 mL sealed tube, and then the resulting mixture was heated to 95 °C, allowed to react for 16 h, and then cooled to room temperature. 25 mL of water was added, and the mixture was extracted with ethyl acetate (25 mL × 3), washed with a saturated sodium chloride solution (25 mL × 2), dried over anhydrous sodium sulfate, and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 65%-85%, flow rate: 30 mL/min) to give the title compound **24** (230 mg, yield: 49%).

MS m/z (ESI): 365.1 [M+1].

¹H NMR (400 MHz, DMSO-*d₆*) δ 9.97 (s, 1H), 8.87 (s, 1H), 8.31 (d, 1H), 7.72 (d, 1H), 7.48 (d, 1H), 7.37 (d, 1H), 7.09 (d, 1H), 6.83 (d, 1H), 3.95 (s, 3H).

### Example 25

### 5,8-Dichloro-N-(2,2-difluorobenzo[d][1,3]dioxol-5-yl)quinolin-2-amine 25

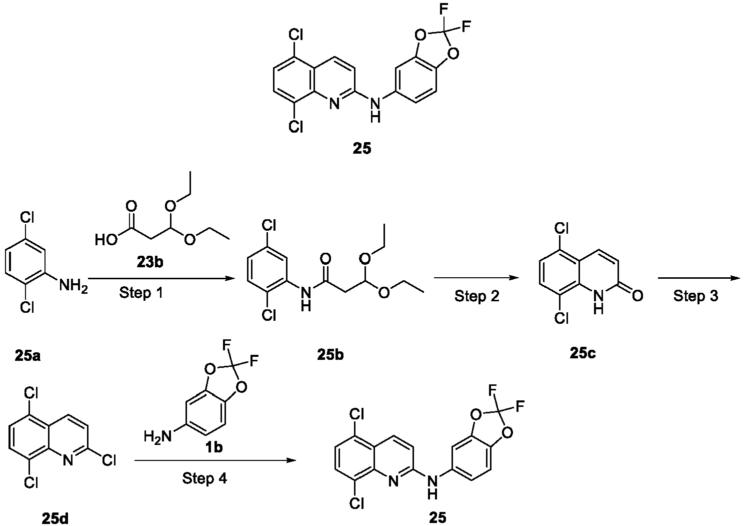

### Step 1

### N-(2,5-Dichlorophenyl)-3,3-diethoxypropanamide 25b

*N,N*-Dimethylformamide (5 mL), 2,5-dichloroaniline **25a** (0.5 g, 3.08 mmol, Bide Pharmatech Ltd.), and compound **23b** (0.50 g, 3.39 mmol) were added sequentially to a 100 mL three-necked flask, and 2-(7-azabenzotriazol)-*N,N,N,N*-tetramethyluronium hexafluorophosphate (2.01 g, 5.29 mmol) and N,N-diisopropylethylamine (0.91 g, 7.06 mmol) were slowly added at 0 °C. The system was then warmed to room temperature and allowed to react for 24 h. 50 mL of a saturated aqueous sodium bicarbonate solution was added to the system to quench the reaction, and the reaction mixture was extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure to give the title compound **25b** (crude product, 0.94 g, yield: 99%), which was directly used in the next step.

### Step 2

### 5,8-Dichloroquinolin-2(1H)-one 25c

Dichloromethane (2 mL) and compound **25b** (0.51 g, 1.67 mmol) were added sequentially to a 100 mL single-necked flask, and concentrated sulfuric acid (1.33 mL, 25.05 mmol) was slowly added dropwise at 0 °C. The ice bath was then removed, and the mixture was allowed to react for 4 h and then concentrated under reduced pressure to remove the solvent. The reaction mixture was dropped into 50 mL of ice water, and the resulting mixture was extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **25c** (0.21 g, yield: 56%).

MS m/z (ESI): 214.1 [M+1].

### Step 3

### 2,5,8-Trichloroquinoline 25d

Compound **25c** (213 mg, 0.99 mmol) and phosphorus oxychloride (2 mL) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was extracted with ethyl acetate (50 mL × 3), and the organic phase was washed with a saturated sodium chloride solution (50 mL × 2), dried over anhydrous sodium sulfate, filtered, and concentrated to give the title compound **25d** (crude product, 230 mg, yield: 99%), which was directly used in the next step.

MS m/z (ESI): 231.9 [M+1].

### Step 4

### 5,8-Dichloro-N-(2,2-difluorobenzo[d][1,3]dioxol-5-yl)quinolin-2-amine 25

Isopropanol (3 mL), compound **25d** (234 mg, 1.01 mmol), compound **1b** (174 mg, 1.01 mmol), and trifluoroacetic acid (344 mg, 3.02 mmol) were added sequentially to a 25 mL sealed tube, and then the resulting mixture was heated to 95 °C, allowed to react for 16 h, and then cooled to room temperature. 25 mL of water was added, and the resulting mixture was extracted with ethyl acetate (25 mL × 3), washed with a saturated sodium chloride solution (25 mL × 2), dried over anhydrous sodium sulfate, and filtered. The filtrate was concentrated under reduced pressure to remove the solvent, and the residue was purified by preparative high performance liquid chromatography (Waters 2767-SQ Detecor2, elution system: a 10 mmol/L aqueous ammonium bicarbonate solution and acetonitrile, gradient of acetonitrile: 80%-95%, flow rate: 30 mL/min) to give the title compound **25** (230 mg, yield: 59%).

MS m/z (ESI): 369.0 [M+1].

¹H NMR (400 MHz, DMSO-*d₆*) δ 10.18 (s, 1H), 8.79 (d, 1H), 8.35 (d, 1H), 7.81 (d, 1H), 7.51 (dd, 1H), 7.46 (d, 1H), 7.40 (d, 1H), 7.27 (d, 1H).

### Example 26

### 8-Chloro-2-((2,2-difluorobenzo[d][1,3]dioxol-5-yl)amino)quinoline-6-carbonitrile 26

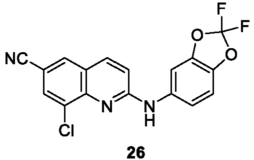

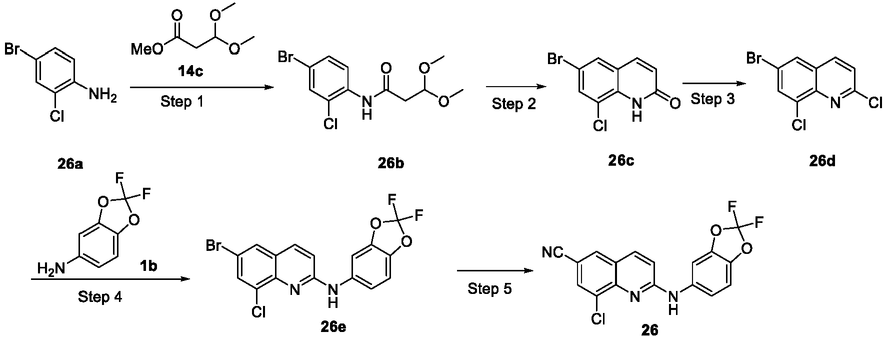

### Step 1

### N-(4-Bromo-2-chlorophenyl)-3,3-dimethoxypropanamide 26b

Tetrahydrofuran (25 mL), 4-bromo-2-chloroaniline **26a** (3.0 g, 14.53 mmol, J&K Scientific), and compound **14c** (2.36 g, 15.98 mmol) were added sequentially to a 100 mL three-necked flask, and sodium bis(trimethylsilyl)amide (a 2 M solution in tetrahydrofuran, 10.89 mL, 21.79 mmol) was slowly added dropwise at 0 °C. The system was then warmed to room temperature and allowed to react for 24 h. 50 mL of a saturated ammonium chloride solution was added to the system to quench the reaction, and the reaction mixture was concentrated under reduced pressure to remove the solvent and then extracted with ethyl acetate (50 mL × 3). The organic phases were combined, washed with a saturated sodium chloride solution (50 mL × 2), dried over anhydrous sodium sulfate, and filtered. The filtrate was concentrated under reduced pressure to give the title compound **26b** (crude product, 4.60 g, yield: 98%), which was directly used in the next step.

### Step 2

### 6-Bromo-8-chloroquinolin-2(1H)-one 26c

Dichloromethane (5 mL) and compound **26b** (4.60 g, 14.25 mmol) were added sequentially to a 100 mL single-necked flask, and concentrated sulfuric acid (11.40 mL, 213.89 mmol) was slowly added dropwise at 0 °C. The ice bath was then removed, and the mixture was heated to 90 °C, allowed to react for 2 h, and then concentrated under reduced pressure to remove the solvent. The residue was dropped into 50 mL of ice water, and the resulting mixture was extracted with ethyl acetate (50 mL × 5). The organic phases were combined, washed with a saturated sodium chloride solution (50 mL × 2), dried over anhydrous sodium sulfate, and filtered. The filtrate was concentrated under reduced pressure, and the resulting residue was purified by silica gel column chromatography with eluent system B to give the title compound **26c** (0.46 g, yield: 12%).

MS m/z (ESI): 257.9 [M+1].

### Step 3

### 6-Bromo-2,8-dichloroquinoline 26d

Compound **26c** (460 mg, 1.78 mmol) and phosphorus oxychloride (2 mL) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was extracted with ethyl acetate (50 mL × 3), and the organic phase was washed with a saturated sodium chloride solution (50 mL × 2), dried over anhydrous sodium sulfate, filtered, and concentrated to give the title compound **26d** (crude product, 440 mg, yield: 89%), which was directly used in the next step.

### Step 4

### 6-Bromo-8-chloro-N-(2,2-difluorobenzo[d][1,3]dioxol-5-yl)quinolin-2-amine 26e

Isopropanol (3 mL), compound **26d** (280 mg, 1.01 mmol), compound **1b** (175 mg, 1.01 mmol), and trifluoroacetic acid (345 mg, 3.03 mmol) were added sequentially to a 25 mL sealed tube, and then the resulting mixture was heated to 95 °C, allowed to react for 16 h, and then cooled to room temperature. 25 mL of a saturated sodium bicarbonate solution was added, and the mixture was extracted with ethyl acetate (25 mL × 3). The organic phase was washed with a saturated sodium chloride solution (25 mL × 2), dried over anhydrous sodium sulfate, and concentrated under reduced pressure to remove the solvent. The resulting residue was purified by silica gel column chromatography with eluent system B to give the title compound **26e** (260 mg, yield: 62%).

MS m/z (ESI): 412.9 [M+1].

### Step 5

### 8-Chloro-2-((2,2-difluorobenzo[d][1,3]dioxol-5-yl)amino)quinoline-6-carbonitrile 26

*N,N*-Dimethylacetamide (3 mL), compound **26e** (150 mg, 0.36 mmol), zinc cyanide (126 mg, 1.01 mmol), zinc powder (3.50 mg, 0.05 mmol), tris(dibenzylideneacetone)dipalladium(0) (36 mg, 0.04 mmol), and 1,1'-bis(diphenylphosphino)ferrocene (46 mg, 0.08 mmol) were added sequentially to a 25 mL three-necked flask, and the resulting mixture was heated to 135 °C, allowed to react for 3 h, and then cooled to room temperature. 25 mL of water was added, and the mixture was extracted with ethyl acetate (25 mL × 3), washed with a saturated sodium chloride solution (25 mL × 2), dried over anhydrous sodium sulfate, and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 80%-95%, flow rate: 30 mL/min) to give the title compound **26** (55 mg, yield: 63%).

MS m/z (ESI): 360.1 [M+1].

¹H NMR (400 MHz, DMSO-*d₆*) δ 10.33 (s, 1H), 8.74 (s, 1H), 8.36 (s, 1H), 8.22-8.12 (m, 2H), 7.53 (d, 1H), 7.41 (d, 1H), 7.22 (d, 1H).

### Example 27

### 8-Chloro-6-cyclopropyl-N-(2,2-difluorobenzo[d][1,3]dioxol-5-yl)quinolin-2-amine 27

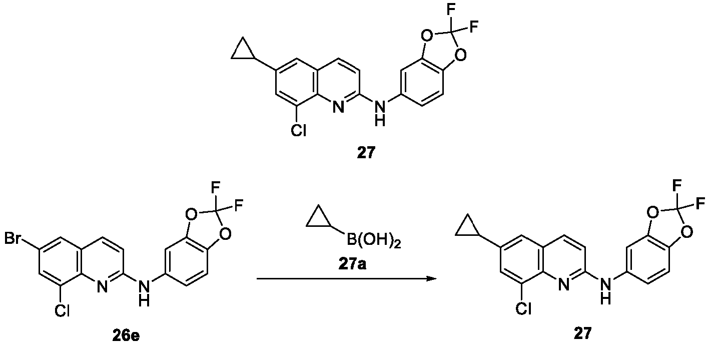

Compound **26e** (165 mg, 0.40 mmol) was dissolved in 7.5 mL of 1,4-dioxane and water (V/V = 4:1), and cyclopropylboronic acid **27a** (41 mg, 0.48 mmol, Accela ChemBio (Shanghai) Inc.), sodium carbonate (127 mg, 1.20 mmol), and [1,1'-bis(di-tert-butylphosphine)ferrocene palladium(II) dichloride (39 mg, 0.06 mmol, Bide Pharmatech Ltd.) were added. The resulting mixture was purged with nitrogen three times, and then heated to 100 °C and allowed to react for 3 h. The reaction mixture was cooled to room temperature, and a saturated sodium bicarbonate solution (25 mL) was added. The resulting mixture was extracted with ethyl acetate (50 mL × 2), and the organic phases were combined, dried over anhydrous sodium sulfate, and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 75%-95%, flow rate: 30 mL/min) to give the title compound **27** (20 mg, yield: 13%).

MS m/z (ESI): 375.1 [M+1].

¹H NMR (400 MHz, DMSO-*d₆*) δ 9.90 (s, 1H), 8.85 (d, 1H), 8.05 (d, 1H), 7.55 (d, 1H), 7.48-7.44 (m, 2H), 7.36 (d, 1H), 7.09 (d, 1H), 2.08-2.03 (m, 1H), 1.06-0.97 (m, 2H), 0.81-0.75 (m, 2H).

### Example 28

### 8-Chloro-N-(2,2-difluorobenzo[d][1,3]dioxol-5-yl)-6-(tetrahydro-2H-pyran-4-yl)quinoli n-2-amine 28

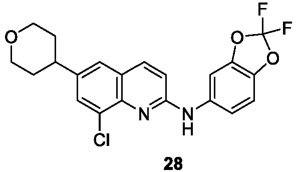

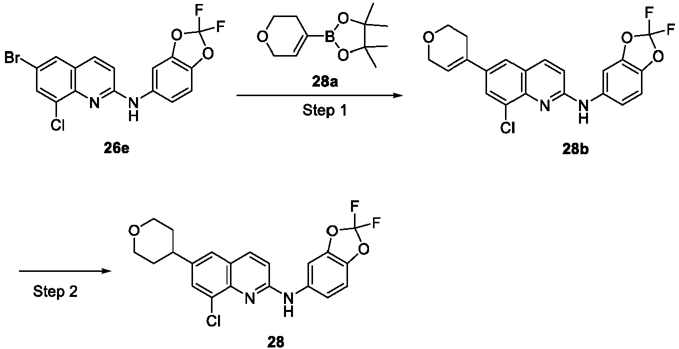

### Step 1

### 8-Chloro-N-(2,2-difluorobenzo[d][1,3]dioxol-5-yl)-6-(3,6-dihydro-2H-pyran-4-yl)quino lin-2-amine 28b

Compound **26e** (181 mg, 0.44 mmol) was dissolved in 7.5 mL of 1,4-dioxane and water (V/V = 4:1) and 3,6-dihydro-2*H*-pyran-4-boronic acid pinacol ester **28a** (92 mg, 0.44 mmol, Accela ChemBio (Shanghai) Inc.), sodium carbonate (140 mg, 1.31 mmol), and [1,1'-bis(di-*tert*-butylphosphine)ferrocene palladium(II) dichloride (29 mg, 0.044 mmol) were added. The resulting mixture was purged with nitrogen three times, and then heated to 100 °C and allowed to react for 3 h. The reaction mixture was cooled to room temperature, and a saturated sodium bicarbonate solution (25 mL) was added. The resulting mixture was extracted with ethyl acetate (50 mL × 2), and the organic phases were combined, dried over anhydrous sodium sulfate, and concentrated under reduced pressure to remove the solvent. The resulting residue was purified by silica gel column chromatography with eluent system B to give the title compound **28b** (140 mg, yield: 77%).

MS m/z (ESI): 417.0 [M+1].

### Step 2

### 8-Chloro-N-(2,2-difluorobenzo[d][1,3]dioxol-5-yl)-6-(tetrahydro-2H-pyran-4-yl)quinoli n-2-amine 28

Compound **28b** (140 mg, 0.34 mmol) was dissolved in 10 mL of ethyl acetate, and platinum on activated carbon (70 mg, 5% Wt., 50%-70% water content, Accela ChemBio (Shanghai) Inc.) was added. The resulting mixture was purged with hydrogen three times and allowed to react under a hydrogen atmosphere for 24 h. The reaction mixture was filtered through celite and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 70%-95%, flow rate: 30 mL/min) to give the title compound **28** (70 mg, yield: 50%).

MS m/z (ESI): 419.0 [M+1].

¹H NMR (400 MHz, DMSO-*d₆*) δ 9.92 (s, 1H), 8.86 (d, 1H), 8.11 (d, 1H), 7.74 (d, 1H), 7.61 (d, 1H), 7.47 (dd, 1H), 7.37 (d, 1H), 7.10 (d, 1H), 3.99 (dd, 2H), 3.46 (td, 2H), 2.93-2.85 (m, 1H), 1.83-1.68 (m, 4H).

### Example 29

### 8-Chloro-7-cyclopropyl-N-(2,2-difluorobenzo[d][1,3]dioxol-5-yl)quinolin-2-amine 29

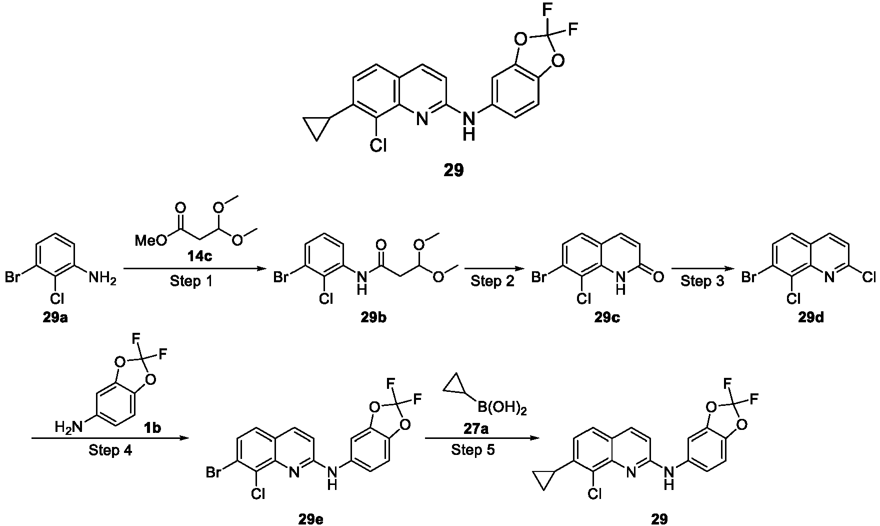

### Step 1

### N-(3-Bromo-2-chlorophenyl)-3,3-dimethoxypropanamide 29b

3-Bromo-2-chloro-aniline **29a** (2.0 g, 9.72 mmol, Bide Pharmatech Ltd.) was dissolved in 15 mL of tetrahydrofuran, and compound **14c** (1.58 g, 10.68 mmol) was added. The resulting mixture was cooled to 0 °C, and sodium bis(trimethylsilyl)amide (a 2 M solution in tetrahydrofuran, 5.4 mL, 10.7 mmol) was added dropwise. The system was then warmed to room temperature and allowed to react for 16 h. A saturated ammonium chloride solution (100 mL) was added to the reaction mixture, and the resulting mixture was extracted with ethyl acetate (100 mL × 3) and washed with a saturated sodium chloride solution (100 mL × 2). The organic phases were combined, dried over anhydrous sodium sulfate, and filtered. The filtrate was concentrated under reduced pressure and dried *in vacuo* to give the title compound **29b** (crude product, 3.1 g, yield: 99%), which was directly used in the next step without purification.

MS m/z (ESI): 321.9 [M+1].

### Step 2

### 7-Bromo-8-chloroquinolin-2(1H)-one 29c

Compound **29b** (3.1 g, 9.61 mmol) was dissolved in 3 mL of dichloromethane. The resulting solution was cooled to 0 °C, and concentrated sulfuric acid (7.7 mL, 144 mmol) was added. The ice bath was then removed, and the mixture was heated to 90 °C and allowed to react for 2 h. The reaction mixture was cooled to room temperature and then concentrated under reduced pressure to remove the solvent. The residue was dropped into 50 mL of ice water, and the resulting mixture was extracted with ethyl acetate (50 mL × 5). The organic phases were combined, washed with a saturated sodium chloride solution (50 mL × 2), dried over anhydrous sodium sulfate, filtered, and concentrated under reduced pressure to remove the solvent. The resulting residue was purified by silica gel column chromatography with eluent system B to give the title compound **29c** (0.56 g, yield: 23%).

MS m/z (ESI): 257.9 [M+1].

### Step 3

### 7-Bromo-2,8-dichloroquinoline 29d

Compound **29c** (560 mg, 2.17 mmol) and phosphorus oxychloride (3 mL) were added sequentially to a 50 mL single-necked flask, and the system was heated to 95 °C and allowed to react for 90 min. The reaction mixture was concentrated under reduced pressure to remove the phosphorus oxychloride, and 50 mL of ice water was added. The resulting mixture was extracted with ethyl acetate (50 mL × 3), and the organic phase was washed with a saturated sodium chloride solution (50 mL × 2), dried over anhydrous sodium sulfate, filtered, and concentrated to give the title compound **29d** (crude product, 599 mg, yield: 99%), which was directly used in the next step without purification.

### Step 4

### 7-Bromo-8-chloro-N-(2,2-difluorobenzo[d][1,3]dioxol-5-yl)quinolin-2-amine 29e

Isopropanol (3 mL), compound **29d** (600 mg, 2.17 mmol), compound **1b** (319 mg, 1.84 mmol), and trifluoroacetic acid (741 mg, 6.50 mmol) were added sequentially to a 25 mL sealed tube, and then the resulting mixture was heated to 95 °C, allowed to react for 16 h, and then cooled to room temperature. 25 mL of a saturated sodium bicarbonate solution was added, and the mixture was extracted with ethyl acetate (25 mL × 3). The organic phase was washed with a saturated sodium chloride solution (25 mL × 2), dried over anhydrous sodium sulfate, and filtered. The filtrate was concentrated under reduced pressure to remove the solvent, and the resulting residue was purified by silica gel column chromatography with eluent system B to give the title compound **29e** (554 mg, yield: 62%).

MS m/z (ESI): 412.9 [M+1].

### Step 5

### 8-Chloro-7-cyclopropyl-N-(2,2-difluorobenzo[d][1,3]dioxol-5-yl)quinolin-2-amine 29

Compound **29e** (140 mg, 0.34 mmol) was dissolved in 7.5 mL of 1,4-dioxane and water (V/V = 4:1), and compound **27a** (37.8 mg, 0.44 mmol), sodium carbonate (107.6 mg, 1.02 mmol), and [1,1'-bis(di-*tert*-butylphosphine)ferrocene palladium(II) dichloride (33.0 mg, 0.051 mmol) were added. The resulting mixture was purged with nitrogen three times, and then heated to 100 °C and allowed to react for 3 h. The reaction mixture was cooled to room temperature, and a saturated sodium bicarbonate solution (25 mL) was added. The resulting mixture was extracted with ethyl acetate (50 mL × 2), and the organic phases were combined, washed with a saturated sodium chloride solution (50 mL × 2), dried over anhydrous sodium sulfate, and concentrated under reduced pressure to remove the solvent. The residue was purified by preparative high performance liquid chromatography (Waters 2767-SQ Detecor2, elution system: a 10 mmol/L aqueous ammonium bicarbonate solution and acetonitrile, gradient of acetonitrile: 55%-95%, flow rate: 30 mL/min) to give the title compound **29** (30 mg, yield: 23%).

MS m/z (ESI): 375.4 [M+1].

¹H NMR (400 MHz, DMSO-*d₆*) δ 9.92 (s, 1H), 8.92 (d, 1H), 8.08 (d, 1H), 7.64 (d, 1H), 7.48 (dd, 1H), 7.37 (d, 1H), 7.05 (d, 1H), 6.90 (d, 1H), 2.50-2.42 (m, 1H), 1.15-1.10 (m, 2H), 0.86-0.81 (m, 2H).

### Example 30

### 8-Chloro-N-(2,2-difluorobenzo[d][1,3]dioxol-5-yl)-7-(tetrahydro-2H-pyran-4-yl)quinoli n-2-amine 30

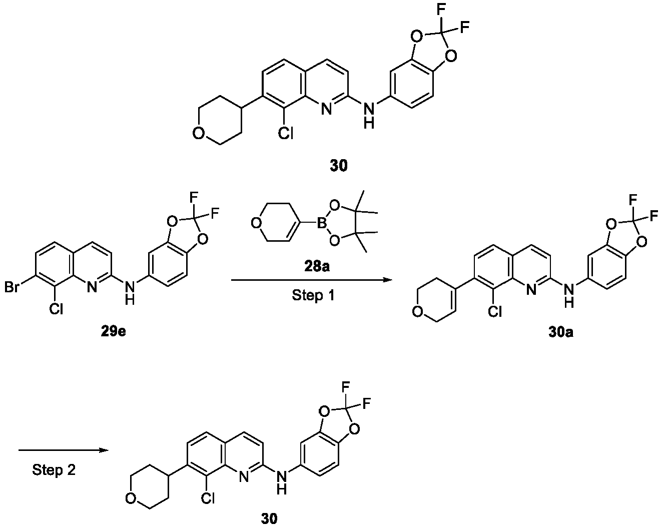

### Step 1

### 8-Chloro-N-(2,2-difluorobenzo[d][1,3]dioxol-5-yl)-7-(3,6-dihydro-2H-pyran-4-yl)quino lin-2-amine 30a

Compound **29e** (170 mg, 0.41 mmol) was dissolved in 7.5 mL of 1,4-dioxane and water (V/V = 4:1), and compound **28a** (104 mg, 0.49 mmol), sodium carbonate (131 mg, 1.23 mmol), and [1,1'-bis(di-tert-butylphosphine)ferrocene palladium(II) dichloride (16 mg, 0.025 mmol) were added. The resulting mixture was purged with nitrogen three times, and then heated to 100 °C and allowed to react for 3 h. The reaction mixture was cooled to room temperature, and a saturated sodium bicarbonate solution (25 mL) was added. The resulting mixture was extracted with ethyl acetate (50 mL × 2), and the organic phases were combined, washed with a saturated sodium chloride solution (50 mL × 2), dried over anhydrous sodium sulfate, and filtered. The filtrate was concentrated under reduced pressure to remove the solvent, and the resulting residue was purified by silica gel column chromatography with eluent system B to give the title compound **30a** (70 mg, yield: 41%).

MS m/z (ESI): 417.0 [M+1].

### Step 2

### 8-Chloro-N-(2,2-difluorobenzo[d][1,3]dioxol-5-yl)-7-(tetrahydro-2H-pyran-4-yl)quinoli n-2-amine 30

Compound **30a** (70 mg, 0.17 mmol) was dissolved in 5 mL of ethyl acetate, and platinum on activated carbon (35 mg, 5% Wt., 50%-70% water content, Accela ChemBio (Shanghai) Inc.) was added. The resulting mixture was purged with hydrogen three times and allowed to react under a hydrogen atmosphere for 24 h. The reaction mixture was filtered through celite and concentrated under reduced pressure to remove the solvent. The residue was purified by preparative high performance liquid chromatography (Waters 2767-SQ Detecor2, elution system: a 10 mmol/L aqueous ammonium bicarbonate solution and methanol, gradient of methanol: 75%-95%, flow rate: 30 mL/min) to give the title compound **30** (10 mg, yield: 14%).

MS m/z (ESI): 419.1 [M+1].

¹H NMR (400 MHz, DMSO-*d₆*) δ 9.94 (s, 1H), 8.90 (d, 1H), 8.12 (d, 1H), 7.74 (d, 1H), 7.48 (dd, 1H), 7.40-7.35 (m, 2H), 7.08 (d, 1H), 4.01 (dd, 2H), 3.54 (td, 2H), 2.04-1.95 (m, 1H), 1.87-1.69 (m, 4H).

### Example 31

### 5-((8-Chloroquinolin-2-yl)amino)-3-methylbenzo[d]oxazol-2(3H)-one 31

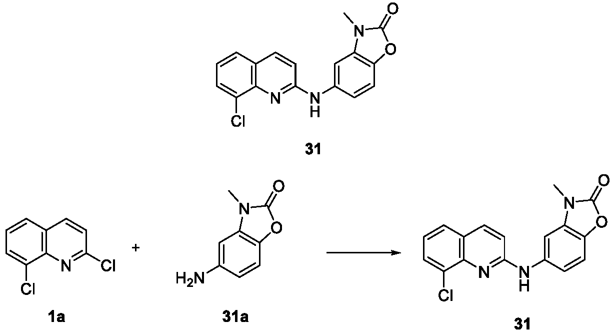

5-Amino-3-methyl-1,3-benzoxazol-2(3H)-one **31a** (123 mg, 0.75 mmol, Bide Pharmatech Ltd.), compound **1a** (135 mg, 0.68 mmol), cesium carbonate (333 mg, 1.02 mmol), 4,5-bis(diphenylphosphino)-9,9-dimethylxanthene (78.9 mg, 0.14 mmol), and tris(dibenzylideneacetone)dipalladium(0) (63 mg, 0.068 mmol) were dissolved in 5 mL of 1,4-dioxane, and the resulting solution was heated to 100 °C, allowed to react for 12 h, and then cooled to room temperature. 30 mL of water was added, and the resulting mixture was extracted with ethyl acetate (50 mL × 3), washed with a saturated sodium chloride solution (50 mL × 2), dried over anhydrous sodium sulfate, and filtered. The filtrate was concentrated under reduced pressure, and the residue was purified by preparative high performance liquid chromatography (Waters 2767-SQ Detecor2, elution system: a 10 mmol/L aqueous ammonium bicarbonate solution and acetonitrile, gradient of acetonitrile: 45%-95%, flow rate: 30 mL/min) to give the title compound 31 (74 mg, yield: 33%).

MS m/z (ESI): 324.1 [M-1].

¹H NMR (500 MHz, CDCl₃) δ 9.89 (s, 1H), 9.00 (d, 1H), 8.14 (d, 1H), 7.80 (d, 1H), 7.75 (d, 1H),7.33-7.22 (m, 3H), 7.14 (d, 1H), 3.39 (s, 3H).

### Biological Evaluation

Description of abbreviations used in the following:
p.o.: oral administration
bid: twice daily
qd: once daily
MC: sodium carboxymethylcellulose

### Test Example 1. Prophylactic and Therapeutic Effects of Compound Disclosed Herein on Ulcerative Colitis (UC) in Mice

### 1. Abstract

In the experiment, female C57BL/6 mice from Vital River were selected to establish an ulcerative colitis (UC) model induced by dextran sulfate sodium salt (DSS), and the prophylactic and therapeutic effects of a positive compound ABX-464 (see compound 90 in WO2015001518A1) and compound **1** disclosed herein on the DSS-induced ulcerative colitis were evaluated.

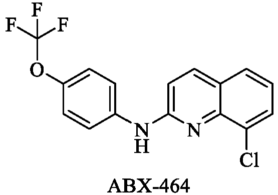

### 2. Experimental method and materials

### 2.1. Experimental animals and housing conditions

Female C57BL/6 mice for experiments, purchased from Vital River Laboratory Animal Technology Co., Ltd. (production license number: SCXK (Zhejiang) 2019-0001, animal certification number: 20210401Abzz0619000795), weighing 20-22g at the time of purchase, were bred at 5 mice/cage in an independent SPF space (in 12/12-hour light/dark cycle, at a constant temperature of 23 ± 1 °C and a humidity of 50%-60%) and given *ad libitum* access to food and water. After purchase, the animals were acclimatized for at least 1 week, and then the experiment began.

### 2.2. Experimental reagents and instruments

Dextran sulfate sodium salt (DSS): MP Biomedicals, catalog No. 160110, lot No. S5036. The DSS preparation was prepared with sterile water and then filtered, which could not be autoclaved and was replaced every two days.

Ethanol: Baxter Healthcare (Shanghai) Co., Ltd., lot No. S2001050.

Olive oil: Sinopharm Chemical Reagent Co., Ltd., catalog No. 30189828, lot No. 20180104.

Methylcellulose M450: Sinopharm Chemical Reagent Co., Ltd., catalog No. 69016460, lot No. 20170308.

Microplate reader: manufacturer BMGlabtech, model PHERAstar Fs.

Benchtop low-speed centrifuge: manufacturer Eppendorf, model 5417R.

Electronic balance: Mettler-Toledo Instruments Co., Ltd., model AL204.

### 2.3. Experimental design and procedures

### 2.3.1. Grouping of animals

The mice were acclimatized and then grouped as follows:

| Group | Modeling | Number of mice | Dose |
|---|---|---|---|
| Normal control group (Naive) | Water for 10 days | 16 | Solvent (bid) |
| DSS model group | 2.5% DSS for 7 days + water for 3 days | 16 | Solvent (bid) |
| Positive drug group 1 | 2.5% DSS for 7 days + water for 3 days | 10 | ABX-464 50 mg/kg (qd) |
| Positive drug group 2 | 2.5% DSS for 7 days + water for 3 days | 10 | ABX-464 50 mg/kg (bid) |
| Test drug group | 2.5% DSS for 7 days + water for 3 days | 10 | Compound **1** disclosed herein 50 mg/kg (bid) |

| | | | |
|---|---|---|---|
| Solvent: 0.5% MC suspension | | | |

### 2.3.2. Preparation of drugs

Preparation of DSS: 25 g of DSS + 1 L of ultrapure water, subjected to sterile filtration, stored at 4 °C.

Preparation of 50 mg/kg ABX-464: 100 mg of ABX-464 + 20 mL of 0.5% MC, triturated, stored at 4 °C. The preparation was carried out twice.

Preparation of 50 mg/kg compound **1** disclosed herein: 100 mg of compound **1** disclosed herein + 20 mL of 0.5% MC, triturated, stored at 4 °C.

### 2.3.3. Experimental procedures

The mice were randomly divided by body weight into the following 5 groups: normal control group (Naïve group), model group (DSS group), ABX-464 (50 mg/kg, p.o., qd) group, ABX-464 (50 mg/kg, p.o., bid) group, and compound **1** disclosed herein (50 mg/kg, p.o., bid) group. After acclimatization, the mice were subjected to 2.5% DSS feeding from day 0. After 7 days of DSS feeding, the mice were fed normal water until day 10. The corresponding solvents and drugs were administered intragastrically for 10 consecutive days from day 0 to day 10, and the mice were observed daily for changes in body weight from day 0 to day 10. On day 10, the mice were weighed, and the colon length was measured.

### 2.4. Data expression and statistical processing

The experimental data are expressed as mean ± standard error (SEM). Statistical comparison was performed using Excel software t-test. The data of the model group and the data of the normal control group were analyzed and compared. #P < 0.05 indicates a significant difference between the model group and the normal control group, ##P < 0.01 indicates a highly significant difference between the model group and the normal control group, and ###P < 0.001 indicates a very highly significant difference between the model group and the normal control group. *P < 0.05 indicates a significant difference between the administration group and the model group, **P < 0.01 indicates a highly significant difference between the administration group and the model group, and ***P < 0.001 indicates a very highly significant difference between the administration group and the model group.

### 3. Results

### 3.1. Effect of compound 1 disclosed herein on the body weight of DSS-induced UC mice

The results of the body weight experiment (FIG. 1) show that compared with the normal control group, the mice in the DSS model group showed a significant decrease in body weight from day 4, and the body weight decreasing rates gradually increased, reaching 30.0% on day 10 (P < 0.001); compared with the DSS model group, all of the administration groups showed significant weight regain from day 7, and on day 10, the groups that received 50 mg/kg ABX-464 (qd), ABX-464 (bid) and compound **1** disclosed herein (bid) showed body weight decreasing rates of 14.1% (P < 0.001), 10.3% (P < 0.001) and 0.4% (P < 0.001), respectively. The body weight recovery rates at the end of the experiment were in the following order from strongest to weakest: 50 mg/kg compound **1** disclosed herein (bid) > 50 mg/kg ABX-464 (bid) > 50 mg/kg ABX-464 (qd).

### 3.2. Effect of compound 1 disclosed herein on the colon length of DSS-induced UC mice

The results of the colon length (FIG. 2) show that compared with the normal control group, the colon length of the DSS model group was significantly shorter (P < 0.001) and was only 75.4% of that of the normal control group; compared with the DSS model group, all of the administration groups showed a significant increase in colon length, wherein the colon lengths of the groups that received 50 mg/kg ABX-464 (qd), ABX-464 (bid) and compound **1** disclosed herein (bid) were 85.5% (P < 0.05), 89.3% (P < 0.05) and 91.9% (P < 0.01) of that the normal control group, respectively. The colon lengths were in the following order from longest to shortest: 50 mg/kg compound **1** disclosed herein (bid) > 50 mg/kg ABX-464 (bid) > 50 mg/kg ABX-464 (qd).

### 4. Conclusion

The DSS model was used as a UC simulation animal model of IBD disease, and the molecular weight (36000-50000), lot number and preservation configuration of DSS, the feeding environment and species of mice, and the like all affected the effect of the model. This modeling was relatively successful as the mice showed significant changes in both body weight and colon length. The results show that compound **1** disclosed herein exhibited better efficacy in terms of body weight and colon length than the positive drug ABX-464. Therefore, 50 mg/kg ABX-464 and compound **1** disclosed herein had certain prophylactic and therapeutic effects on the DSS-induced UC, and compound **1** disclosed herein had the strongest efficacy which was higher than that of the ABX-464 with the same dosage.

### Test Example 2. Up-Regulation of miR-124 by Compound Disclosed Herein

### 1. Abstract

This assay was used to evaluate the up-regulation of miR-124 by the compound disclosed herein.

### 2. Experimental materials and instruments

1. Dynabead Human T-Activator CD3/CD28 for T Cell Expansion and Activation (Gibco, 11131D)
2. Pan T Cell Isolation Kit, human (Miltenyi, 130-096-535)
3. Human IL-2 (Peprotech, 200-02-100)
4. microRNA extraction kit (Qiagen, 217004)
5. miScript II RT Kit (Qiagen, 218161)
6. miScript SYBR Green PCR Kit (Qiagen, 218073)
7. Phosphate-buffered saline (PBS), pH 7.4 (Shanghai BasalMedia Technologies Co., Ltd., B320)
8. Bovine serum albumin (BSA) (Beyotime, ST023)
9. EDTA (0.5 M), pH 8.0 (Invitrogen, AM9260G)
10. LS Columns (Miltenyi, 130-042-401)
11. 24-well cell culture plate (Corning, 3524)
12. 96-well plate (Corning, 3788)
13. Cell incubator (Thermo, Steri cycle i160)
14. Real-time fluorescence quantitative PCR instrument (Applied biosystem, QuantStudio6 Flex)
15. PCR instrument (Applied biosystem, ProFlex)
16. 96-well clear PCR plate, 0.2 mL (Applied biosystems, N8010560)
17. RPMI1640 medium (Gibco, 11875119)
18. Fetal bovine serum (FBS) (Gibco, 10099-141)
19. Magnetic rack (Invitrogen, DynaMag^{™}-2)
20. Six-well cell culture plate (Thermo, 150239)
21. Spectrophotometer (IMPLEN, NP80)
22. QuadroMACS Separator (Miltenyi, 130-090-976)
23. miR124-3P-F primer (customized by Genewiz)
24. hsa-U6 detection primer (Tiangen, CD201-0145)

### 3. Experimental procedures

The effect of the compound on miR-124 expression levels was detected in CD3/CD28 antibody-activated T cells. After the activated T cells were treated with the compound, the total cellular RNA was extracted, the cDNA obtained from reverse transcription was used as a template, and quantification was performed by an SYBR green fluorescence quantitative PCR method using a specific miR-124 primer.

Isolation of T cells: Purchased human peripheral blood mononuclear cells (PBMCs) were counted, centrifuged, and washed once with a separation buffer (PBS, pH 7.4, containing 0.5% BSA and 2 mM EDTA), and the supernatant was discarded. The components were added in an amount of 40 µL of a buffer and 10 µL of pan T Cell Biotin-Antibody Cocktail per 1 × 10⁷ cells, and the pellet was resuspended. The resulting suspension was mixed and incubated in a refrigerator at 4 °C for 5 min. After the incubation was completed, the components were added in an amount of 30 µL of a buffer and 20 µL of Pan T Cell MicroBeads Cocktail per 1 × 10⁷ cells, and the resulting suspension was mixed and incubated in a refrigerator at 4 °C for 10 min. The separation column (LS column) was rinsed with 3 mL of a cell separation buffer in advance, loaded with the above cell suspension, and then repeatedly washed 3 times with 1 mL of the cell separation buffer. The effluent cell fluid was collected in a 15 mL centrifuge tube, thus giving the enriched T cells. The cells were counted and added at a density of 1 × 10⁶ cells/mL to an RPMI1640 medium (complete medium) containing 10% FBS and 40 U/mL IL-2, and the resulting mixture was kept on ice for later use.

Activation of T cells: The corresponding Dynabead Human T-Activator CD3/CD28 for T Cell Expansion and Activation were added to a 1.5 mL centrifuge tube in an amount of 25µL of the activated magnetic beads per 1 × 10⁶ cells, which should be shaken on a shaker for about 30 s before aspiration. In the centrifuge tube, at a volume ratio of greater than 1: 1, the activated magnetic beads were washed 3 times with a medium, and all the washing liquid was removed after the last washing. A complete medium with a volume equal to the starting volume was added to resuspend the activated magnetic beads. The washed activated magnetic beads were added to the cell resuspension, and the resulting mixture was well mixed. The cells were added to a six-well plate at 3 mL/well and incubated in a cell incubator at 37 °C with 5% CO₂ for 2 days.

Compound treatment: A 20 mM compound stock solution was diluted to 200 µM with DMSO, and then diluted 4-fold to 50 µM (50×) with a complete medium. The resulting mixture was well mixed for later use. 4-fold diluted DMSO (25% DMSO) was added to negative control wells. T cells were activated for two days and pipetted uniformly. The 1.5 mL centrifuge tube was mounted on a magnetic rack, and the activated magnetic beads were removed. The cell suspension was collected, and the cells were counted and then centrifuged at 300× g for 10 min to discard the supernatant. The cells were then resuspended to 1.02 × 10⁶ cells/mL, and 980 µL of the cell suspension and 20 µL of the 50× compound were added to each 24-well plate, with the final concentration of the compound being 1 µM. The cells were placed in a cell incubator at 37 °C with 5% CO₂ for another 3 days.

RNA extraction: T cells were collected by centrifugation at 1500 rpm for 3 min, washed once with PBS, and centrifuged again to discard the supernatant. The total cellular RNA was extracted using a microRNA extraction kit according to the package insert. 700 µL of a Trizol cell lysis buffer was added to the cell pellet, and the resulting mixture was pipetted uniformly and left at room temperature for 5 min. 140 µL of chloroform was added, and the resulting mixture was well mixed with shaking and left to stand at room temperature for 3 min. The chloroform-cell lysis buffer mixture was centrifuged at 12000× g and 4 °C for 15 min. The supernatant was transferred to a new RNase-free centrifuge tube, and 1.5 volumes of absolute ethanol were added. The resulting solution was pipetted several times, then transferred to an RNA adsorption column, and centrifuged at 8000× g for 15 s. The column was washed once with 700 µL of an RWT solution, centrifuged at 8000× g for 15 s, then washed twice with 500 µL of an RPE solution, and centrifuged at 8000× g for 2 min. The adsorption column was placed in a new 2 mL centrifuge tube and centrifuged at 12000× g for 1 min to remove the residual washing liquid. The adsorption column was placed in a new 1.5 mL centrifuge tube, and 30-50 µL of RNase-free water was added. The tube was centrifuged at 12000× g for 2 min, and a solution was collected as an RNA solution. RNA concentration was measured using a spectrophotometer. The RNA solution was stored in a refrigerator at -80 °C.

Reverse transcription: The above extracted RNA template was placed on ice. The miScript II RT Kit was taken, and some of the components (containing 5 × miScript HiSpec Buffer, 10 × miScript nucleics Mix, and RNase-free water) were thawed at room temperature. The miScript Reverse Transcriptase mix component was thawed on ice. The components of each reaction (10 µL) were as follows: 5 × miScript HiSpec Buffer (2 µL), 10 × miScript nucleics Mix (1 µL), miScript Reverse Transcriptase mix (1 µL), RNase-free water (2 µL), and RNA template (4 µL). The above reaction was prepared on ice. The sample was placed in a PCR instrument, and the procedure was set as follows: 37 °C for 60 min; 95 °C for 5 min; stored at 4 °C. The sample obtained after the completion of the reaction was a cDNA sample.

Fluorescence quantitative PCR: The transcription level of miR-124 was detected using a SYBR green staining method, and meanwhile the transcription level of housekeeping gene U6 was detected as an internal reference. All reagents required for the miScript SYBR Green PCR Kit were thawed to normal temperature, and each cDNA sample template was diluted 10-fold with RNase-free water and then diluted 5-fold. A reaction mixture was prepared as in Table 1 below and then added to a 96-well PCR plate. The plate was sealed with a plate sealing film and centrifuged. The PCR reaction was performed on a fluorescence quantitative PCR instrument according to the procedures in Table 2.

**Table 1. Components of fluorescence quantitative PCR reaction**

| Components | miR-124 detection (µL) | U6 housekeeping gene detection (µL) |
|---|---|---|
| | | |
| 2×QuantiTest SYBR Green PCR Master Mix | 12.5 | 12.5 |
| 10 × miScript universal primer | 2.5 | 2.5 |
| 10 × forward primer (5 µM) | 2.5 (miR124-3p-F) | 2.5 (Hsa-U6 primer) |
| RNase-free water | 5 | 5 |
| cDNA template | 2.5 (diluted 10-fold) | 2.5 (diluted 50-fold) |
| Total volume | 25 | 25 |

**Table 2. Fluorescence quantitative PCR procedures**

| Procedures | | Temperature | Time | Number of cycles |
|---|---|---|---|---|
| Initial | | 95°C | 15min | 1 |
| 3-step cycle | Denaturation | 94°C | 15s | 40 |
| | Annealing | 55°C | 30s | |
| | Extension | 70°C | 30s | |

**Table 3. Primers for fluorescence quantitative PCR detection**

| Primer name | Sequence (5'-3') | Source |
|---|---|---|
| miR124-3p-F | TAAGGCACGCGGTG AATGCC | customized by Genewiz |
| Hsa-U6 primer | / | miRNA primer library of Tiangen Biotech Co., Ltd. |
| 10 × miScript universal primer | / | included with miScript SYBR Green PCR Kit |

Data analysis: Based on the CT values calculated by the software, the ratio of the expression level of miR-124 to the expression level of the internal reference U6 was calculated for each sample, i.e., ΔCT (test compound) = CT_{miRNA-124} (test compound) - CT_{U6} (test compound). The relative expression level was calculated by the following formula: relative expression level (test compound) = 2^{(-[ΔCT (test compound)} - ΔCT (DMSO)]).

**Table 4. Activity of the compounds disclosed herein in miR-124 up-regulation**

| Compound | miR-124 up-regulation (fold) |
|---|---|
| DMSO | 1.0 |
| 1 | 3.9 |
| 2 | 1.2 |
| 3 | 5.2 |
| 4 | 5.4 |
| 5 | 1.4 |
| 6 | 2.7 |
| 7 | 5.2 |
| 8 | 5.4 |
| 9 | 2.4 |
| 10 | 2.1 |
| 11 | 2.4 |
| 12 | 1.8 |
| 13 | 2.8 |
| 14 | 1.5 |
| 15 | 5.7 |
| 16 | 3.9 |
| 17 | 5.9 |
| 18 | 2.7 |
| 20 | 5.0 |
| 21 | 1.9 |
| 22 | 1.7 |
| 23 | 2.3 |
| 24 | 3.0 |
| 25 | 6.7 |
| 26 | 2.4 |

Conclusion: The compounds disclosed herein had good activity in promoting miR-124 up-regulation.

### Test Example 3. Pharmacokinetic Study of Compound Disclosed Herein

### 1. Abstract

With rats as test animals, the plasma concentration of the compound of Example 2 and a comparative compound A (see compound (1) of Example 3 in WO2016135052A1) were determined by LC/MS/MS at different time points after intragastric administration and intravenous injection to the rats. The pharmacokinetic behavior in rats of the compound disclosed herein was studied and its pharmacokinetic profile was evaluated.

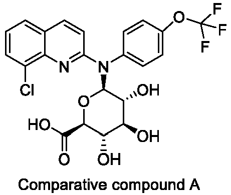

### 2. Methodology

### 2.1. Test drug

Compound of Example 2, comparative compound A.

### 2.2. Test animals

16 healthy adult SD rats (half male and half female, purchased from Vital River Laboratory Animal Technology Co., Ltd.) were evenly divided into 4 groups.

### 2.3. Drug preparation

A certain amount of the compound was weighed out, and 5% of DMSO, 5% of Tween 80, and 90% of normal saline were added to give a colorless and clear solution.

### 2.4. Administration

Intragastric administration group: SD rats were fasted overnight, and then the compound was administered intragastrically to the rats at a dose of 2 mg/kg and a volume of 10.0 mL/kg.

Intravenous administration group: SD rats were fasted overnight, and then the compound was administered to the rats by intravenous injection at a dose of 1 mg/kg and a volume of 5.0 mL/kg.

### 3. Procedures

Intragastric administration group: The compound of Example 2 and the comparative compound A were administered intragastrically to the rats, and 0.1 mL of blood was collected from the orbit before administration and 0.25 h, 0.5 h, 1.0 h, 2.0 h, 4.0 h, 6.0 h, 8.0 h, 11.0 h and 24.0 h after administration. The blood was placed in an EDTA-K2 anticoagulation tube and centrifuged at 10000 rpm for 1 min at 4 °C. The plasma was separated within 1 h and stored at -20 °C before analysis. The process from blood collection to centrifugation was performed in an ice bath. Two hours after administration, feeding was resumed.

Intravenous administration group: The rats were injected intravenously with the compound of Example 2 and the comparative compound A, and the blood was collected before administration and 5 min, 15 min, 0.5 h, 1.0 h, 2.0 h, 4.0 h, 8.0 h, 11.0 h and 24.0 h after administration and treated in the same way as the blood of the intragastric administration group.

Determination of plasma concentrations in rats after intragastric administration and intravenous injection of the test compounds at different concentrations: 20 µL of rat plasma at each time point after administration was mixed with 50 µL of an internal standard solution (camptothecin, 100 ng/mL) and 200 µL of acetonitrile, and the mixture was vortexed for 5 min and centrifuged for 10 min at 3700-4000 rpm. 1.0-2.0 µL of supernatant of the plasma sample was taken for LC/MS/MS analysis.

### 4. Pharmacokinetic parameters

**Table 5. Pharmacokinetic parameters for intragastric administration of the compound disclosed herein**

| | Pharmacokinetic experiment (intragastric administration, 2 mg/kg) | | | | | |
|---|---|---|---|---|---|---|
| No. | Plasma concentration Cmax (ng/mL) | Area under curve AUC (ng/mL *h) | Half-life t1/2 (h) | Residence time MRT (h) | Clearance CL/F (mL/min/kg) | Apparent volume of distribution Vz/F (mL/kg) |
| 2 | 242 | 1215 | 2.47 | 4.32 | 28.7 | 6184 |
| Comparative compound A | 105 | 311 | 2.57 | 3.76 | 95.7 | 21376 |

**Table 6. Pharmacokinetic parameters for intravenous injection of the compound disclosed herein**

| | Pharmacokinetic experiment (intravenous injection, 1 mg/kg) | | | | |
|---|---|---|---|---|---|
| No. | Area under curve AUC (ng/mL *h) | Half-life t1/2 (h) | Residence time MRT (h) | Clearance CL (mL/min/kg) | Apparent volume of distribution Vz (mL/kg) |
| 2 | 23007 | 2.85 | 2.10 | 0.781 | 178 |
| Comparative compound A | 11873 | 1.24 | 1.17 | 1.45 | 151 |

Conclusion: As can be seen from Tables 5 and 6, the compound of Example 2 of the present disclosure demonstrated good absorption profile and significant pharmacokinetic superiority as compared to the comparative compound A.

## Claims

1. A compound of general formula (I) or a pharmaceutically acceptable salt thereof: 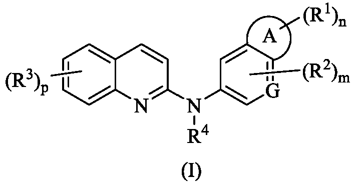 wherein
ring A is 3- to 8-membered cycloalkyl or 3- to 8-membered heterocyclyl;
G is N atom or CR^{2a};
each R¹ is identical or different and is independently selected from the group consisting of hydrogen atom, deuterium atom, halogen, C₁₋₆ alkyl, C₁₋₆ alkoxy, oxo, and -C(O)R⁸; each R² is identical or different and is independently selected from the group consisting of hydrogen atom, halogen, and C₁₋₆ alkyl;
each R³ is identical or different and is independently selected from the group consisting of halogen, C₁₋₆ alkyl, C₁₋₆ alkoxy, 3- to 8-membered cycloalkyl, 3- to 8-membered heterocyclyl, and cyano;
or two adjacent R³, together with the carbon atom on the benzene ring to which they are attached, form a 5- or 6-membered cycloalkyl;
R⁴ is hydrogen atom or 3- to 8-membered heterocyclyl, wherein the 3- to 8-membered heterocyclyl is substituted with one or more identical or different substituents selected from the group consisting of hydroxy and carboxyl;
R⁸ is C₁₋₆ alkyl;
R^{2a} is selected from the group consisting of hydrogen atom, halogen and C₁₋₆ alkyl; n is 0, 1, 2, 3, or 4;
m is 0, 1, or 2;
p is 1, 2, 3, or 4.

2. The compound of general formula (I) or the pharmaceutically acceptable salt thereof according to claim 1, being a compound of general formula (IC) or a pharmaceutically acceptable salt thereof: 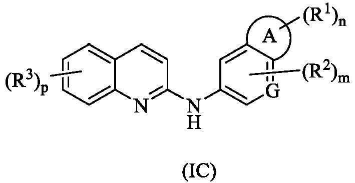 wherein
ring A, G, R¹ to R³, n, m, and p are as defined in claim 1.

3. The compound of general formula (I) or the pharmaceutically acceptable salt thereof according to claim 1, being a compound of general formula (I-1) or general formula (I-2) or a pharmaceutically acceptable salt thereof: 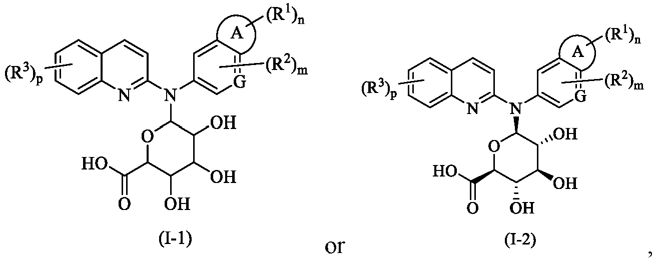 wherein
ring A, G, R¹ to R³, n, m, and p are as defined in claim 1.

4. The compound of general formula (I) or the pharmaceutically acceptable salt thereof according to any one of claims 1 to 3, wherein ring A is 5- or 6-membered cycloalkyl or 5- or 6-membered heterocyclyl; and/or each R¹ is identical or different and is independently selected from the group consisting of hydrogen atom, deuterium atom, fluorine, acetyl, methyl, and oxo.

5. The compound of general formula (I) or the pharmaceutically acceptable salt thereof according to claim 1, being a compound of general formula (II) or a pharmaceutically acceptable salt thereof: 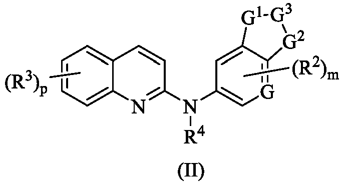 wherein 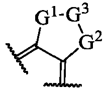 is selected from the group consisting of 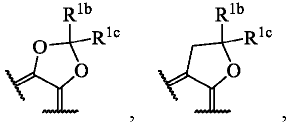 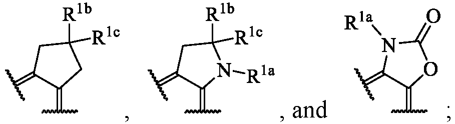
R^{1a} is selected from the group consisting of hydrogen atom, C₁₋₆ alkyl, and -C(O)R⁸,
R^{1b} and R^{1c} are identical or different and are each independently selected from the group consisting of hydrogen atom, deuterium atom, halogen and C₁₋₆ alkyl;
G, R² to R⁴, R⁸, m, and p are as defined in claim 1.

6. The compound of general formula (I) or the pharmaceutically acceptable salt thereof according to any one of claims 1 to 5, wherein R² is hydrogen atom; and/or R³ is halogen; and/or R^{2a} is hydrogen atom.

7. The compound of general formula (I) or the pharmaceutically acceptable salt thereof according to any one of claims 1 and 4 to 6, wherein R⁴ is hydrogen atom; or R⁴ is

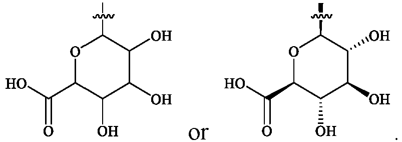

8. The compound of general formula (I) or the pharmaceutically acceptable salt thereof according to any one of claims 1 to 7, being selected from any one of the following compounds:

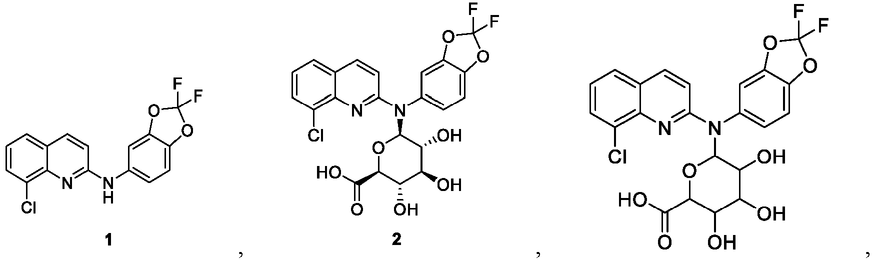

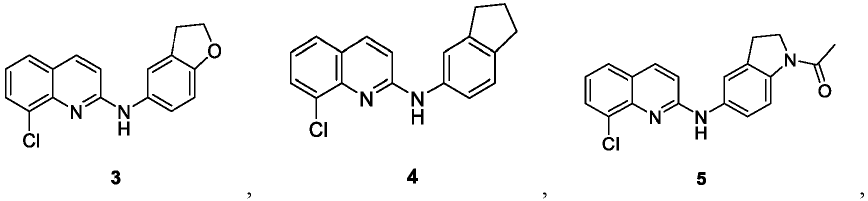

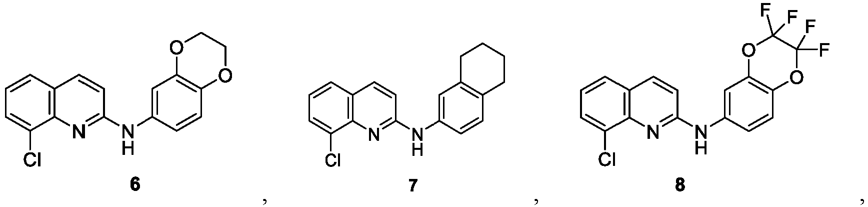

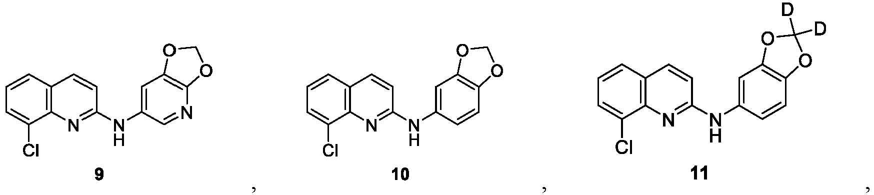

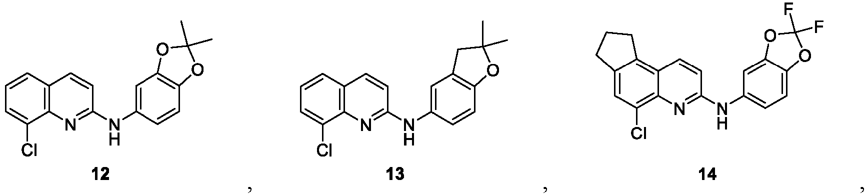

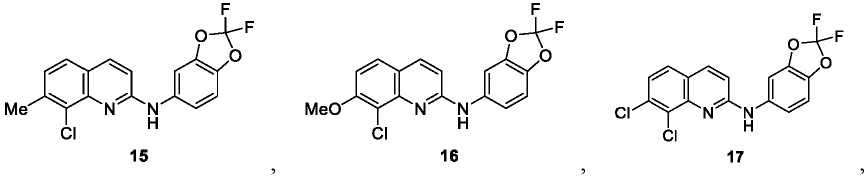

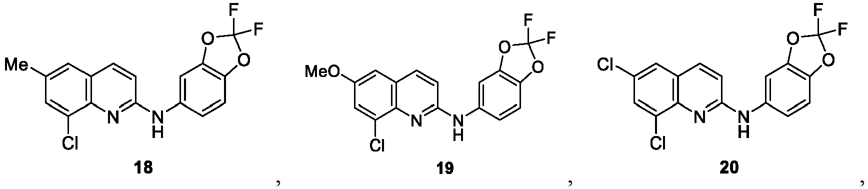

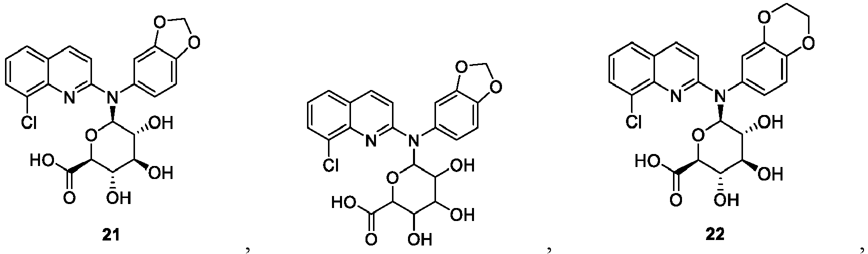

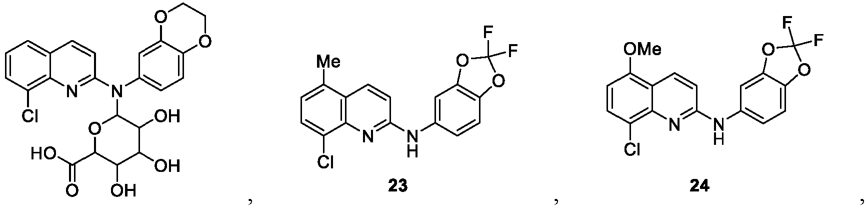

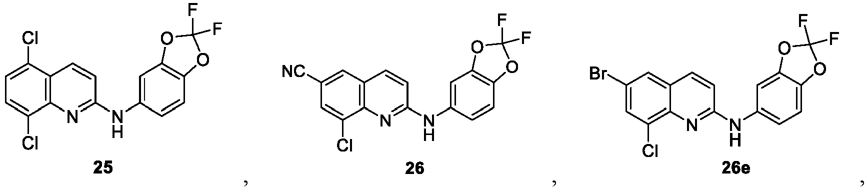

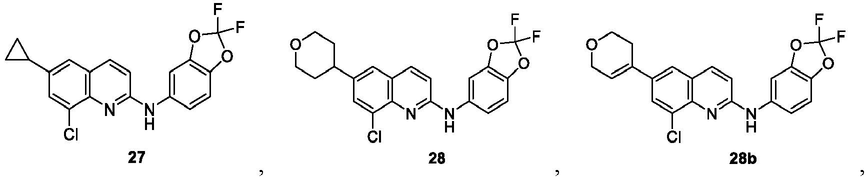

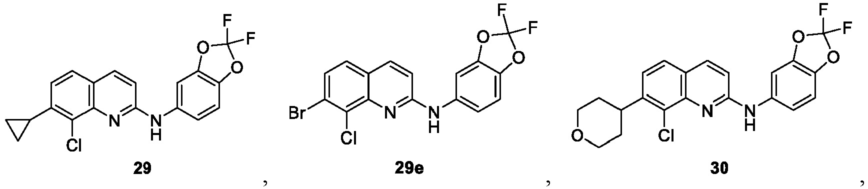

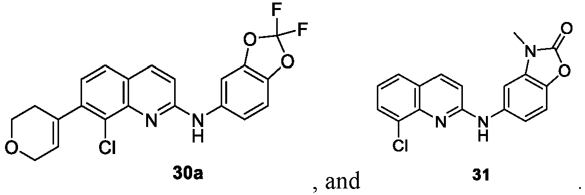

9. A compound of general formula (I-1C) or (I-2C) or a salt thereof: 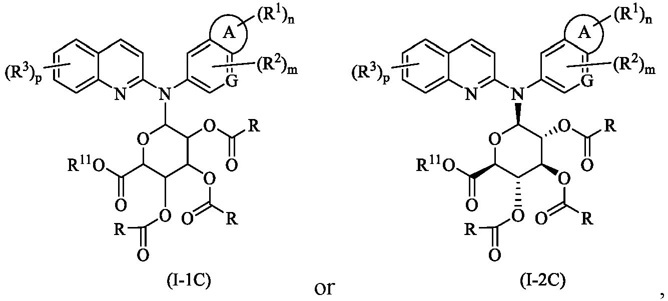 wherein
R is C₁₋₆ alkyl; R¹¹ is C₁₋₆ alkyl;
ring A, G, R¹ to R³, m, n, and p are as defined in claim 1.

10. The compound or the salt thereof according to claim 9, being selected from the group consisting of the following compounds:

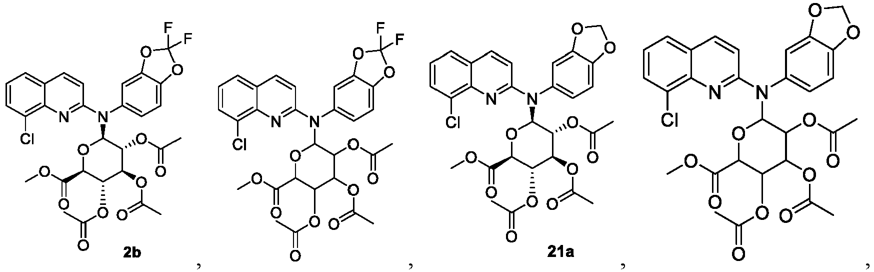

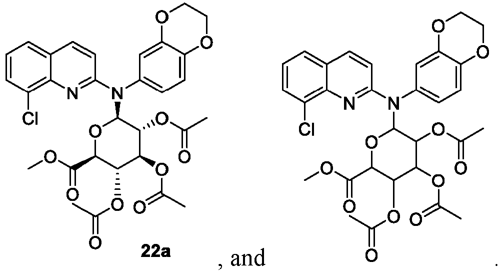

11. A method for preparing the compound of general formula (IC) or the pharmaceutically acceptable salt thereof according to claim 2, wherein the method comprises the following step: 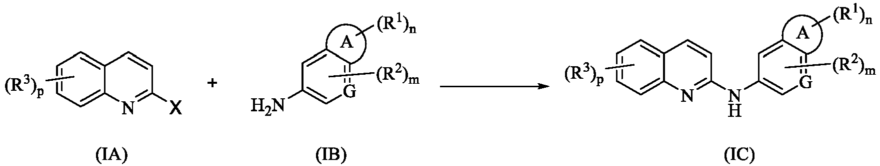
reacting a compound of general formula (IA) or a salt thereof with a compound of general formula (IB) or a salt thereof to give the compound of general formula (IC) or the pharmaceutically acceptable salt thereof,
wherein
X is halogen, preferably Cl atom;
ring A, G, R¹ to R³, m, n, and p are as defined in claim 2.

12. A method for preparing the compound of general formula (I) or the pharmaceutically acceptable salt thereof according to claim 1, wherein the method comprises the following steps: 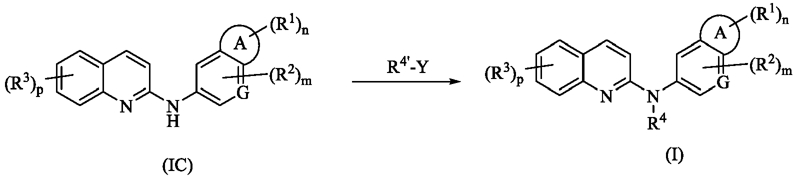
reacting a compound of general formula (IC) or a pharmaceutically acceptable salt thereof with an R^{4'}-Y compound, and then removing the protecting group on R^{4'} to give the compound of general formula (I) or the pharmaceutically acceptable salt thereof, wherein
Y is halogen, preferably Br atom;
R^{4'} is 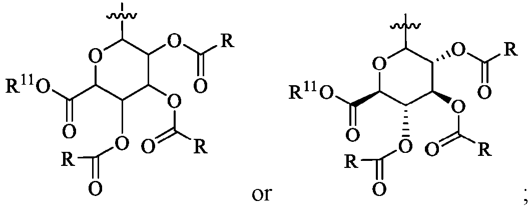
**R is** C₁₋₆ alkyl; R¹¹ is C₁₋₆ alkyl;
R⁴ is 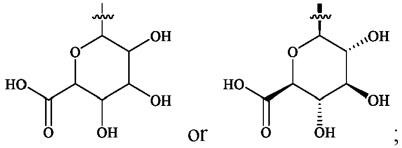
ring A, G, R¹ to R³, m, n, and p are as defined in claim 1.

13. A pharmaceutical composition, wherein the pharmaceutical composition comprises a therapeutically effective amount of the compound of general formula (I) or the pharmaceutically acceptable salt thereof according to any one of claims 1 to 8, and one or more pharmaceutically acceptable carriers, diluents or excipients.

14. The compound of general formula (I) or the pharmaceutically acceptable salt thereof according to any one of claims 1 to 8 or the pharmaceutical composition according to claim 13 for use as a medicament; preferably, for use as a medicament for regulating the level of a miRNA.

15. The compound of general formula (I) or the pharmaceutically acceptable salt thereof according to any one of claims 1 to 8 or the pharmaceutical composition according to claim 13 for use in treating and/or preventing a disease or condition, wherein the disease or condition is selected from the group consisting of a viral infection, an inflammation, and a cancer;
preferably the inflammation is selected from the group consisting of an autoimmune-related inflammatory disease, an inflammatory disease in the central nervous system (CNS), an inflammatory disease in the joints, an inflammatory disease in the digestive tract, an inflammatory disease in the skin, other inflammatory diseases related to epithelial cells, a cancer-related inflammation, an irritation-related inflammation, and an injury-related inflammation; and/or the inflammation is selected from the group consisting of inflammatory bowel disease, rheumatoid arthritis, multiple sclerosis, Alzheimer's disease, Parkinson's disease, osteoarthritis, atherosclerosis, ankylosing spondylitis, psoriasis, dermatitis, systemic lupus erythematosus, Sjogren's syndrome, bronchitis, asthma, and a colon cancer-related inflammation;
more preferably, the inflammation is inflammatory bowel disease; preferably, the inflammatory bowel disease is ulcerative colitis (UC) or Crohn's disease (CD);
preferably, the cancer is selected from the group consisting of leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous carcinoma, papillary carcinoma, cystadenocarcinoma, medullary carcinoma, bronchogenic carcinoma, liver cancer, cholangiocarcinoma, choriocarcinoma, seminoma, embryonal carcinoma, Wilms' tumor, cervical cancer, uterine cancer, testicular cancer, lung cancer, bladder cancer, neuroglioma, medulloblastoma, craniopharyngioma, ependymoma, pinealoma, hemangioblastoma, acoustic neuroma, schwannoma, neurofibroma, retinoblastoma, melanoma, skin cancer, kidney cancer, nasopharyngeal cancer, gastric cancer, esophageal cancer, head and neck cancer, colorectal cancer, small intestine cancer, gallbladder cancer, pediatric tumor, urothelial cancer, ureteral tumor, thyroid cancer, osteoma, neuroblastoma, brain tumor, and myeloma.

16. The compound of general formula (I) or the pharmaceutically acceptable salt thereof according to any one of claims 1 to 8 or the pharmaceutical composition according to claim 13 for use in treating and/or preventing AIDS or an AIDS-related condition or human immunodeficiency virus (HIV).

## Patentansprüche

1. Verbindung der allgemeinen Formel (I) oder ein pharmazeutisch verträgliches Salz davon: 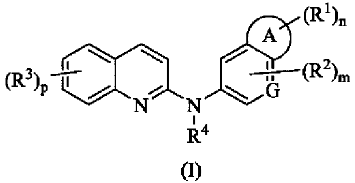 wobei
Ring A 3- bis 8-gliedriges Cycloalkyl oder 3- bis 8-gliedriges Heterocyclyl ist;
G ein N-Atom oder CR^{2a} ist;
jedes R¹ identisch oder unterschiedlich ist und unabhängig aus der Gruppe ausgewählt ist, bestehend aus Wasserstoffatom, Deuteriumatom, Halogen, C₁₋₆-Alkyl, C₁₋₆-Alkoxy, Oxo und -C(O)R⁸;
jedes R² identisch oder unterschiedlich ist und unabhängig aus der Gruppe ausgewählt ist, bestehend aus Wasserstoffatom, Halogen und C₁₋₆-Alkyl;
jedes R³ identisch oder unterschiedlich ist und unabhängig aus der Gruppe ausgewählt ist, bestehend aus Halogen, C₁₋₆-Alkyl, C₁₋₆-Alkoxy, 3- bis 8-gliedrigem Cycloalkyl, 3- bis 8-gliedrigem Heterocyclyl und Cyano;
oder zwei angrenzende R³ zusammen mit dem Kohlenstoffatom an dem Benzolring, an den sie gebunden sind, ein 5- oder 6-gliedriges Cycloalkyl ausbilden;
R⁴ ein Wasserstoffatom oder 3- bis 8-gliedriges Heterocyclyl ist, wobei das 3- bis 8-gliedrige Heterocyclyl mit einem oder mehreren identischen oder unterschiedlichen Substituenten substituiert ist, ausgewählt aus der Gruppe, bestehend aus Hydroxy und Carboxyl;
R⁸ C₁₋₆-Alkyl ist;
R^{2a} aus der Gruppe ausgewählt ist, bestehend aus Wasserstoffatom, Halogen und C₁₋₆-Alkyl;
n 0, 1, 2, 3 oder 4 ist;
m 0, 1 oder 2 ist;
p 1, 2, 3 oder 4 ist.

2. Verbindung der allgemeinen Formel (I) oder das pharmazeutisch verträgliche Salz davon nach Anspruch 1, die eine Verbindung der allgemeinen Formel (IC) oder ein pharmazeutisch verträgliches Salz davon ist: 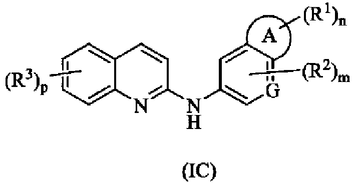 wobei
Ring A, G, R¹ bis R³, n, m und p wie in Anspruch 1 definiert sind.

3. Verbindung der allgemeinen Formel (I) oder das pharmazeutisch verträgliche Salz davon nach Anspruch 1, die eine Verbindung der allgemeinen Formel (I-1) oder der allgemeinen Formel (1-2) oder ein pharmazeutisch verträgliches Salz davon ist: 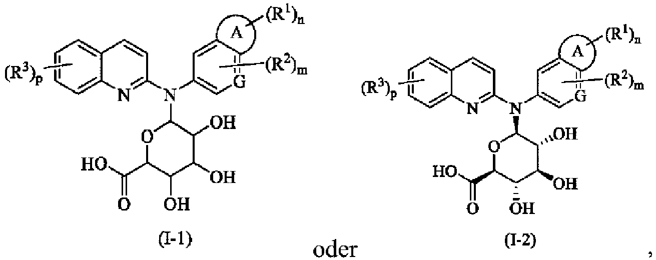 wobei
Ring A, G, R¹ bis R³, n, m und p wie in Anspruch 1 definiert sind.

4. Verbindung der allgemeinen Formel (I) oder das pharmazeutisch verträgliche Salz davon nach einem der Ansprüche 1 bis 3, wobei Ring A 5- oder 6-gliedriges Cycloalkyl oder 5- oder 6-gliedriges Heterocyclyl ist; und/oder jedes R¹ identisch oder unterschiedlich ist und unabhängig ausgewählt ist aus der Gruppe, bestehend aus Wasserstoffatom, Deuteriumatom, Fluor, Acetyl, Methyl und Oxo.

5. Verbindung der allgemeinen Formel (I) oder das pharmazeutisch verträgliche Salz davon nach Anspruch 1, die eine Verbindung der allgemeinen Formel (II) oder ein pharmazeutisch verträgliches Salz davon ist: 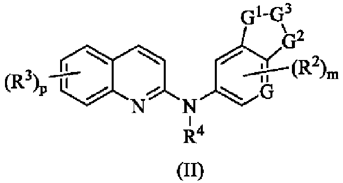 wobei 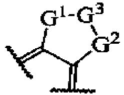 ausgewählt ist aus der Gruppe bestehend aus 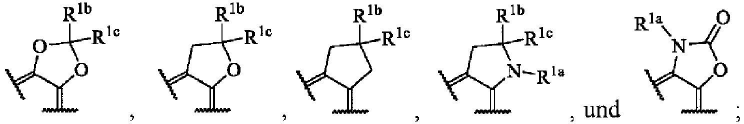
R^{1a} **aus der** Gruppe ausgewählt ist, bestehend aus Wasserstoffatom, C₁₋₆-Alkyl und C(O)R⁸,
R^{1b} und R^{1c} identisch oder unterschiedlich sind und jeweils unabhängig ausgewählt sind aus der Gruppe, bestehend aus Wasserstoffatom, Deuteriumatom, Halogen und C₁₋₆-Alkyl;
G, R² bis R⁴, R⁸, m und p wie in Anspruch 1 definiert sind.

6. Verbindung der allgemeinen Formel (I) oder das pharmazeutisch verträgliche Salz davon nach einem der Ansprüche 1 bis 5, wobei R² ein Wasserstoffatom ist; und/oder R³ Halogen ist; und/oder R^{2a} ein Wasserstoffatom ist.

7. Verbindung der allgemeinen Formel (I) oder das pharmazeutisch verträgliche Salz davon nach einem der Ansprüche 1 und 4 bis 6, wobei R⁴ ein Wasserstoffatom ist; oder R⁴ ist

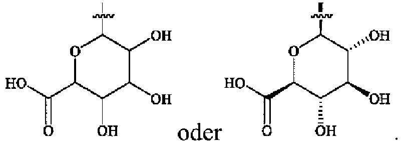

8. Verbindung der allgemeinen Formel (I) oder das pharmazeutisch verträgliche Salz davon nach einem der Ansprüche 1 bis 7, ausgewählt aus einer beliebigen der folgenden Verbindungen:

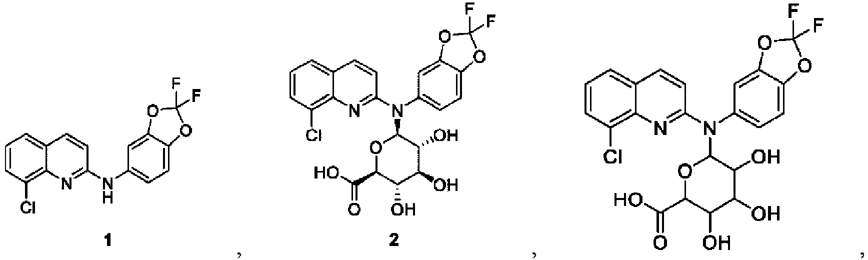

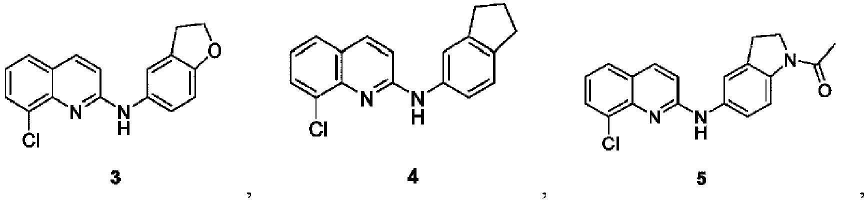

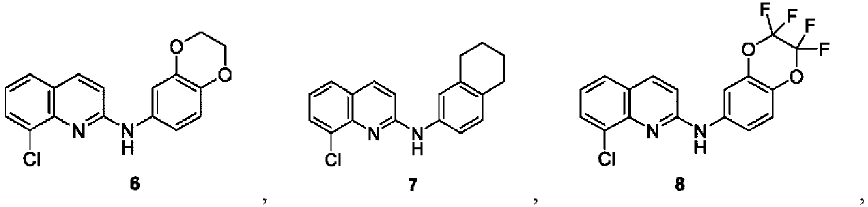

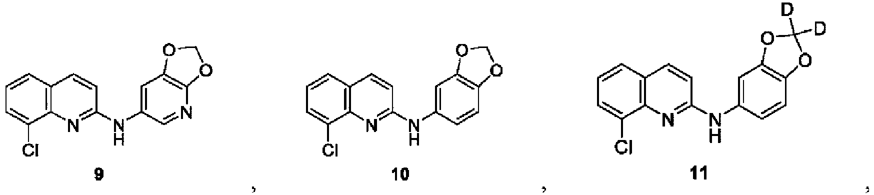

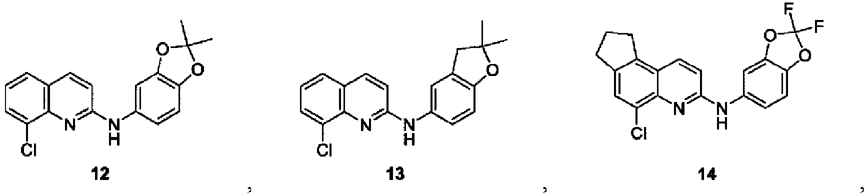

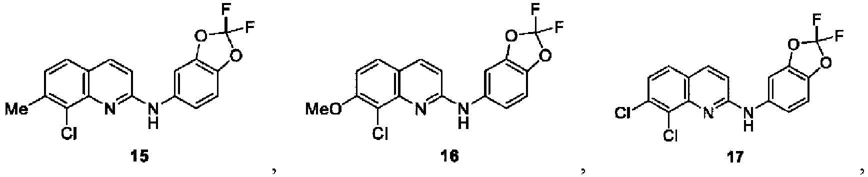

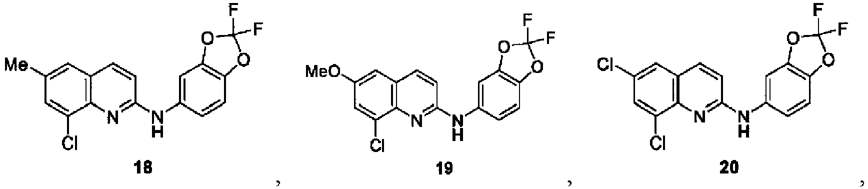

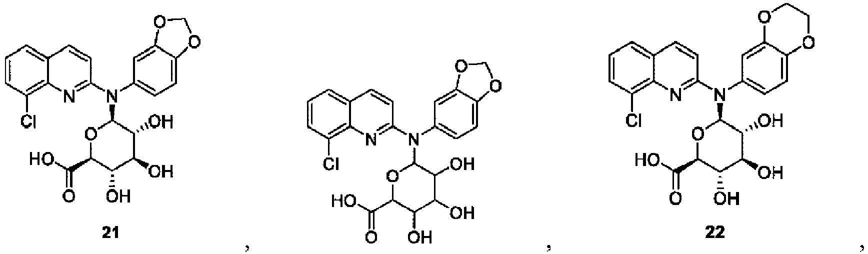

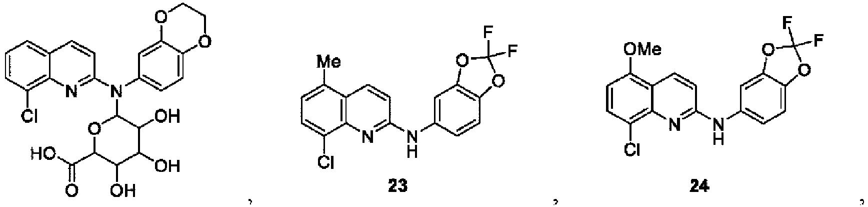

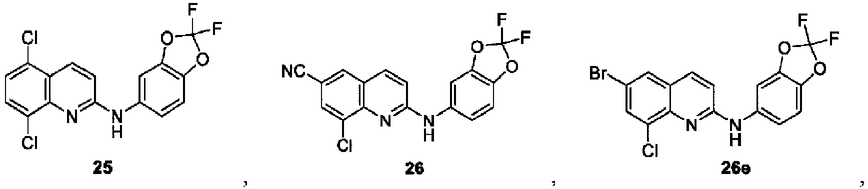

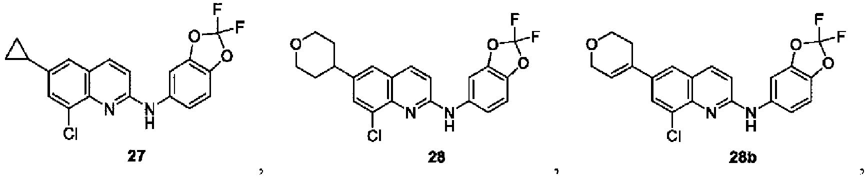

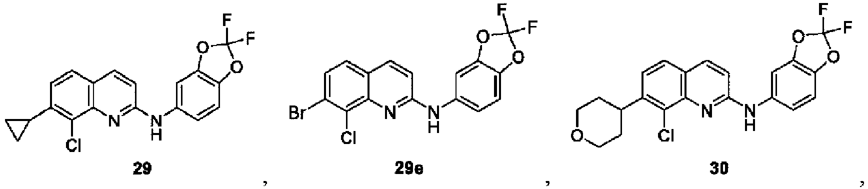

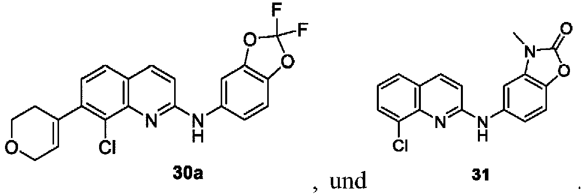

9. Verbindung der allgemeinen Formel (I-1C) oder (I-2C) oder ein Salz davon: 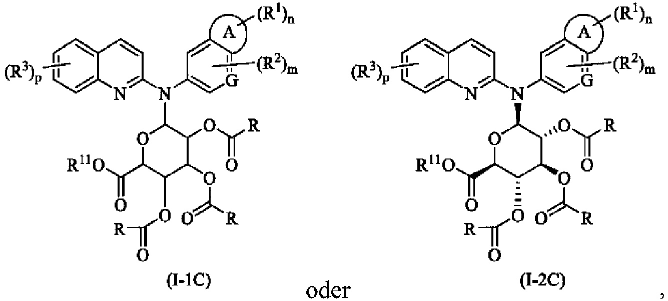 wobei
R C₁₋₆-Alkyl ist; R¹¹ C₁₋₆-Alkyl ist;
Ring A, G, R¹ bis R³, m, n und p wie in Anspruch 1 definiert sind.

10. Verbindung oder das Salz davon nach Anspruch 9, ausgewählt aus der Gruppe, bestehend aus den folgenden Verbindungen:

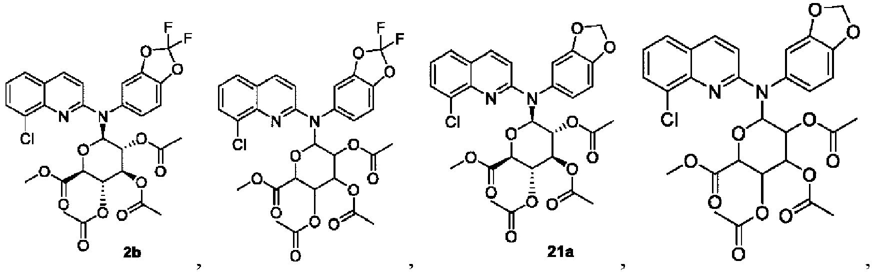

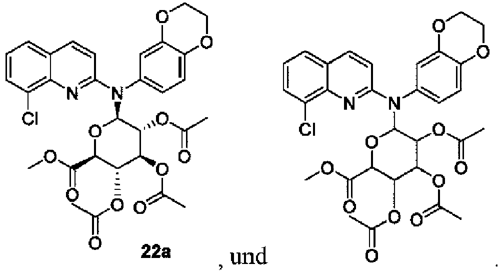

11. Verfahren zum Herstellen der Verbindung der allgemeinen Formel (IC) oder des pharmazeutisch verträglichen Salzes davon nach Anspruch 2, wobei das Verfahren den folgenden Schritt umfasst: 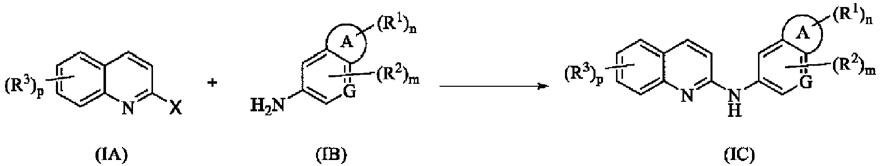
Umsetzen einer Verbindung der allgemeinen Formel (IA) oder eines Salzes davon mit einer Verbindung der
allgemeinen Formel (IB) oder eines Salzes davon, um die Verbindung der allgemeinen Formel (IC) oder das pharmazeutisch verträgliche Salz davon zu ergeben,
wobei
X Halogen ist, vorzugsweise ein CI-Atom;
Ring A, G, R¹ bis R³, m, n und p wie in Anspruch 2 definiert sind.

12. Verfahren zum Herstellen der Verbindung der allgemeinen Formel (I) oder des pharmazeutisch verträglichen Salzes davon nach Anspruch 1, wobei das Verfahren die folgenden Schritte umfasst: 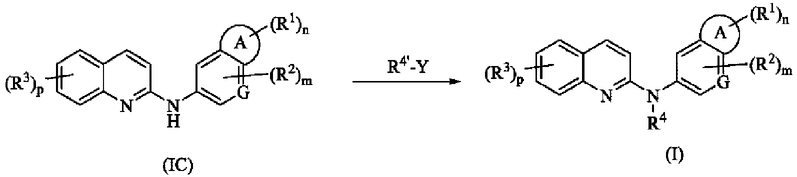
Umsetzen einer Verbindung der allgemeinen Formel (IC) oder eines pharmazeutisch verträglichen Salzes davon mit einer R^{4'}-Y-Verbindung und anschließendes Entfernen der Schutzgruppe an R^{4'}, um die Verbindung der allgemeinen Formel (I) oder das pharmazeutisch verträgliche Salz davon zu ergeben,
wobei
Y Halogen ist, vorzugsweise ein Br-Atom;
R^{4'} 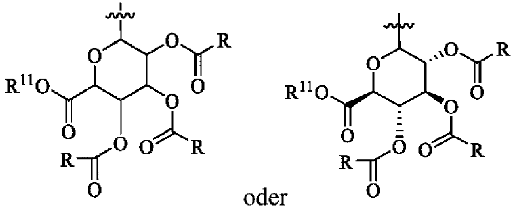 ist;
R C₁₋₆-Alkyl ist; R¹¹ C₁₋₆-Alkyl ist;
R⁴ 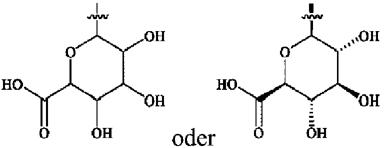 ist;
Ring A, G, R¹ bis R³, m, n und p wie in Anspruch 1 definiert sind.

13. Pharmazeutische Zusammensetzung, wobei die pharmazeutische Zusammensetzung eine therapeutisch wirksame Menge der Verbindung der allgemeinen Formel (I) oder des pharmazeutisch verträglichen Salzes davon nach einem der Ansprüche 1 bis 8 und einen oder mehrere pharmazeutisch verträgliche Träger, Verdünnungsmittel oder Bindemittel umfasst.

14. Verbindung der allgemeinen Formel (I) oder das pharmazeutisch verträgliche Salz davon nach einem der Ansprüche 1 bis 8 oder die pharmazeutische Zusammensetzung nach Anspruch 13 zur Verwendung als ein Arzneimittel; vorzugsweise zur Verwendung als ein Arzneimittel zum Regulieren des Spiegels einer miRNA.

15. Verbindung der allgemeinen Formel (I) oder das pharmazeutisch verträgliche Salz davon nach einem der Ansprüche 1 bis 8 oder die pharmazeutische Zusammensetzung nach Anspruch 13 zur Verwendung bei einem Behandeln und/oder Vorbeugen einer Erkrankung oder eines Leidens, wobei die Erkrankung oder das Leiden aus der Gruppe ausgewählt ist, bestehend aus einer Virusinfektion, einer Entzündung und einem Krebs;
vorzugsweise die Entzündung ausgewählt ist aus der Gruppe, bestehend aus einer autoimmunbezogenen entzündlichen Erkrankung, einer entzündlichen Erkrankung in dem Zentralnervensystem (ZNS), einer entzündlichen Erkrankung in den Gelenken, einer entzündlichen Erkrankung in dem Verdauungstrakt, einer entzündlichen Erkrankung in der Haut, anderen entzündlichen Erkrankungen bezogen auf Epithelzellen, einer krebsbezogenen Entzündung, einer reizungsbezogenen Entzündung und einer verletzungsbezogenen Entzündung; und/oder die Entzündung ausgewählt ist aus der Gruppe, bestehend aus entzündlicher Darmerkrankung, rheumatoider Arthritis, Multipler Sklerose, Alzheimer-Krankheit, Parkinson-Krankheit, Osteoarthritis, Atherosklerose, Spondylitis ankylosans, Psoriasis, Dermatitis, systemischem Lupus erythematodes, Sjögren-Syndrom, Bronchitis, Asthma und einer Kolonkarzinombezogenen Entzündung;
mehr bevorzugt die Entzündung eine entzündliche Darmerkrankung ist; vorzugsweise die entzündliche Darmerkrankung Colitis ulcerosa (UC) oder Morbus Crohn (CD) ist;
vorzugsweise der Krebs ausgewählt ist aus der Gruppe, bestehend aus Leukämie, Lymphom, Makroglobulinämie, Schwerkettenkrankheit, Sarkom, Karzinom, Pankreaskrebs, Brustkrebs, Eierstockkrebs, Prostatakrebs, Plattenepithelkarzinom, Schweißdrüsenkarzinom, Talgdrüsenkarzinom, papillärem Karzinom, Zystadenokarzinom, medullärem Karzinom, Bronchialkarzinom, Leberkrebs, Cholangiokarzinom, Chorionkarzinom, Seminom, embryonalem Karzinom, Wilms-Tumor, Gebärmutterhalskrebs, Gebärmutterkrebs, Hodenkrebs, Lungenkrebs, Blasenkrebs, Neurogliom, Medulloblastom, Kraniopharyngeom, Ependymom, Pinealom, Hämangioblastom, Akustikusneurinom, Schwannom, Neurofibrom, Retinoblastom, Melanom, Hautkrebs, Nierenkrebs, Nasopharynxkrebs, Magenkrebs, Speiseröhrenkrebs, Kopf- und Halskrebs, kolorektalem Krebs, Dünndarmkrebs, Gallenblasenkrebs, pädiatrischem Tumor, Urothelkarzinom, Harnleitertumor, Schilddrüsenkrebs, Osteom, Neuroblastom, Hirntumor und Myelom.

16. Verbindung der allgemeinen Formel (I) oder das pharmazeutisch verträgliche Salz davon nach einem der Ansprüche 1 bis 8 oder die pharmazeutische Zusammensetzung nach Anspruch 13 zur Verwendung bei dem Behandeln und/oder Vorbeugen von AIDS oder einem AIDS-bezogenen Zustand oder dem Humanen Immundefizienz-Virus (HIV).

## Revendications

1. Composé de formule générale (I) ou sel pharmaceutiquement acceptable de celui-ci : 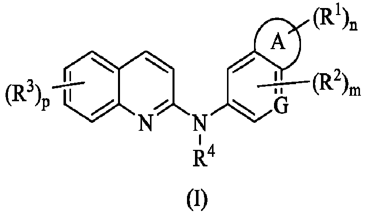 dans lequel
le cycle A est un cycloalkyle de 3 à 8 chaînons ou un hétérocyclyle de 3 à 8 chaînons ;
G est un atome de N ou CR^{2a} ;
chaque R¹ est identique ou différent et est indépendamment choisi dans le groupe constitué d'atome d'hydrogène, atome de deutérium, halogène, alkyle en C_{1 à 6}, alcoxy en C_{1 à 6}, oxo et -C(O)R⁸ ;
chaque R² est identique ou différent et est indépendamment choisi dans le groupe constitué d'atome d'hydrogène, halogène et alkyle en C_{1 à 6} ;
chaque R³ est identique ou différent et est indépendamment choisi dans le groupe constitué d'halogène, alkyle en C_{1 à 6}, alcoxy en C_{1 à 6}, cycloalkyle de 3 à 8 chaînons, hétérocyclyle de 3 à 8 chaînons et cyano ;
ou deux R³ adjacents, conjointement avec l'atome de carbone sur le cycle benzénique auquel ils sont fixés, forment un cycloalkyle de 5 ou 6 chaînons ;
R⁴ est un atome d'hydrogène ou un hétérocyclyle de 3 à 8 chaînons, dans lequel l'hétérocyclyle de 3 à 8 chaînons est substitué par un ou plusieurs substituants identiques ou différents choisis dans le groupe constitué d'hydroxy et carboxyle ;
R⁸ est alkyle en C_{1 à 6} ;
R^{2a} est choisi dans le groupe constitué d'atome d'hydrogène, halogène et alkyle en C_{1 à 6} ;
n vaut 0, 1, 2, 3 ou 4 ;
m vaut 0, 1 ou 2 ;
p vaut 1, 2, 3 ou 4.

2. Composé de formule générale (I) ou sel pharmaceutiquement acceptable de celui-ci selon la revendication 1, étant un composé de formule générale (IC) ou un sel pharmaceutiquement acceptable de celui-ci : 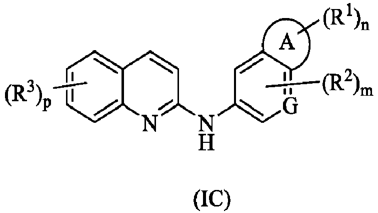 dans lequel
le cycle A, G, R¹ à R³, n, m et p sont tels que définis dans la revendication 1.

3. Composé de formule générale (I) ou sel pharmaceutiquement acceptable de celui-ci selon la revendication 1, étant un composé de formule générale (I-1) ou de formule générale (I-2) ou un sel pharmaceutiquement acceptable de celui-ci 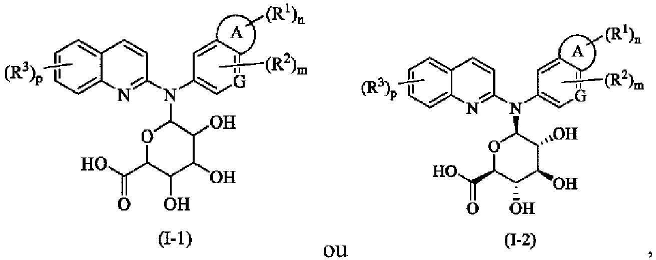 dans lequel
le cycle A, G, R¹ à R³, n, m et p sont tels que définis dans la revendication 1.

4. Composé de formule générale (I) ou sel pharmaceutiquement acceptable de celui-ci selon l'une quelconque des revendications 1 à 3, dans lequel le cycle A est cycloalkyle de 5 ou 6 chaînons ou hétérocyclyle de 5 ou 6 chaînons ; et/ou chaque R¹ est identique ou différent et est indépendamment choisi dans le groupe constitué d'atome d'hydrogène, atome de deutérium, fluor, acétyle, méthyle et oxo.

5. Composé de formule générale (I) ou sel pharmaceutiquement acceptable de celui-ci selon la revendication 1, étant un composé de formule générale (II) ou un sel pharmaceutiquement acceptable de celui-ci : 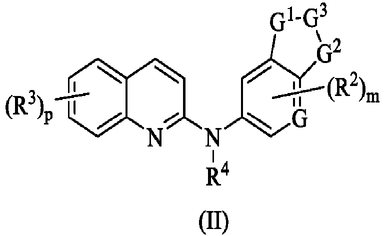 dans lequel 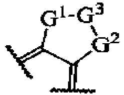 est choisi dans le groupe constitué de 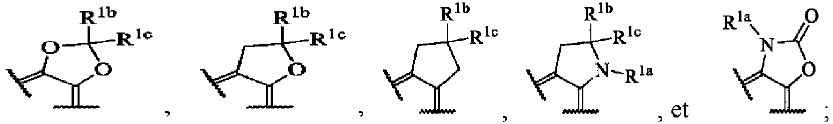
R^{1a} est choisi dans le groupe constitué d'atome d'hydrogène, alkyle en C_{1 à 6} et -C(O)R⁸,
R^{1b} et R^{1c} sont identiques ou différents et sont chacun indépendamment choisis dans le groupe constitué d'atome d'hydrogène, atome de deutérium, halogène et alkyle en C_{1 à 6} ;
G, R² à R⁴, R⁸, m et p sont tels que définis dans la revendication 1.

6. Composé de formule générale (I) ou sel pharmaceutiquement acceptable de celui-ci selon l'une quelconque des revendications 1 à 5, dans lequel R² est un atome d'hydrogène ; et/ou R³ est un halogène ; et/ou R^{2a} est un atome d'hydrogène.

7. Composé de formule générale (I) ou sel pharmaceutiquement acceptable de celui-ci selon l'une quelconque des revendications 1 et 4 à 6, dans lequel R⁴ est un atome d'hydrogène ; ou R⁴ est

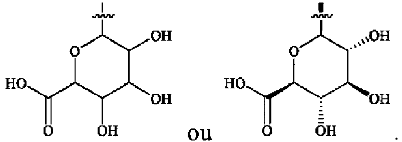

8. Composé de formule générale (I) ou sel pharmaceutiquement acceptable de celui-ci selon l'une quelconque des revendications 1 à 7, étant choisi parmi l'un quelconque des composés suivants :

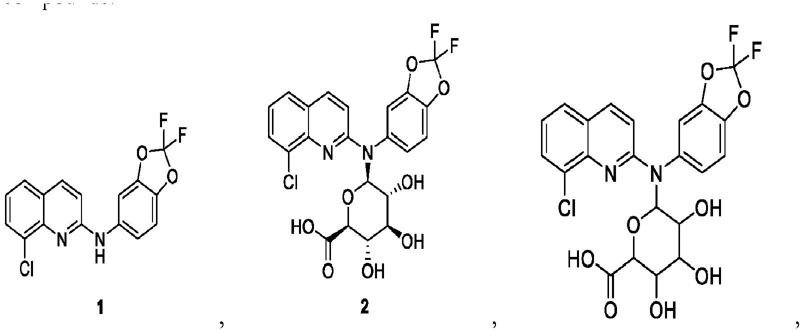

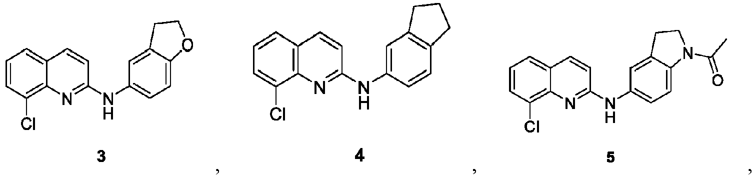

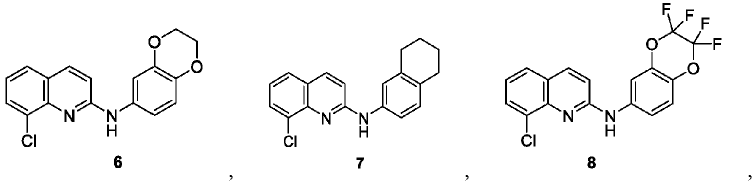

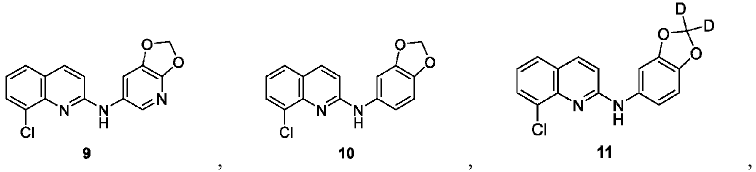

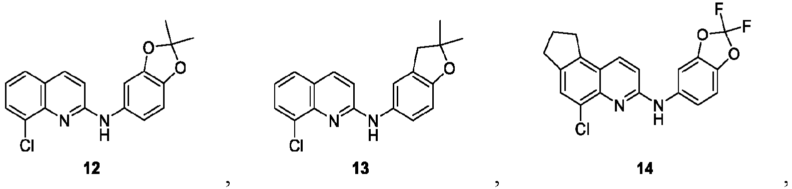

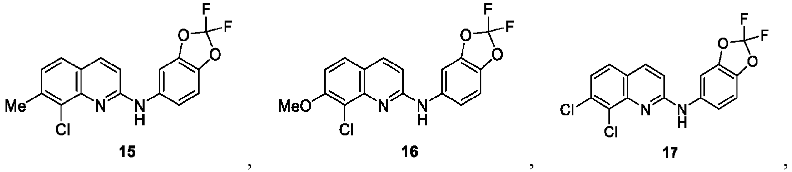

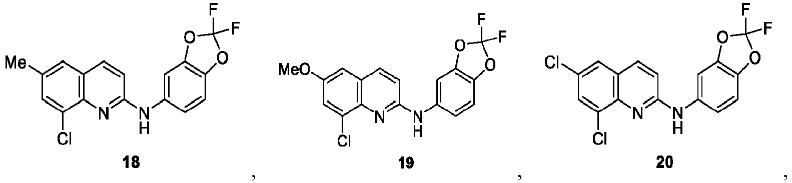

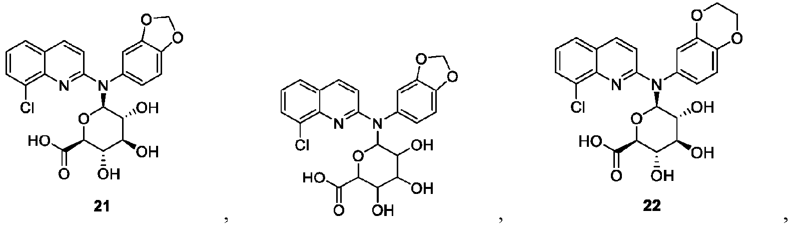

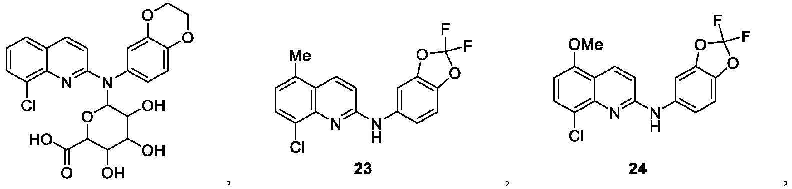

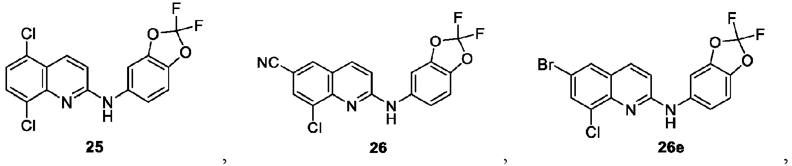

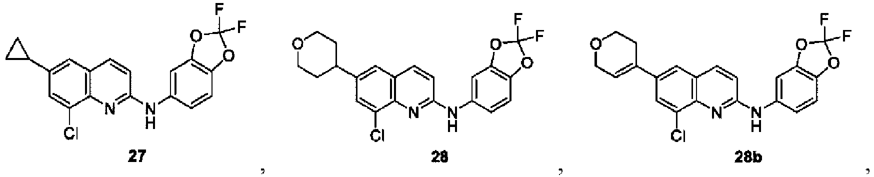

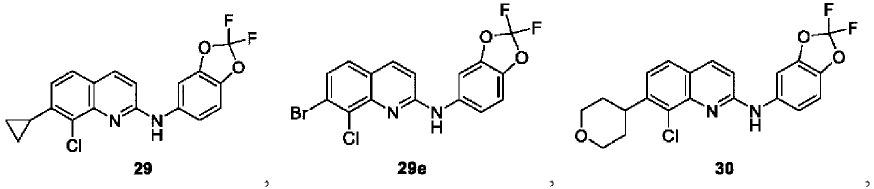

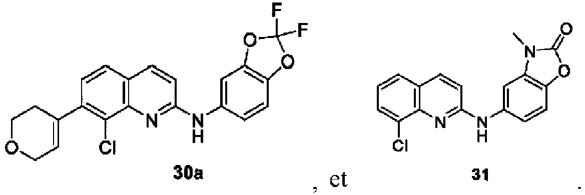

9. Composé de formule générale (I-1C) ou (I-2C) ou sel de celui-ci : 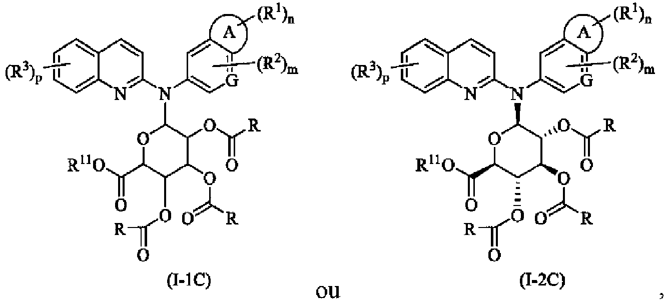 dans lequel
R est alkyle en C_{1 à 6} ; R¹¹ est alkyle en C_{1 à 6} ;
le cycle A, G, R¹ à R³, m, n et p sont tels que définis dans la revendication 1.

10. Composé ou sel de celui-ci selon la revendication 9, étant choisi dans le groupe constitué des composés suivants :

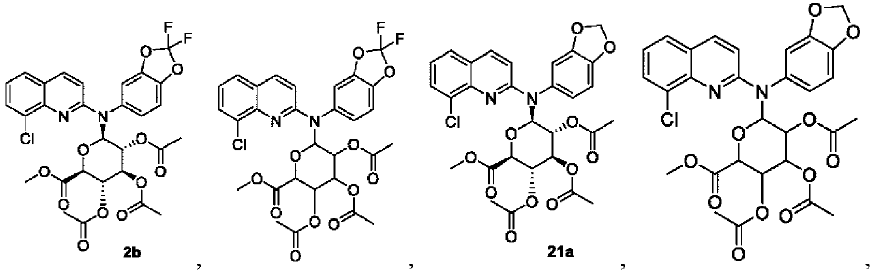

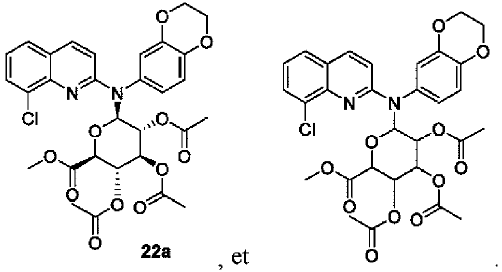

11. Procédé permettant de préparer le composé de formule générale (IC) ou le sel pharmaceutiquement acceptable de celui-ci selon la revendication 2, dans lequel le procédé comprend l'étape suivante : 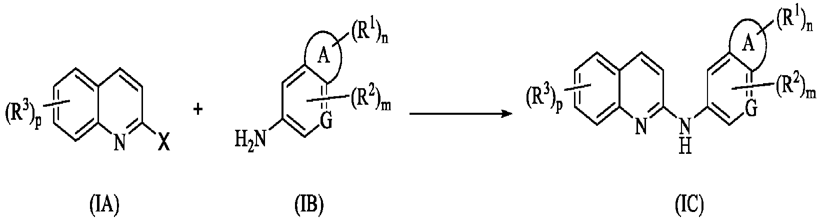
la réaction d'un composé de formule générale (IA) ou d'un sel de celui-ci avec un composé de formule générale (IB) ou un sel de celui-ci pour donner le composé de formule générale (IC) ou le sel pharmaceutiquement acceptable de celui-ci,
dans lequel
X est un halogène, de préférence un atome de CI ;
le cycle A, G, R¹ à R³, m, n et p sont tels que définis dans la revendication 2.

12. Procédé permettant de préparer le composé de formule générale (I) ou le sel pharmaceutiquement acceptable de celui-ci selon la revendication 1, dans lequel le procédé comprend les étapes suivantes : 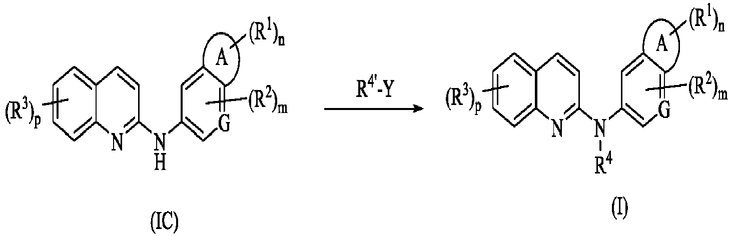
la réaction d'un composé de formule générale (IC) ou d'un sel pharmaceutiquement acceptable de celui-ci avec un composé R^{4'}-Y, puis le retrait du groupe protecteur sur R^{4'} pour donner le composé de formule générale (I) ou le sel pharmaceutiquement acceptable de celui-ci,
dans lequel
Y est un halogène, de préférence un atome de Br ;
R^{4'} est 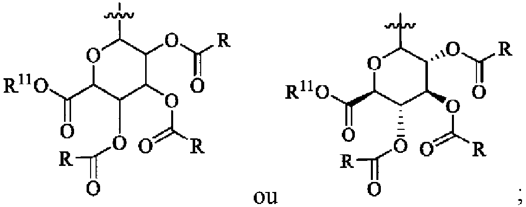
R est alkyle en C_{1 à 6} ; R¹¹ est alkyle en C_{1 à 6} ;
R⁴ est 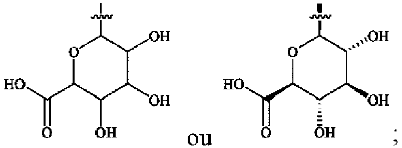
le cycle A, G, R¹ à R³, m, n et p sont tels que définis dans la revendication 1.

13. Composition pharmaceutique, dans laquelle la composition pharmaceutique comprend une quantité thérapeutiquement efficace du composé de formule générale (I) ou du sel pharmaceutiquement acceptable de celui-ci selon l'une quelconque des revendications 1 à 8, et un ou plusieurs supports, diluants ou excipients pharmaceutiquement acceptables.

14. Composé de formule générale (I) ou sel pharmaceutiquement acceptable de celui-ci selon l'une quelconque des revendications 1 à 8 ou composition pharmaceutique selon la revendication 13 pour une utilisation en tant que médicament ; de préférence, pour une utilisation en tant que médicament permettant de réguler le taux d'un miARN.

15. Composé de formule générale (I) ou sel pharmaceutiquement acceptable de celui-ci selon l'une quelconque des revendications 1 à 8 ou composition pharmaceutique selon la revendication 13 pour une utilisation dans le traitement et/ou la prévention d'une maladie ou affection, dans lequel la maladie ou affection est choisie dans le groupe constitué d'une infection virale, d'une inflammation et d'un cancer ;
de préférence l'inflammation est choisie dans le groupe constitué d'une maladie inflammatoire d'origine auto-immune, une maladie inflammatoire dans le système nerveux central (SNC), une maladie inflammatoire dans les articulations, une maladie inflammatoire dans le tube digestif, une maladie inflammatoire de la peau, d'autres maladies inflammatoires liées aux cellules épithéliales, une inflammation liée à un cancer, une inflammation liée à une irritation et une inflammation liée à une lésion ; et/ou l'inflammation est choisie dans le groupe constitué de maladie intestinale inflammatoire, polyarthrite rhumatoïde, sclérose en plaques, maladie d'Alzheimer, maladie de Parkinson, arthrose, athérosclérose, spondylarthrite ankylosante, psoriasis, dermatite, lupus érythémateux disséminé, syndrome de Sjogren, bronchite, asthme et une inflammation liée au cancer du côlon ;
plus préférablement, l'inflammation est une maladie intestinale inflammatoire ; de préférence, la maladie intestinale inflammatoire est une rectocolite hémorragique (UC) ou la maladie de Crohn (CD) ;
de préférence, le cancer est choisi dans le groupe constitué de leucémie, lymphome, macroglobulinémie, maladie des chaînes lourdes, sarcome, carcinome, cancer du pancréas, cancer du sein, cancer de l'ovaire, cancer de la prostate, carcinome épidermoïde, carcinome des glandes sudoripares, carcinome sébacé, carcinome papillaire, cystadénocarcinome, carcinome médullaire, carcinome bronchique, cancer du foie, cholangiocarcinome, choriocarcinome, séminome, carcinome embryonnaire, tumeur de Wilms, cancer du col de l'utérus, cancer de l'utérus, cancer des testicules, cancer du poumon, cancer de la vessie, neurogliome, médulloblastome, craniopharyngiome, épendymome, pinéalome, hémangioblastome, neurinome, schwannome, neurofibrome, rétinoblastome, mélanome, cancer de la peau, cancer du rein, cancer du nasopharynx, cancer gastrique, cancer de l'œsophage, cancer de la tête et du cou, cancer colorectal, cancer de l'intestin grêle, cancer de la vésicule biliaire, tumeur pédiatrique, cancer urothélial, tumeur de l'uretère, cancer de la thyroïde, ostéome, neuroblastome, tumeur cérébrale et myélome.

16. Composé de formule générale (I) ou sel pharmaceutiquement acceptable de celui-ci selon l'une quelconque des revendications 1 à 8 ou composition pharmaceutique selon la revendication 13 pour une utilisation dans le traitement et/ou la prévention du SIDA ou d'une affection liée au SIDA ou du virus de l'immunodéficience humaine (VIH).
